(19) Europäisches Patentamt
European Patent Office
Office européen des brevets

(11) EP 4 682 168 A1

(12) **EUROPEAN PATENT APPLICATION**
published in accordance with Art. 153(4) EPC

(43) Date of publication:
**21.01.2026 Bulletin 2026/04**

(21) Application number: **24770029.7**

(22) Date of filing: **15.03.2024**

(51) International Patent Classification (IPC):
***C07K 16/28*** *(2006.01)* ***A61K 39/395*** *(2006.01)*
***C07K 16/30*** *(2006.01)* ***C07K 16/46*** *(2006.01)*
***A61P 35/00*** *(2006.01)*

(52) Cooperative Patent Classification (CPC):
**A61K 39/395; A61P 35/00; C07K 16/28; C07K 16/30; C07K 16/46**

(86) International application number:
**PCT/CN2024/081911**

(87) International publication number:
**WO 2024/188339 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.03.2023 CN 202310249801**

(71) Applicants:
- **BioRay Pharmaceutical Co., Ltd.**
  **Jiaojiang District,**
  **Taizhou**
  **Zhejiang 318000 (CN)**
- **Bioray Pharmaceutical (Hangzhou) Co., Ltd.**
  **Hangzhou, Zhejiang 311404 (CN)**
- **Bioray Pharmaceutical Corp.**
  **San Diego, CA 92121 (US)**

(72) Inventors:
- **ZHAO, Xiaobei**
  **San Diego, CA 92121-2901 (US)**
- **ZHU, Jie**
  **San Diego, CA 92121-2901 (US)**
- **ZHOU, Yaqiong**
  **Zhejiang 318000 (CN)**
- **NIE, Lei**
  **Zhejiang 318000 (CN)**
- **WANG, Xinzeng**
  **Zhejiang 318000 (CN)**
- **WANG, Xiaoze**
  **Zhejiang 318000 (CN)**
- **XU, Tong**
  **Hangzhou, Zhejiang 311404 (CN)**
- **WU, Zhenhua**
  **Zhejiang 318000 (CN)**
- **MEI, Xiaofen**
  **Zhejiang 318000 (CN)**
- **CHEN, Juan**
  **Zhejiang 318000 (CN)**
- **CHEN, Xuchen**
  **Zhejiang 318000 (CN)**
- **SUN, Yan**
  **Hangzhou, Zhejiang 311404 (CN)**
- **CHEN, Gang**
  **San Diego, CA 92121-2901 (US)**
- **WANG, Haibin**
  **Hangzhou, Zhejiang 311404 (CN)**

(74) Representative: **Kador & Partner Part mbB**
**Corneliusstraße 15**
**80469 München (DE)**

Remarks:
The complete document including Reference Table(s) and the Sequence Listing(s) can be downloaded from the EPO website

# (54) ANTI-LIV-1 ANTIBODY AND ANTI-LIV-1 ANTIBODY-DRUG CONJUGATE, AND PHARMACEUTICAL USE THEREOF

(57) Provided are an anti-human-LIV-1 antibody or an antigen-binding fragment thereof, and an antibody-drug conjugate containing the anti-human-LIV-1 antibody or the antigen-binding fragment thereof. Also provided is the use of the anti-LIV-1 antibody or the antigen-binding fragment thereof and the antibody-drug conjugate or a pharmaceutically acceptable salt or solvate compound thereof.



Processed by Luminess, 75001 PARIS (FR)

## Description

TECHNICAL FIELD

The present disclosure relates to the field of antibody drugs. Specifically, the present disclosure discloses an anti-LIV-1 antibody or antigen-binding fragment thereof, a chimeric antibody or a humanized antibody comprising CDR regions of the anti-LIV-1 antibody, an anti-LIV-1 antibody-drug conjugate or a pharmaceutically acceptable salt or solvate thereof, and use thereof as an anti-cancer medicament.

BACKGROUND

The statement herein merely provides background information related to the present disclosure to help the understanding of the present disclosure, and may not necessarily constitute the prior art.

All publications, patents, and patent applications described herein are incorporated by reference to the same extent as if each individual publication, patent, or patent application was specifically and individually incorporated by reference. In the event that the definition or use of a term in an incorporated publication, patent, or patent application is inconsistent with or contrary to the definition of the term provided herein, the definition of the term provided herein shall prevail.

Zinc transporter ZIP6, also known as SLC39A6 or LIV-1, is a multiple transmembrane protein with zinc transporter and metalloprotease activities. LIV-1 is regulated by estrogen and highly expressed in estrogen receptor-positive breast cancer (Taylor et al., Mol Med, 2007 13(7-8): 396-406). Recent studies have found that LIV-1 is highly expressed in various tumor cells such as breast cancer, prostate cancer, pancreatic cancer, cervical cancer, and liver cancer, but is limitedly expressed in normal tissues, making it a promising candidate for ADC therapy and very likely to become a prognostic and detection indicator for certain cancers. There are a variety of variants of LIV-1, among which the variant numbered Swiss Prot Q13433 (SEQ ID NO: 25) is a representative target for screening anti-LIV-1 antibodies.

Antibody-drug conjugate (ADC) technology is one that accurately delivers anti-tumor drugs (e.g., cytotoxic agents, cytostatic agents, small molecule chemotherapeutic agents, etc.) to target tumor cells by utilizing the ability of antibodies to specifically recognize specific antigens on the surface of the tumor cells, causing the anti-tumor drugs to be endocytosed and released, thereby accurately killing tumors. Antibody-drug conjugates typically consist of three components: an antibody or antibody-like ligand, a small molecule drug, and a linker that conjugates the antibody or antibody-like ligand to the drug. Antibody-drug conjugates have been considered as the most promising anti-tumor drugs due to their proper molecular weight, high stability, strong targeting property, and low toxic and side effects.

Anti-LIV-1 ADCs have been used to treat cancer; for example, an anti-LIV-1 antibody is linked to an anti-cancer drug via a maleimide linker (see US RE48,959). However, this linkage mode has disadvantages such as easy detachment and poor stability, which limits the use of the drug and increases side effects.

Thus, there is an urgent need in the art to provide research and development on a novel antibody-drug conjugate targeting LIV-1 that improves serum stability and reduces toxicity.

SUMMARY

In one aspect, the present disclosure provides an anti-LIV-1 antibody or antigen-binding fragment thereof. The antibody or antigen-binding fragment thereof specifically binds to an extracellular domain (SEQ ID NO: 25) consisting of amino acids 29-325 of the LIV-1 protein. The anti-LIV-1 antibody or antigen-binding fragment thereof is an antibody A, an antibody B, or an antibody C.

With respect to specific sequences, the anti-LIV-1 antibody or antigen-binding fragment thereof comprises a heavy chain variable region and a light chain variable region, wherein the heavy chain variable region and the light chain variable region comprise heavy chain complementarity determining regions 1 to 3 (CDR-H1, CDR-H2, and CDR-H3) and light chain complementarity determining regions 1 to 3 (CDR-L1, CDR-L2, and CDR-L3) shown below, respectively, wherein:

the heavy chain variable region of the antibody A comprises CDR-H1, CDR-H2, and CDR-H3 having the amino acid sequences set forth in SEQ ID NO: 7, SEQ ID NO: 8, and SEQ ID NO: 9, respectively; and the light chain variable region comprises CDR-L1, CDR-L2, and CDR-L3 having the amino acid sequences set forth in SEQ ID NO: 10, SEQ ID NO: 11, and SEQ ID NO: 12, respectively;

the heavy chain variable region of the antibody B comprises CDR-H1, CDR-H2, and CDR-H3 having the amino acid sequences set forth in SEQ ID NO: 13, SEQ ID NO: 14, and SEQ ID NO: 15, respectively; and the light chain variable region comprises CDR-L1, CDR-L2, and CDR-L3 having the amino acid sequences set forth in SEQ ID NO: 16, SEQ

ID NO: 17, and SEQ ID NO: 18, respectively;

the heavy chain variable region of the antibody C comprises CDR-H1, CDR-H2, and CDR-H3 having the amino acid sequences set forth in SEQ ID NO: 19, SEQ ID NO: 20, and SEQ ID NO: 21, respectively; and the light chain variable region comprises CDR-L1, CDR-L2, and CDR-L3 having the amino acid sequences set forth in SEQ ID NO: 22, SEQ ID NO: 23, and SEQ ID NO: 24, respectively.

The CDR sequences of the above antibodies are obtained according to the Kabat numbering scheme. However, the CDR sequences of the antibodies of the present disclosure can also be obtained by those skilled in the art according to other known numbering schemes (e.g., IMGT, Chothia, etc.).

In some embodiments, the anti-LIV-1 antibody or antigen-binding fragment thereof comprises a heavy chain and a light chain, and the heavy chain and the light chain comprise a heavy chain variable region (VH) and a light chain variable region (VL) shown below, respectively, wherein:

the VH sequence of the antibody A comprises the amino acid sequence set forth in SEQ ID NO: 1 or a variant thereof, and the VL sequence comprises the amino acid sequence set forth in SEQ ID NO: 2 or a variant thereof;

the VH sequence of the antibody B comprises the amino acid sequence set forth in SEQ ID NO: 3 or a variant thereof, and the VL sequence comprises the amino acid sequence set forth in SEQ ID NO: 4 or a variant thereof;

the VH sequence of the antibody C comprises the amino acid sequence set forth in SEQ ID NO: 5 or a variant thereof, and the VL sequence comprises the amino acid sequence set forth in SEQ ID NO: 6 or a variant thereof.

In some embodiments, the anti-LIV-1 antibody or antigen-binding fragment thereof comprises a heavy chain and a light chain, and the heavy chain and the light chain comprise a heavy chain variable region (VH) and a light chain variable region (VL) shown below, respectively, wherein:
the VH sequence of the antibody comprises the amino acid sequence set forth in SEQ ID NO: 32 or a variant thereof, and the VL sequence comprises the amino acid sequence set forth in SEQ ID NO: 2 or a variant thereof.

In the context of the present disclosure, a "variant of an amino acid sequence" refers to an amino acid sequence having at least 75% sequence identity (any percent identity $\geq$ 75%, e.g., at least 80%, preferably at least 85%, more preferably at least 90%, further preferably at least 91%, 92%, 93%, 94%, 95%, 96%, 97%, 98%, or even 99% identity, etc.) to the amino acid sequence.

In some embodiments, the anti-LIV-1 antibody or antigen-binding fragment thereof comprises a heavy chain and a light chain, and the heavy chain and the light chain comprise a heavy chain variable region (VH) and a light chain variable region (VL) shown below, respectively, wherein:

the VH of the antibody A comprises a sequence having at least 95% identity to the amino acid sequence of SEQ ID NO: 1, and the VL of the antibody A comprises a sequence having at least 95% identity to the amino acid sequence of SEQ ID NO: 2;

the VH of the antibody A comprises a sequence having at least 95% identity to the amino acid sequence of SEQ ID NO: 32, and the VL of the antibody A comprises a sequence having at least 95% identity to the amino acid sequence of SEQ ID NO: 2;

the VH of the antibody B comprises a sequence having at least 95% identity to the amino acid sequence of SEQ ID NO: 3, and the VL of the antibody B comprises a sequence having at least 95% identity to the amino acid sequence of SEQ ID NO: 4;

the VH of the antibody C comprises a sequence having at least 95% identity to the amino acid sequence of SEQ ID NO: 5, and the VL of the antibody C comprises a sequence having at least 95% identity to the amino acid sequence of SEQ ID NO: 6.

In some embodiments, the anti-LIV-1 antibody or antigen-binding fragment thereof may be a tetramer comprising two light chains and two heavy chains, an antibody comprising one or more heavy chains or light chains, a Fab, a Fab', a F(ab')2, and an Fv, or may be a single-chain antibody.

In particular, the anti-LIV-1 antibody or antigen-binding fragment thereof of the present disclosure comprises at least a heavy chain variable region and a light chain variable region, both of which comprise the above CDRs and interspersed framework regions (FRs), and the domains are arranged as follows: FR1-CDR1-FR2-CDR2-FR3-CDR3-FR4. Thus, with respect to the heavy chain variable region and the light chain variable region contained in the anti-LIV-1 antibody or fragment thereof of the present disclosure, the "at least 75% sequence identity" resulting in up to 25% difference in amino acid sequence may exist in any framework region within the heavy chain variable region or light chain variable region. Alternatively, with respect to the entirety of the anti-LIV-1 antibody or fragment thereof of the present disclosure, the up to 25% difference may be present in any domain or sequence other than the heavy chain variable region and the light chain variable region in the antibody or the antigen-binding fragment thereof of the present disclosure. The difference may result from amino acid deletions, additions, or substitutions at any position, wherein the substitutions may be conservative or non-conservative substitutions.

Preferably, the anti-LIV-1 antibody or antigen-binding fragment thereof provided by the present disclosure may further comprise a constant region. Preferably, the anti-LIV-1 antibody or antigen-binding fragment thereof further comprises a human or murine heavy chain constant region (CH) and/or light chain constant region (CL), more preferably a heavy chain constant region selected from IgG, IgA, IgM, IgD, and IgE, and/or a κ-type or λ-type light chain constant region.

Preferably, the antibody is a monoclonal antibody, preferably a murine, chimeric, or humanized monoclonal antibody; more preferably, the heavy chain constant region of the monoclonal antibody is IgG1 or IgG4 subtype, and the light chain constant region is κ type. Alternatively, for example, the antibody is an immunoglobulin, specifically IgA, IgD, IgE, IgG, or IgM, e.g., a human subtype of IgA, IgD, IgE, IgG, or IgM, and more preferably the human IgG1, IgG2, IgG3, or IgG4 subtype.

According to a specific embodiment of the present disclosure, the anti-LIV-1 antibody or the antigen-binding fragment thereof comprises a heavy chain constant region, and the heavy chain constant region comprises the amino acid sequence set forth in SEQ ID NO: 26 or a variant thereof. Alternatively, the anti-LIV-1 antibody or antigen-binding fragment thereof comprises a light chain constant region, and the light chain constant region comprises the amino acid sequence set forth in SEQ ID NO: 27 or a variant thereof. As defined above, a "variant of an amino acid sequence" refers to an amino acid sequence having at least 75% sequence identity to the amino acid sequence.

In some embodiments, the anti-LIV-1 antibody described above comprises a light chain set forth in SEQ ID NO: 30 and a heavy chain set forth in SEQ ID NO: 31.

According to the above antibody provided by the present disclosure, the present disclosure provides a nucleic acid molecule encoding the anti-LIV-1 antibody or antigen-binding fragment thereof described in the present disclosure.

The nucleic acid molecule of the present disclosure can be cloned into a vector, which in turn transforms or transfects a host cell. Thus, in another aspect, the present disclosure provides a vector comprising the nucleic acid molecule of the present disclosure. The vector may be a eukaryotic expression vector, a prokaryotic expression vector, an artificial chromosome, a phage vector, or the like.

The vector or the nucleic acid molecule of the present disclosure can be used to transform or transfect a host cell or enter the host cell in any way for the purpose of preserving or expressing the antibody. Thus, in yet another aspect, the present disclosure provides a host cell, wherein the host cell comprises the nucleic acid molecule and/or the vector of the present disclosure, or the host cell is transformed or transfected with the nucleic acid molecule and/or the vector of the present disclosure. The host cell may be any prokaryotic or eukaryotic cell, such as a bacterial cell, an insect cell, a fungal cell, a plant cell, or an animal cell (e.g., a CHO cell).

The anti-LIV-1 antibody or antigen-binding fragment thereof provided by the present disclosure can be obtained using any method known in the art. For example, the heavy chain variable region and/or the light chain variable region of the antibody, or the heavy chain and/or the light chain of the antibody molecule, may be first obtained from the nucleic acid molecule provided by the present disclosure, and then assembled with optional other domains of the antibody molecule to form the antibody; or the host cell is cultured under conditions that allow the host cell provided by the present disclosure to express the heavy chain variable region and/or the light chain variable region of the antibody molecule or the heavy chain and/or the light chain of the antibody molecule for assembly into the antibody. Optionally, the method further comprises a step of recovering the produced antibody molecule.

In addition, the present disclosure further provides a multispecific antibody, such as a bispecific antibody, comprising the aforementioned anti-LIV-1 antibody or antigen-binding fragment thereof. The multispecific antibody simultaneously targets an LIV-1 antigen and a non-LIV-1 antigen, wherein the non-LIV-1 antigen is selected from CD3, EGFR, HER2, HER3, PD-L1, c-MET, TROP-2, CEA5, B7-H3, SIRPα, PSMA, ROR1, CD47, etc., and a combination thereof.

In another aspect, the present disclosure provides an antibody-drug conjugate targeting LIV-1 or a pharmaceutically acceptable salt thereof, which comprises an anti-LIV-1 antibody or antigen-binding fragment thereof covalently linked to a drug, and the drug linked to the antibody by a linker. The antibody-drug conjugate or the pharmaceutically acceptable salt thereof comprises the following structure (formula I):

[Formula I] Ab-[(AG)k-(L1)x-(OP)p-(L2)y-D]z, wherein:

Ab represents any one of the aforementioned anti-human LIV-1 antibodies or antigen-binding fragments thereof;

AG is a conjugating group, wherein k is 0 or 1; AG is linked to L1; AG is selected from succinimidyl (SUCC), acetyl, and carbonyl, and corresponds to the structures shown below:

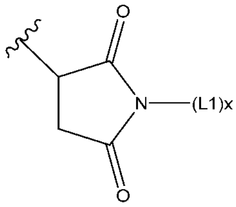 , 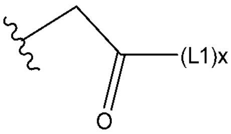 , or 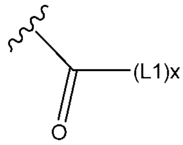 ,

wherein the wavy line indicates the linkage to the antibody;

(L1)x is a first linker group, wherein x is 0 or 1, and L1 represents a chain containing 1 to 18 groups selected from $-CH_2-$, -C(=O)-, -NH-, -O-, and -S-, wherein each NH, O, and S is separated from another NH, O, or S by at least two carbon atoms from $-CH_2-$ and/or -C(=O)-;

(OP)p is an enzyme-cleavable oligopeptide, wherein p is the number of condensed amino acids of the oligopeptide, and p is 0 or 2-10, preferably 0, 2, or 3;

(L2)y is a second linker group, wherein y is 0 or 1.

In a first embodiment, L2 represents a chain containing 1 to 18 groups selected from $-CH_2-$, -C(=O)-, -NH-, -O-, and -S-, and each NH, O, and S is separated from another NH, O, or S by at least two groups selected from $-CH_2-$ and/or -C(=O)-.

In a second embodiment, L2 represents o-, m-, or p-hydroxyphenyl, or o-, m-, or p-aminophenyl.

In a third embodiment, L2 represents a combination of the chain from the first embodiment and the group from the second embodiment.

D is the drug of the antibody-drug conjugate represented by the formula [Formula I], and D may be any biologically active moiety, such as a cytotoxic compound, an immunomodulator, or an enzyme or hormone inhibitor, preferably a cytotoxic compound.

Each antibody may be linked to one or more (AG)k-(L1)x-(OP)p-(L2)y-D groups. The drug-to-antibody ratio is z with a value of a decimal or integer of 1-24. In some embodiments, z is 3.5. In some embodiments, z is 4. In some embodiments, z is 3.5-4. In some embodiments, z is 2-8.

In another preferred embodiment, L1 is selected from $-(CH_2-)o-C(=O)-$, wherein o is 1-10, preferably 1-7, and more preferably 2, 3, 4, or 5; or L1 is selected from $-(NH)j-(CH_2CH_2O)n-(CH_2)q-(C=O)-$, wherein j is 0-4, preferably 0 or 1, n is 2-8, preferably 4, and q is 1-6, preferably 2.

In another preferred embodiment, L1 is selected from $-(CH_2)_2-C(=O)-$, $-(CH_2)_3-C(=O)-$, $-(CH_2)_4-C(=O)-$, and $-(CH_2)_5-C(=O)-$.

In another preferred embodiment, L1 is selected from $-(CH_2-CH_2-O)_4-CH_2-CH_2-C=O-$ and $-NH-(CH_2CH_2O)_4-(CH_2)_2-(C=O)-$.

In another preferred embodiment, x is 0, i.e., L1 is absent, and the AG group and the (OP)p group are directly linked.

In another preferred embodiment, the enzyme in the "enzyme-cleavable oligopeptide" is a cathepsin or β-glucuronidase.

Preferably, the enzyme in the "enzyme-cleavable oligopeptide" is a cathepsin, wherein the cathepsin-cleavable oligopeptide is selected from the group consisting of: ValCit, ValAla, AlaAlaAsn, GlyGlyPhe, GlyGlyTyr, GlyGlyPheGly, and derivatives thereof, wherein a hydroxyl group of tyrosine or a terminal amino group of asparagine may be optionally substituted with galactosyl, glucosyl, or 1-deoxy-2-acetamido-2-deoxy-glucosyl (glycosylation).

The "glycosylation" refers to the transfer of a glycosyl or oligosaccharide group to a hydroxyl or amino group of a compound by chemical or enzymatic methods.

In one embodiment, the glycosylation refers to a structural modification of amino acid residues in the oligopeptide (OP)p by reacting certain groups (e.g., a phenolic hydroxyl group of tyrosine or an amide group of asparagine) or a hydroxyl substituent on the benzene ring in L2 with a saccharide.

In another preferred embodiment, (OP)p is selected from oligopeptides formed from a combination of valine, citrulline, alanine, glycine, asparagine, tyrosine, phenylalanine, proline, isoleucine, lysine, serine, glutamic acid, threonine, or asparagine, such as dipeptides, tripeptides, or tetrapeptides formed; optionally, the phenolic hydroxyl group of tyrosine or the amide group of asparagine is glycosylated, preferably with glucuronic acid, N-acetylglucosamine (GlcNAc), glucose, or galactose.

In another preferred embodiment, (OP)p is a dipeptide formed from valine and citrulline. In some embodiments, (OP)p is -valine-citrulline-dipeptide.

In another preferred embodiment, (OP)p is a tripeptide formed from glycine and tyrosine; optionally, the phenolic hydroxyl group of tyrosine is glycosylated. In some embodiments, (OP)p is -glycine-glycine-tyrosine-tripeptide; optionally, the phenolic hydroxyl group of tyrosine is glycosylated.

In another preferred embodiment, (OP)p is a dipeptide formed from alanine and asparagine, wherein the amide group of asparagine is glycosylated.

In another preferred embodiment, p is 0, i.e., (OP)p is absent, and the (L1)x group and the (L2)y group are directly linked.

In another preferred embodiment, y in (L2)y is 1, and L2 represents: -NH-Ph-$CH_2$-O-C(=O)-, wherein the benzene ring optionally contains a hydroxyl substituent; more preferably, L is p-aminobenzyloxycarbonyl or p-hydroxy-m-aminobenzyloxycarbonyl; optionally, the hydroxyl group on the benzene ring is glycosylated, preferably with glucuronic acid, N-acetylglucosamine (GlcNAc), glucose, or galactose.

In one embodiment, L2 represents p-hydroxy-m-aminobenzyloxycarbonyl, wherein the hydroxyl group on the benzene ring is glycosylated with glucuronic acid, N-acetylglucosamine (GlcNAc), glucose, or galactose.

In some embodiments, the antibody-drug conjugate and the pharmaceutically acceptable salt thereof comprise a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is succinimidyl; L1 is -($CH_2$-)o-C(=O)-, wherein o is 1-7; (OP)p is -valine-citrulline-dipeptide, -glycine-glycine-tyrosine-tripeptide, or -alanine-alanine-asparagine-tripeptide, and optionally, the phenolic hydroxyl group of tyrosine or the amide group of asparagine is glycosylated with glucose, N-acetylglucosamine, or galactose; L2 is p-aminobenzyloxycarbonyl (PAB); and k is 1, x is 1, y is 1, and D and z are as previously defined.

In some embodiments, AG is succinimidyl; L1 is -($CH_2$)o-C(=O)-, wherein o is 3 or 5; and (OP)p is -valine-citrulline-dipeptide.

In some embodiments, AG is succinimidyl; L1 is -($CH_2$)o-C(=O)-, wherein o is 3 or 5; and (OP)p is -glycine-glycine-tyrosine-tripeptide.

In some embodiments, AG is succinimidyl; L1 is -($CH_2$)o-C(=O)-, wherein o is 3 or 5; and (OP)p is -glycine-glycine-tyrosine-tripeptide, wherein the phenolic hydroxyl group of tyrosine is glycosylated with galactose or glucose.

In some embodiments, AG is succinimidyl; L1 is -($CH_2$)o-C(=O)-, wherein o is 3 or 5; and (OP)p is alanine-alanine-asparagine-tripeptide, wherein the amide group of asparagine is glycosylated with glucose.

In some embodiments, the antibody-drug conjugate and the pharmaceutically acceptable salt thereof comprise a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -$CH_2$-C(=O)-; L1 is -(NH)j-($CH_2CH_2O$)n-($CH_2$)q-(C=O)-, wherein j is 1-4, n is 2-8, and q is 1-6; (OP)p is -valine-citrulline-tripeptide or -glycine-glycine-tyrosine-tripeptide, and optionally, the phenolic hydroxyl group of tyrosine is glycosylated with glucose or galactose; L2 is p-aminobenzyloxycarbonyl (PAB) or hydroxy-m-aminobenzyloxycarbonyl, wherein optionally, the hydroxyl group on the benzene ring is glycosylated with glucuronic acid, N-acetylglucosamine (GlcNAc), glucose, or galactose; and k is 1, x is 0 or 1, y is 1, and D and z are as previously defined.

In some embodiments, the antibody-drug conjugate and the pharmaceutically acceptable salt thereof comprise a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -$CH_2$-C(=O)-; L1 is -(NH)j-($CH_2CH_2O$)n-($CH_2$)q-(C=O)-, wherein j is 1-4, n is 2-8, and q is 1-6; p is 0; L2 is p-aminobenzyloxycarbonyl (PAB) or hydroxy-m-aminobenzyloxycarbonyl, wherein optionally, the hydroxyl group on the benzene ring is glycosylated with glucuronic acid, N-acetylglucosamine (GlcNAc), glucose, or galactose; and k is 1, x is 0 or 1, y is 1, and D and z are as previously defined.

In some embodiments, L1 is -NH-$(CH_2CH_2O)_4$-$(CH_2)_2$-(C=O)-.

In some embodiments, (OP)p is -valine-citrulline-dipeptide.

In some embodiments, (OP)p is -glycine-glycine-tyrosine-tripeptide.

In some embodiments, (OP)p is -glycine-glycine-tyrosine-tripeptide, wherein the phenolic hydroxyl group of tyrosine is glycosylated with glucose.

In some embodiments, p is 0, i.e., (OP)p is absent, and the (L1)x group and the (L2)y group are directly linked.

In some embodiments, L2 is p-hydroxy-m-aminobenzyloxycarbonyl, wherein the hydroxyl group on the benzene ring is glycosylated with glucuronic acid.

In some embodiments, the antibody-drug conjugate and the pharmaceutically acceptable salt thereof comprise a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-; L1 is $-(CH_2-)o-C(=O)-$, wherein o is 1-7, or L1 is $-(NH)j-(CH_2CH_2O)n-(CH_2)q-(C=O)-$, wherein j is 0-4, n is 2-8, and q is 1-6; (OP)p is - valine-citrulline-dipeptide; L2 is PAB; and k is 1, x is 1, y is 1, and D and z are as previously defined.

In some embodiments, L1 is $-(CH_2)_2-C(=O)-$.

In some embodiments, L1 is $-(CH_2)_4-C(=O)-$.

In some embodiments, L1 is $-(CH_2CH_2O)_4-(CH_2)_2-(C=O)-$.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; x is 0; (OP)p is -valine-citrulline-dipeptide; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; x is 0; (OP)p is -glycine-glycine-tyrosine-tripeptide, wherein the phenolic hydroxyl group of tyrosine is glycosylated with glucose; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; x is 0; p is 0; L2 is p-hydroxy-m-aminobenzyloxycarbonyl, wherein the hydroxyl group on the benzene ring is glycosylated with glucuronic acid, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; L1 is $-NH-(CH_2-CH_2-O)_4-CH_2-CH_2-C=O-$, and x is 1; (OP)p is -valine-citrulline-dipeptide; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof;

AG is -C(=O)-, and k is 1; L1 is $-NH-(CH_2-CH_2-O)_4-CH_2-CH_2-C=O-$, and x is 1; p is 0; L2 is p-hydroxy-m-aminobenzyloxycarbonyl, wherein the hydroxyl group on the benzene ring is glycosylated with glucuronic acid, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is succinimidyl, and k is 1; L1 is $-(CH_2)_5-C(=O)-$, and x is 1; (OP)p is -valine-citrulline-dipeptide; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is succinimidyl, and k is 1; L1 is $-(CH_2)_5-C(=O)-$, and x is 1; (OP)p is -glycine-glycine-tyrosine-tripeptide, wherein the phenolic hydroxyl group of tyrosine is glycosylated with galactose; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is succinimidyl, and k is 1; L1 is $-(CH_2)_5-C(=O)-$, and x is 1; (OP)p is -glycine-glycine-tyrosine-tripeptide; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is succinimidyl, and k is 1; L1 is $-(CH_2)_5-C(=O)-$, and x is 1; (OP)p is -glycine-glycine-tyrosine-tripeptide, wherein the phenolic hydroxyl group of tyrosine is glycosylated with glucose; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is succinimidyl, and k is 1; L1 is $-(CH_2)_3-C(=O)-$, and x is 1; (OP)p is -alanine-alanine-asparagine-tripeptide, wherein the amide group of asparagine is glycosylated with glucose; L2 is p-aminobenzyloxycarbonyl, and y is 1; D is a drug; z is a decimal or integer of 1-10, preferably 3.5-4.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; L1 is $-(CH_2)_2-C(=O)-$, and x is 1; (OP)p is -valine-citrulline-dipeptide; L2 is p-aminobenzyloxycarbonyl, and y is 1; D is a drug; z is a decimal or integer of 1-10, preferably 1-2.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; L1 is $-(CH_2)_4-C(=O)-$, and x is 1; (OP)p is -valine-citrulline-dipeptide; L2 is p-aminobenzyloxycarbonyl, and y is 1; D is a drug; z is a decimal or integer of 1-10, preferably 1-2.

In one embodiment, the antibody-drug conjugate or the pharmaceutically acceptable salt thereof comprises a structure represented by [Formula I] below:

[Formula I] Ab-[(AG)k-(L1)x-(OP)p-(L2)y-D]z

wherein Ab represents any one of the aforementioned anti-human LIV-1 antibodies or antigen-binding fragments thereof; AG is -C(=O)-, and k is 1; L1 is $-(CH_2-CH_2-O)_4-(CH_2)_2-C(=O)-$, and x is 1; (OP)p is -valine-citrulline-dipeptide; L2 is p-aminobenzyloxycarbonyl, and y is 1; D is a drug; z is a decimal or integer of 1-10, preferably 1-2.

In some preferred embodiments, -(AG)k-(L1)x-(OP)p-(L2)y- has the following structure, wherein "1" indicates the linkage to the antibody Ab, and "2" indicates the linkage to the drug D.

| | |
|---|---|
| 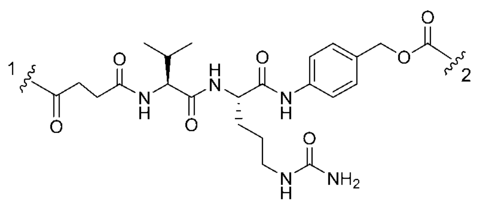 | 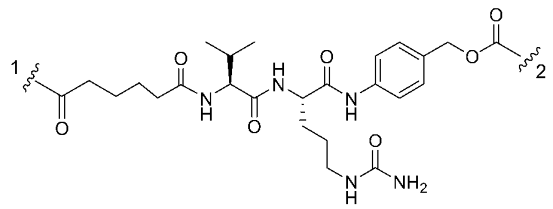 |
| 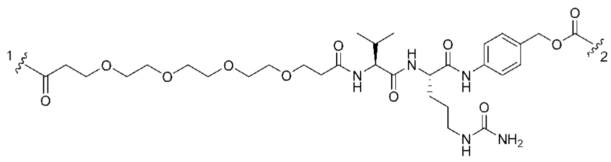 | 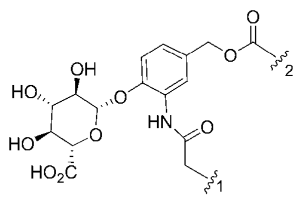 |
| 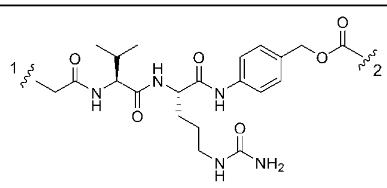 | 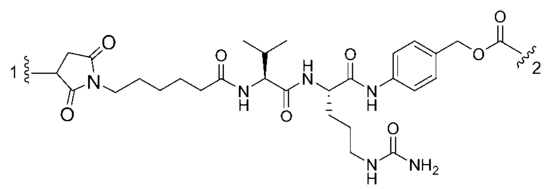 |
| 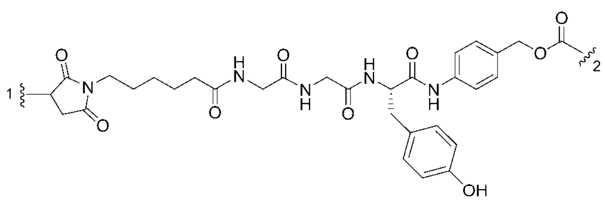 | 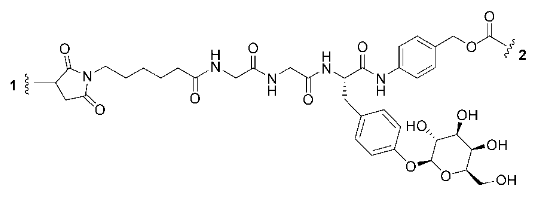 |
| 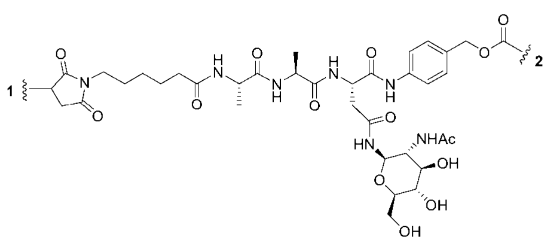 | 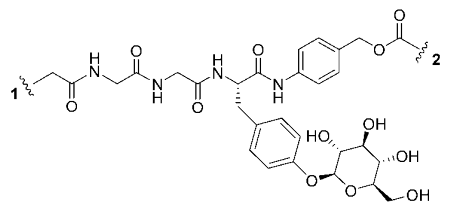 |

| | |
|---|---|
| 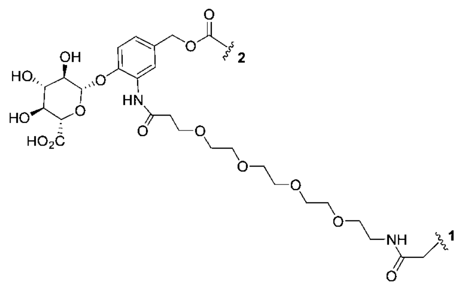 | 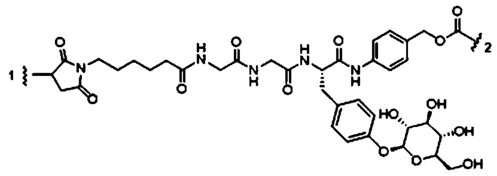 |
| 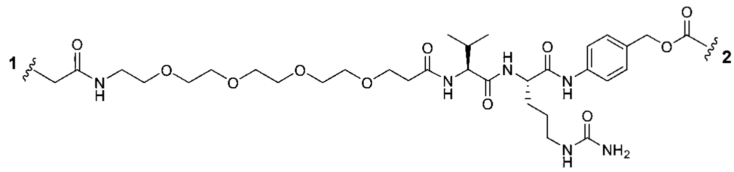 | |

In another preferred embodiment, the cytotoxic compound is a DNA-alkylating compound or a compound that inhibits topoisomerase-1, topoisomerase-2, tubulin, or polymerization by RNA polymerase. Alternatively, the cytotoxic compound methylates DNA. The drug is selected from monomethyl auristatin E (MMAE), monomethyl auristatin F (MMAF), maytansine, SN-38, and exatecan.

In another preferred embodiment, [(AG)k-(L1)x-(OP)p-(L2)y-D] in [Formula I] has the following structure:

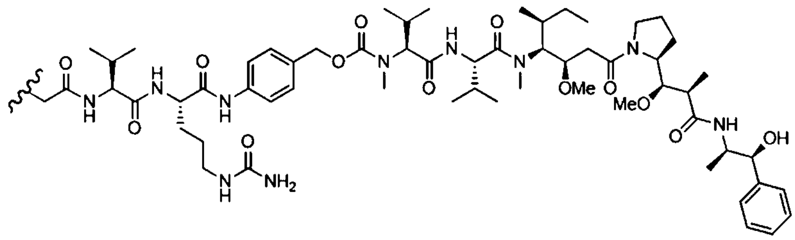

,

wherein the wavy line indicates the linkage to the antibody Ab.

In another preferred embodiment, [(AG)k-(L1)x-(OP)p-(L2)y-D] in [Formula I] has the following structure:

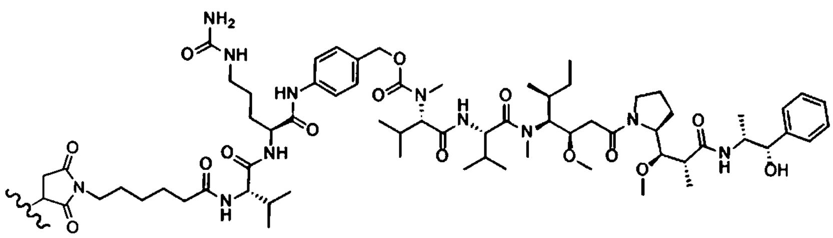

,

wherein the wavy line indicates the linkage to the antibody Ab.

In some embodiments, the antibody-drug conjugate of [Formula I] and the pharmaceutically acceptable salt thereof have a structure selected from the following:

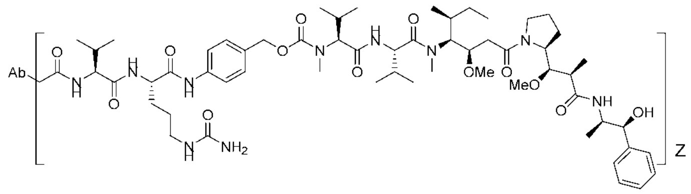

,

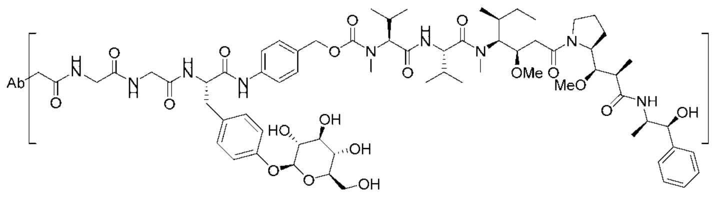

,

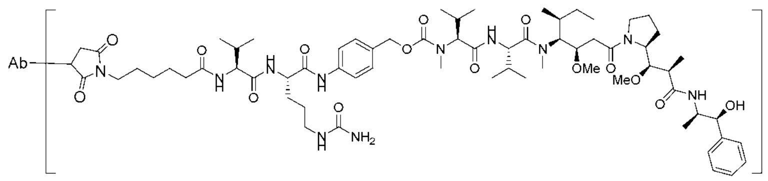

,

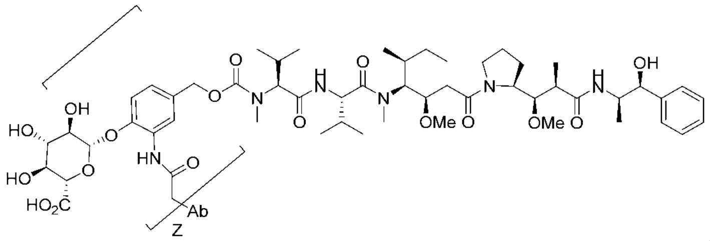

,

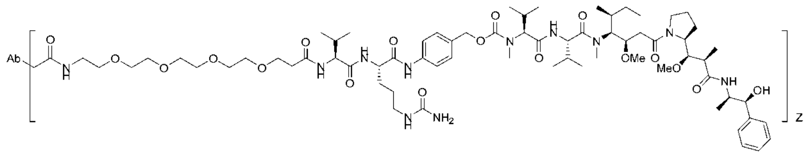

,

and

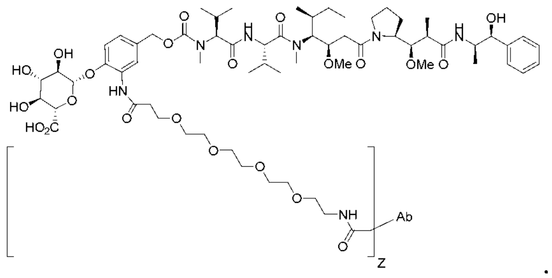

.

In some embodiments, the VH of Ab comprises three CDRs, CDR-H1, CDR-H2, and CDR-H3, wherein:

the CDR-H1 comprises the amino acid sequence set forth in SEQ ID NO: 7;

the CDR-H2 comprises the amino acid sequence set forth in SEQ ID NO: 8;

the CDR-H3 comprises the amino acid sequence set forth in SEQ ID NO: 9;

and the VL of Ab comprises three CDRs, CDR-L1, CDR-L2, and CDR-L3, wherein:

the CDR-L1 comprises the amino acid sequence set forth in SEQ ID NO: 10;

the CDR-L2 comprises the amino acid sequence set forth in SEQ ID NO: 11;

the CDR-L3 comprises the amino acid sequence set forth in SEQ ID NO: 12.

In some embodiments, the VH of Ab comprises the amino acid sequence of SEQ ID NO: 1, and the VL of Ab comprises the amino acid sequence of SEQ ID NO: 2.

In some embodiments, Ab comprises a light chain set forth in SEQ ID NO: 30 and a heavy chain set forth in SEQ ID NO: 31, and z is a decimal or integer of 2-8. In another preferred embodiment, the antibody moiety of the antibody-drug conjugate or the salt thereof is a multispecific antibody comprising the aforementioned anti-LIV-1 antibody or antigen-binding fragment thereof. For example, the antibody is a bivalent tetramer comprising two light chains and two heavy chains, wherein a first pair of heavy and light chains comprises the heavy chain variable region and the light chain variable region of the aforementioned anti-LIV-1 antibody or antigen-binding fragment thereof, and a second pair of heavy or light chains of the tetramer is capable of binding to a non-LIV-1 antigen, wherein the non-LIV-1 antigen is selected from CD3, EGFR, HER2, HER3, PD-L1, c-MET, TROP-2, CEA5, B7-H3, SIRP$\alpha$, PSMA, ROR1, CD47, and the like.

The antibody-drug conjugate targeting LIV-1 or the pharmaceutically acceptable salt thereof, the anti-LIV-1 antibody or the antigen-binding fragment thereof, the nucleic acid molecule, the vector, or the host cell provided by the present disclosure may be comprised in a composition, more particularly in a pharmaceutical formulation, for various purposes according to actual needs. Thus, in another aspect, the present disclosure further provides a composition comprising the antibody-drug conjugate targeting LIV-1 or the pharmaceutically acceptable salt thereof, the anti-LIV-1 antibody or the antigen-binding fragment thereof, the nucleic acid molecule, the vector, and/or the host cell provided by the present disclosure. Preferably, the composition is a pharmaceutical composition, which optionally further comprises a pharmaceutically acceptable carrier, auxiliary material, or excipient.

The present disclosure provides use of the antibody-drug conjugate targeting LIV-1 or the pharmaceutically acceptable salt thereof, the anti-LIV-1 antibody or antigen-binding fragment thereof, the nucleic acid molecule, the vector, the host cell, and/or the composition as a medicament (i.e., for therapeutic use).

In another aspect, the present disclosure further provides use of the antibody-drug conjugate targeting LIV-1 or the pharmaceutically acceptable salt thereof, the anti-LIV-1 antibody or the antigen-binding fragment thereof, the nucleic acid molecule, the vector, the host cell, and/or the composition in the preparation of a medicament for treating a tumor or cancer. Alternatively, the present disclosure further provides a method for treating a tumor, which comprises administering to a subject in need thereof the antibody-drug conjugate targeting LIV-1 or the pharmaceutically acceptable salt thereof, the anti-LIV-1 antibody or the antigen-binding fragment thereof, the nucleic acid molecule, the vector, the host cell, and/or the composition provided by the present disclosure, wherein the subject is a mammalian animal, preferably a primate, and more preferably a human.

In some embodiments, the antibody-drug conjugate or the pharmaceutically acceptable salt thereof, the anti-LIV-1 antibody or the antigen-binding fragment thereof, the nucleic acid molecule, the vector, the host cell, and/or the composition of the present disclosure may be used as a medicament; in some embodiments, they may be used as a medicament for treating a tumor or cancer.

In some embodiments, the tumor or cancer is a tumor or cancer associated with high expression of LIV-1. In some embodiments, the tumor or cancer is any one selected from the following: bladder cancer, breast cancer, ovarian cancer, pancreatic cancer, hepatocellular carcinoma, gastric cancer, non-Hodgkin's lymphoma, Hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, colorectal cancer, glioma, mesothelioma, cervical cancer, triple-negative breast cancer, lung cancer, head and neck cancer, esophageal cancer, skin cancer, and uterine cancer.

In some embodiments, the tumor or cancer is a solid tumor.

In some embodiments, the tumor or cancer is selected from the group consisting of breast cancer, bladder cancer, ovarian cancer, pancreatic cancer, hepatocellular carcinoma, gastric cancer, multiple myeloma, prostate cancer, non-small cell lung cancer, small cell lung cancer, malignant melanoma, squamous cell carcinoma, glioblastoma, renal cell carcinoma, gastrointestinal tumors, colorectal cancer, glioma, mesothelioma, cervical cancer, triple-negative breast cancer, lung cancer, head and neck cancer, esophageal cancer, skin cancer, and uterine cancer.

In some embodiments, the tumor or cancer is a hematological tumor.

In some embodiments, the tumor or cancer is selected from the group consisting of non-Hodgkin's lymphoma, Hodgkin's lymphoma, acute lymphocytic leukemia, and anaplastic large cell lymphoma.

In some embodiments, the tumor or cancer is selected from the group consisting of lung cancer, breast cancer, and prostate cancer.

In some embodiments, the tumor or cancer is breast cancer, particularly triple-negative breast cancer.

In another aspect, the present disclosure provides an immunoconjugate comprising:

(a) the antibody or the antigen-binding fragment thereof described in the present disclosure; and

(b) a conjugated moiety selected from the group consisting of a detectable label, a radionuclide, a cytokine, an enzyme, a gold nanoparticle/nanorod, a magnetic nanoparticle, a viral capsid protein, a VLP, and a combination

thereof.

In another preferred embodiment, the radionuclide comprises:

(i) a diagnostic isotope, which is selected from the group consisting of Tc-99m, Ga-68, F-18, 1-123, 1-125, 1-131, In-111, Ga-67, Cu-64, Zr-89, C-11, Lu-177, Re-188, and a combination thereof; and/or

(ii) a therapeutic isotope, which is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and a combination thereof.

In another preferred embodiment, the detectable label is selected from the group consisting of a fluorescent or luminescent label, a radioactive label, an MRI (magnetic resonance imaging) or CT (electronic computer tomography) contrast agent, and an enzyme capable of producing a detectable product.

Thus, the immunoconjugate of the present disclosure may be used to detect LIV-1 in a sample and thus for tumor or cancer diagnosis. Based on this, the present disclosure further provides a method for detecting LIV-1 in a sample (including diagnostic and non-diagnostic methods), which comprises the steps of: (1) contacting the sample with the immunoconjugate described in the present disclosure; and (2) detecting whether an antigen-antibody complex is formed, wherein the formation of the complex indicates the presence of LIV-1 in the sample.

It should be understood that within the scope of the present disclosure, the above technical features of the present disclosure and those specifically described below (e.g., in the examples) may be combined with each other to form new or preferred technical schemes. Due to limited space, such schemes are not described herein.

DESCRIPTION OF DRAWINGS

FIG. 1A shows the binding activity of the antibody A to human LIV-1 as detected by ELISA.

FIG. 1B shows the binding activity of the antibody B and antibody C to human LIV-1 as detected by ELISA.

FIG. 2 shows the binding activity of the antibody-drug conjugate A-BrAcMMAE to human LIV-1 as detected by ELISA.

FIG. 3 shows the inhibitory activity of ADCs against the cell proliferation of the cancer cell line Calu6 detected *in vitro.*

FIG. 4 shows the inhibitory activity of ADCs against the cell proliferation of the human LIV-1-overexpressing cancer cell line MCF7-ATCC-hLIV-1-#7 detected *in vitro.*

FIG. 5 shows the inhibitory activity of ADCs against the cell proliferation of the human LIV-1-overexpressing cancer cell line MCF7-ATCC-hLIV-1-#12 detected *in vitro.*

FIG. 6 shows the inhibitory activity of ADCs against the cell proliferation of the human LIV-1-overexpressing cancer cell line MCF7-ATCC-hLIV-1-#17 detected *in vitro.*

FIG. 7 shows the experimental results of xenografting the MCF7 breast cancer cell line into NSG mice. The dose and administration time are shown in the figure.

FIG. 8 shows the experimental results of xenografting the HCC1806 breast cancer cell line into NSG mice. The dose and administration time are shown in the figure.

FIG. 9 shows the experimental results of xenografting the PC3 prostate cancer cell line into nude mice. The dose and administration time are shown in the figure.

FIG. 10 shows the experimental results of xenografting the Calu-6 lung cancer cell line into nude mice. The dose and administration time are shown in the figure.

FIG. 11 shows the experimental results of xenografting the Calu-6 lung cancer cell line into nude mice. The dose and

administration time are shown in the figure.

FIG. 12 shows the experimental results of xenografting the PC3 prostate cancer cell line into nude mice. The dose and administration time are shown in the figure.

FIG. 13 shows the experimental results of xenografting the PA-1 ovarian cancer cell line into nude mice. The dose and administration time are shown in the figure.

DETAILED DESCRIPTION

Through extensive and intensive studies, the inventors provide an antibody specifically binding to human LIV-1 and conjugate the antibody to a drug load to prepare a series of anti-human LIV-1 antibody-drug conjugates. The anti-human LIV-1 antibody and the anti-human LIV-1 antibody-drug conjugate may be used in the preparation of a medicament for treating a tumor or cancer, thereby treating the tumor or cancer, particularly a tumor or cancer expressing LIV-1. *In vivo* and *in vitro* experiments have proven that the anti-human LIV-1 antibody-drug conjugate of the present disclosure has good targeting property on tumor cells and exhibits excellent anti-tumor activity. In addition, the anti-human LIV-1 antibody of the present disclosure may also be conjugated to a detectable label for detecting the presence of LIV-1 in a sample, thereby for use in the diagnosis of a tumor or cancer. The present disclosure is achieved on this basis.
For a better understanding of the present disclosure, the following terms are defined.
Unless otherwise stated, all singular terms also encompass the plural, active tense, and past tense forms of a term.
Unless otherwise clearly indicated in the context, the term "about" includes values that are within a standard deviation of the stated value.
The phrase "consisting substantially of" means that the compositions and methods may include additional ingredients and/or steps, but only if the additional ingredients and/or steps do not materially alter the basic and novel characteristics of the claimed compositions or methods. The term "consisting of" is intended to exclude all inactive ingredients.
"Subject" or "patient" according to the present disclosure is an animal, including a human patient in need of anti-cancer treatment or therapy. In certain aspects, the present disclosure may also be applied in veterinary practice to any mammal or other animal in need of such LIV-1-targeted anti-cancer treatment. The term may include, e.g., non-human primates, canines, felines, porcines, equines, and any other animal in need of the LIV-1-targeted anti-cancer treatment.
The term "antibody" used herein is intended to include any known type of natural or engineered antigen-binding protein or polypeptide that comprises at least one antigen-specific variable domain (VL or VH, or a similar domain of an engineered functional polypeptide-binding domain). The antibody may be polyclonal, monoclonal, or engineered, such as a humanized monoclonal antibody. Derivatives and fragments of polyclonal and/or monoclonal antibodies are also contemplated for use. These include Fc fragments, Fab fragments, single-chain antibodies, and polymers thereof, synthetic polypeptides having two or more binding specificities, and the like.
In general, antibody fragments (or antigen-binding fragments) will compete with intact antibodies for specific binding to the target, including independent heavy chains, light chains, Fab, Fab', F(ab')2, F(ab)c, diabodies, dabs, nanobodies and Fv. Antibody fragments can be produced by recombinant DNA techniques, or by enzymatic or chemical isolation of intact immunoglobulins. The term "antibody" also includes diabodies (homodimeric Fv fragments) or minibodies (VL-VH-CH3), bispecific antibodies, or analogs. A bispecific or bifunctional antibody is an artificial hybrid antibody having two different heavy/light chain pairs and two different binding sites (see Songsivilai and Lachmann, Clin. Exp. Immunol., 79:315-321 (1990); Kostelny et al., J. Immunol., 148:1547-53 (1992)). The term "antibody" includes an antibody itself (naked antibody) or an antibody conjugated to a cytotoxic or cytostatic drug, referred to herein as an antibody-drug conjugate or ADC.
Monoclonal antibodies generally require isolation and purification. This means that the purity of the antibody is generally at least 50% with respect to interfering proteins and other contaminants generated during the production or purification of the antibody, but does not exclude the possibility of binding the monoclonal antibody to an excess of pharmaceutically acceptable carriers or other carriers intended to facilitate its use. Sometimes, the monoclonal antibody has a purity of at least 60%, 70%, 80%, 90%, 95%, or 99% w/w with respect to the interfering proteins and contaminants during production or purification.
The basic structural unit of a natural antibody is a tetramer subunit structure. Each tetramer comprises two identical pairs of polypeptide chains, each pair having a "light" chain (about 25 kDa) and a "heavy" chain (about 50-70 kDa). The amino-terminal portion of each chain comprises a variable region of about 100 to 110 or more amino acids primarily responsible for antigen recognition. This variable region is linked to a cleavable signal peptide when expressed. The variable region without the signal peptide is sometimes referred to as a mature variable region. Thus, for example, a light chain mature variable region means a light chain variable region without a light chain signal peptide. The carboxyl-terminal

portion of each chain defines a constant region primarily responsible for effector function.

Specific binding of a monoclonal antibody to its target antigen means having an affinity of at least $10^6$, $10^7$, $10^8$, $10^9$, or $10^{10}$ $M^{-1}$. Specific binding is detectably higher in magnitude and different from non-specific binding occurring to at least one unrelated target. Specific binding may be the result of forming bonds between specific functional groups or specific spatial matches (e.g., lock and key type), while nonspecific binding is typically the result of van der Waals forces. However, specific binding does not necessarily mean that the monoclonal antibody binds to one and only one target.

Light chains are classified as κ or λ. Heavy chains are classified as γ, μ, α, δ, or ε, and isotypes of antibodies are defined as IgG, IgM, IgA, IgD, and IgE. In the light and heavy chains, the variable and constant regions are linked by a "J" region of about 12 or more amino acids, and the heavy chain also comprises a "D" region of about 10 or more amino acids. (See generally, Fundamental Immunology, Paul, W., ed., 2nd ed. Raven Press, N.Y., 1989, Ch. 7, which is incorporated by reference in its entirety for all purposes).

The mature variable region of each light/heavy chain pair forms an antibody-binding site. Thus, an intact antibody has two binding sites. Except in bifunctional or bispecific antibodies, the two binding sites are identical. These chains all exhibit the same general structure of relatively conserved framework regions (FRs) linked by three hypervariable regions, also known as complementarity determining regions or CDRs. The CDRs from the two chains of each pair are arranged according to the framework regions, thereby allowing the binding to a specific epitope. From the N-terminus to the C-terminus, the light chain and heavy chain each consist of domains FR1, CDR1, FR2, CDR2, FR3, CDR3, and FR4. The assignment of amino acids to each domain is in accordance with Kabat, Sequences of Proteins of Immunological Interest (National Institutes of Health, Bethesda, Md., 1987 and 1991), or Chothia & Lesk, J. Mol. Biol. 196:901-917(1987); Chothia et al., Nature 342:878-883 (1989)). Kabat also provides a widely used numbering convention (the Kabat numbering) in which corresponding residues between different heavy chains or between different light chains are assigned the same number.

The term "epitope" refers to a site on an antigen to which an antibody binds. An epitope can be formed from contiguous amino acids or non-contiguous amino acids by tertiary folding of one or more proteins. Epitopes formed from contiguous amino acids are typically retained in denaturing solvents, whereas epitopes formed by tertiary folding are typically lost in denaturing solvents. An epitope typically includes at least 3, and more generally at least 5 or 8-10 amino acids forming a unique spatial conformation. Methods for determining spatial conformation of an epitope include, for example, x-ray crystallography and 2-dimensional nuclear magnetic resonance. See, e.g., Epitope Mapping Protocols, in Methods in Molecular Biology, Vol. 66, Glenn E. Morris, Ed. (1996).

Antibodies that recognize the same or overlapping epitopes can be detected by a simple immunoassay to detect the ability of one antibody to compete with another antibody for binding to a target antigen. The epitope where an antibody binds to an antigen can also be determined by x-ray crystallography to determine interacting residues. Alternatively, two antibodies have the same epitope if all amino acid mutations in the antigen that cause reduced or eliminated binding of one antibody also reduce or eliminate binding of the other. Two antibodies have overlapping epitopes if some amino acid mutations in the antigen that cause reduced or eliminated binding of one antibody reduce or eliminate binding of the other.

Competition between antibodies can be determined by an assay in which a test antibody inhibits specific binding of a reference antibody to a common antigen (e.g., Junghans et al., Cancer Res. 50:1495, 1990). In a competitive binding assay, a test antibody competes with a reference antibody if the test antibody is in excess of the reference antibody (e.g., at least 2-fold, 5-fold, 10-fold, 20-fold, or 100-fold) and inhibits binding of the reference antibody by at least 50% but preferably 75%, 90%, or 99% in the competitive binding assay. Antibodies that can be identified by the competition assay (competing antibodies) include antibodies binding to the same epitope as the reference antibody, and antibodies binding to an adjacent epitope as the reference antibody.

To distinguish amino acid substitutions as being conservative or non-conservative, amino acids are classified as follows: class I (hydrophobic side chain): Met, Ala, Val, Leu, and Ile; class II (neutral hydrophilic side chain): Cys, Ser, and Thr; class III (acidic side chain): Asp and Glu; class IV (basic side chain): Asn, Gln, His, Lys, and Arg; class V (residues that influence chain orientation): Gly and Pro; and class VI (aromatic side chain): Trp, Tyr, and Phe. Conservative substitutions include substitutions between amino acids of the same class. Non-conservative substitutions are substitutions of different classes of amino acids.

Sequence alignment identification can be determined by maximally aligning antibody sequences according to the Kabat numbering convention. After alignment, if a structural region of the test antibody (e.g., the entire mature variable region of a heavy or light chain) is compared with the same region of the reference antibody, the percentage of similarity between the aligned sequences of the test antibody and the reference antibody is the number of positions occupied by the same amino acid in the regions of the test and reference antibodies divided by the total number of aligned positions of the two regions, with no gaps calculated, and converted into a percentage by multiplying by 100.

A composition or method "comprising" one or more said elements may include other elements not specifically described. For example, a composition comprising an antibody may comprise the antibody alone or in combination with other ingredients.

The specification of a range of values includes all integers within or defining the range.

Antibody effector functions refer to functions produced by an Fc domain of an Ig. For example, these functions may be antibody-dependent cytotoxicity, antibody-dependent cellular phagocytosis, or complement-dependent cytotoxicity. For example, binding of an Fc effector domain to an Fc receptor on an immune cell with phagocytic or lytic activity, or binding of the Fc effector domain to a component of a complement system, can result in an effector function. Generally, effects mediated by Fc-binding cells or complement components result in growth inhibition and/or apoptosis of target cells expressing LIV-1. The Fc region of the antibody can recruit Fc receptor (FcR)-expressing cells and juxtapose them with target cells that bind to the antibody. Cells expressing FcRs, including FcγRIII (CD16), FcγRII (CD32), and FcγRIII (CD64) on the membrane, can be used as effector cells for killing IgG-bound cells. These effector cells include monocytes, macrophages, natural killer cells, neutrophils, and eosinophils. Engagement of FcγR by IgG can activate antibody-dependent cellular cytotoxicity (ADCC) or antibody-dependent cellular phagocytosis (ADCP). ADCC is mediated by the CD16.sup.+ effector cells through secretion of membrane pore-forming proteins and proteases, while phagocytosis is mediated by CD32.sup.+ and CD64.sup.+ effector cells (see Fundamental Immunology, 4th ed., Paul ed., Lippincott-Raven, New York, 1997, Chapters 3, 17, and 30; Uchida et al., 2004, J. Exp. Med. 199:1659-69; Akewanlop et al., 2001, Cancer Res. 61:4061-65; Watanabe et al., 1999, Breast Cancer Res. Treat. 53:199-207). In addition to ADCC and ADCP, an Fc region of a cell-bound antibody can also activate the complement classical pathway to elicit complement-dependent cytotoxicity (CDC). When an antibody forms a complex with an antigen, C1q of the complement system binds to the Fc region of the antibody. When C1q binds to a cell-bound antibody, a cascade of reactions, including proteolytic activation of C4 and C2, can be initiated to produce C3 convertase. Cleavage of C3 to C3b by the C3 convertase can activate terminal complement components, including C5b, C6, C7, C8, and C9. In general, these proteins form membrane-attack complex pores on antibody-coated cells. These pores disrupt the integrity of the cell membrane, thereby killing target cells (see Immunobiology, 6th ed., Janeway et al., Garland Science, New York, 2005, Chapter 2).

The term "antibody-dependent cytotoxicity" or ADCC is a mechanism for inducing cell death, which depends on the interaction of antibody-coated target cells with immune cells having lytic activity (also known as effector cells). These effector cells include natural killer cells, monocytes/macrophages, and neutrophils. The effector cells are attached to the Fc effector domain of the Ig, while IgG binds to target cells through the antigen-binding site. The activity of effector cells results in the death of antibody-coated target cells.

The term "antibody-dependent cellular phagocytosis" or ADCP refers to the process of total or partial internalization of antibody-coated cells by phagocytic immune cells (e.g., macrophages, neutrophils, and dendritic cells) that bind to the Fc effector domain of the Ig.

The term "complement-dependent cytotoxicity" or CDC refers to a mechanism for inducing cell death in which the Fc effector domain of an antibody that binds to a target cell activates a series of enzymatic reactions, eventually forming pores in the target cell membrane. Generally, an antigen-antibody complex, such as an antigen-antibody complex formed by coating a target cell with an antibody, binds to and activates the complement component C1q, thereby activating the complement cascade reaction and causing the death of the target cell. Activation of a complement may also result in the deposition of complement components on the surface of target cells, which promote ADCC by binding to complement receptors (e.g., CR3) on leukocytes.

"Cytotoxic effect" refers to the depletion, elimination, and/or killing of target cells. "Cytotoxic formulation" refers to a formulation that has a cytotoxic effect on cells. The cytotoxic formulation may be conjugated to an antibody or administered in combination with the antibody.

"Cytostatic effect" refers to the inhibition of cell proliferation. "Cytostatic agent" refers to an agent that has a cytostatic effect on cells and thus inhibits the growth and/or expansion of a particular cell subpopulation. The cytostatic agent may be conjugated to an antibody or administered in combination with the antibody.

The term "pharmaceutically acceptable" means approved or approvable by a regulatory agency or listed in a pharmacopeia or other commonly-recognized pharmacopeias for use in animals, particularly in humans. The term "pharmaceutically compatible ingredient" refers to a pharmaceutically acceptable diluent, adjuvant, excipient, or carrier to which the anti-LIV-1 antibody binds.

The phrase "pharmaceutically acceptable salt" refers to a pharmaceutically acceptable organic or inorganic salt of the anti-LIV-1 antibody or a conjugate thereof or a formulation for use in combination with the anti-LIV-1 antibody. Exemplary salts include sulfate, citrate, acetate, oxalate, chloride, bromide, iodide, nitrate, bisulfate, phosphate, acid phosphate, isonicotinate, lactate, salicylate, citrate, tartrate, oleate, tannate, pantothenate, bitartrate, ascorbate, succinate, maleate, gentisate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylenebis(2-hydroxy-3-naphthoate)). The pharmaceutically acceptable salt may comprise another molecule, such as an acetate ion, a succinate ion, or other counterions. The counterion may be any organic or inorganic moiety to stabilize the charge of the parent compound. In addition, the pharmaceutically acceptable salt may have more than one charged atom in its structure. Examples where a plurality of charged atoms are part of the pharmaceutically acceptable salt may have a plurality of counterions. Thus, the pharmaceutically acceptable salt may have one or more charged atoms and/or one or more counterions.

The terms "host cell", "host cell line", and "host cell culture" are used interchangeably and refer to cells into which exogenous nucleic acids have been introduced, including progeny of such cells. Host cells include "transformants" and "transformed cells", which include primary transformed cells and progeny derived therefrom, regardless of the number of passages. Progeny may not be completely identical to parent cells in terms of nucleic acid content and may contain mutations. As used herein, the term includes mutant progeny that have the same function or biological activity as the cells screened or selected from the primary transformed cells. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica, Pichia, Saccharomycescerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens,* and *Neurospora crassa. Pichia,* any *Saccharomyces, Hansenula polymorpha,* any *Kluyveromyces, Candida albicans,* any *Aspergillus, Trichoderma reesei, Chrysosporium lucknowense,* any *Fusarium, Yarrowia lipolytica,* and *Neurospora crassa.* The host cell of the present patent does not include objects not authorized by the Patent Laws.

The term antibody-drug conjugate (ADC) means that a monoclonal antibody or an antibody fragment is linked to a biologically active toxic drug by a linker unit. The antibody or the antibody fragment described in the present disclosure may be conjugated to the effector molecule by any means. For example, the antibody or the antibody fragment may be chemically or recombinantly attached to the toxic drug. Chemical means for preparing fusions or conjugates are known in the art. The method for conjugating the antibody or the antibody fragment to the drug must be capable of linking the antibody to the toxic drug without interfering with the ability of the antibody or the antibody fragment to bind to the target molecule.

The cytotoxic drug or cytotoxic compound refers to a substance that inhibits or prevents cell functions and/or causes cell death or cell destruction. The cytotoxic drug can kill tumor cells in principle at a sufficiently high concentration; however, due to a lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant, or animal origin, radioisotopes (e.g., $At^{211}$, $I^{131}$, $I^{125}$, $Y^{90}$, $Re^{186}$ , $Re^{188}$, $Sm^{153}$, $Bi^{212}$, $P^{32}$, and radioisotopes of Lu), chemotherapeutic drugs, antibiotics, and nucleolytic enzymes.

The antibody of the present disclosure may be conjugated to the cytotoxic drug by a conjugating agent. Examples of the conjugating agent may be any one or more of a non-selective conjugating agent, a conjugating agent using a carboxyl group, a peptide chain, and a conjugating agent using a disulfide bond. The non-selective conjugating agent refers to a compound that enables an effector molecule and an antibody to form a covalent bond, such as glutaraldehyde. The conjugating agent using a carboxyl group may be any one or more of a cis-aconitic anhydride conjugating agent (such as cis-aconitic anhydride) and an acylhydrazone conjugating agent (conjugating site is acylhydrazone).

Certain residues on an antibody (such as Cys or Lys) are used to link to a variety of functional groups, including imaging agents (e.g., chromophores and fluorophores), diagnostic agents (e.g., MRI contrast agents and radioisotopes), stabilizing agents (e.g., ethylene glycol polymers) and therapeutic agents. An antibody may be conjugated to a functional agent to form an antibody-functional agent conjugate. The functional agent (e.g., a drug, a detection reagent, a stabilizing agent) is conjugated (covalently linked) to the antibody. The functional agent may be linked to the antibody directly, or indirectly through a linker.

An antibody may be conjugated to a drug to form antibody-drug conjugates (ADCs). Typically, an ADC comprises a linker between the drug and the antibody. The term "linker unit" or "linker fragment" refers to a chemical structure fragment or bond that is linked to an antibody or an antigen-binding fragment thereof at one end and to a drug at the other end, and it may also be linked to a drug after being linked to another linker. The linker may be a degradable or non-degradable linker. A degradable linker is typically susceptible to degradation in the intracellular environment. For example, a linker is degraded at the target site, thereby releasing a drug from an antibody. A suitable degradable linker includes, for example, enzymatically degradable linkers including peptidyl-containing linkers that can be degraded by intracellular proteases (e.g., lysosomal proteases or endosomal proteases), or sugar linkers, e.g., glucuronide-containing linkers that can be degraded by glucuronidase. Peptidyl linkers may include, for example, dipeptides such as valine-citrulline, phenylalanine-lysine, or valine-alanine; or tripeptides such as glycine-phenylalanine-glycine; or tetrapeptides such as glycine-glycine-phenylalanine-glycine. Other suitable degradable linkers include, for example, pH-sensitive linkers (e.g., linkers that hydrolyze at a pH of less than 5.5, such as hydrazone linkers) and linkers that degrade under reducing conditions (e.g., disulfide linkers). A non-degradable linker typically releases a drug under conditions in which an antibody is hydrolyzed by a protease.

Prior to attachment to the antibody, the linker has a reactive group capable of reacting with certain amino acid residues, and the attachment is achieved by the reactive group. The thiol-specific reactive group is preferred, and includes: such as maleimide compounds, haloamides (e.g., iodo, bromo, or chloro); halogenated esters (e.g., iodo, bromo, or chloro); halomethyl ketones (e.g., iodo, bromo, or chloro), benzyl halides (e.g., iodo, bromo, or chloro); vinyl sulfones, pyridyl disulfides; mercury derivatives such as 3,6-bis-(mercuric methyl)dioxane, and counter ions are acetate, chloride, or nitrate; and polymethylene dimethyl sulfide thiosulfonates. A linker may comprise, for example, a maleimide linked to an antibody via a thiosuccinimide.

In the present disclosure, the drug-linker compound can be used to form an ADC in one simple step. In other embodiments, the bifunctional linker compound may be used to form an ADC in a two-step or multi-step process. For example, a cysteine residue is reacted with a reactive moiety of a linker in the first step, and in a subsequent step, a functional group on the linker is reacted with a drug, thereby forming an ADC.

Generally, the functional group on a linker is selected to facilitate a specific reaction with a suitable reactive group on a drug moiety. As a non-limiting example, an azide-based moiety may be used to specifically react with the reactive alkynyl group on the drug moiety. The drug is covalently bound to the linker through a 1,3-dipolar cycloaddition between the azide and the alkynyl group. Other useful functional groups include, for example, ketones and aldehydes (suitable for reaction with hydrazides and alkoxyamines), phosphines (suitable for reaction with azides); isocyanates and isothiocyanates (suitable for reaction with amines and alcohols); and activated esters, such as N-hydroxysuccinimide esters (suitable for reaction with amines and alcohols). These and other ligation strategies, such as those described in Bioconjugation Technology (2nd ed., (Elsevier)), are well known to those skilled in the art. It will be appreciated by those skilled in the art that for selective reaction of a drug moiety and a linker, each member of a complementary pair may be used for both the linker and the drug when the reactive functional group of the complementary pair is selected.

The present disclosure further provides a method for preparing an ADC, which may further comprises: binding an antibody to a drug-linker compound (LD) under conditions sufficient to form an antibody conjugate (ADC).

In certain embodiments, the method of the present disclosure comprises: binding an antibody to a linker compound under conditions sufficient to form an antibody-linker conjugate. In these embodiments, the method of the present disclosure further comprises: binding the antibody-linker conjugate to a drug moiety under conditions sufficient to covalently link the drug moiety to the antibody by a linker.

Drug loading, also referred to as drug-to-antibody ratio (DAR), refers to the average number of drugs conjugated to each antibody in an ADC. It may range, for example, from about 1 to about 10 drugs conjugated to each antibody, and in certain embodiments, from about 1 to about 8 drugs conjugated to each antibody, preferably selected from 2-8, 2-7, 2-6, 2-5, 2-4, 3-4, 3-5, 5-6, 5-7, 5-8, and 6-8 drugs conjugated to each antibody. Illustratively, the drug loading may be an average of 1, 2, 3, 4, 5, 6, 7, 8, 9, or 10. The ADC general formulas of the present disclosure include a set of antibody-drug conjugates within a certain range as described above. In the embodiments of the present disclosure, the drug loading may be represented by z, which is a decimal or integer. The drug loading can be determined by conventional methods such as UV/visible spectroscopy, mass spectrometry, ELISA, and HPLC.

In one embodiment of the present disclosure, the cytotoxic drug is conjugated to an antibody by a linker unit.

The loading of the ligand-drug conjugate can be controlled by the following non-limiting methods, including:

(1) controlling the molar ratio of a linker fragment of the drug to the monoclonal antibody,

(2) controlling reaction time and temperature, and

(3) selecting different reaction reagents.

The present disclosure is illustrated with reference to the following specific examples. It will be appreciated by those skilled in the art that these examples are merely illustrative of the present disclosure and are not intended to limit the scope of the present disclosure in any way.

"Sugar" as used herein refers to a monovalent radical of a monosaccharide (e.g., a pyranose or a furanose). The sugar may comprise a hemiacetal or a carboxylic acid (from the oxidation of the pendant $-CH_2OH$ group). In some embodiments, the sugar is in the β-D configuration. In some embodiments, the sugar is glucose, glucuronic acid, N-acetylglucosamine, or galactose.

The term "hydroxy" refers to -OH.

The term "amino" refers to $-NH_2$.

Dichloromethane is abbreviated as DCM.

N,N-Diisopropylethylamine is abbreviated as DIEA.

N,N-Dimethylformamide is abbreviated as DMF.

O-(7-Azabenzotriazol-1-yl)-N,N,N',N'-tetramethyluronium hexafluorophosphate is abbreviated as HATU.

1-Hydroxybenzotriazole is abbreviated as HOBt.

"Substituted" means that one or more, preferably 1-6, more preferably 1-3 hydrogen atoms in the group are independently substituted with a corresponding number of substituents. Those skilled in the art can determine (experimentally or theoretically) possible or impossible substitutions without undue effort. For example, it may be unstable when an amino or hydroxy having free hydrogen is bound to a carbon atom having an unsaturated (e.g., olefinic) bond.

Unless otherwise specified, the experimental methods in the following examples are all conventional methods. The starting materials, reagent materials, and the like used in the following examples are all commercially available products unless otherwise specified.

**Example 1. Preparation of Anti-LIV-1 Antibodies**

The present disclosure relates to a method for preparing antibodies by immunizing mice with a human LIV-1 extracellular region fragment, as well as to humanization of anti-human LIV-1 murine antibodies. Murine antibodies are prepared by injecting mice with a composition comprising a human LIV-1 extracellular region fragment, and the presence of antibodies is determined by taking a serum sample. Lymphocytes from lymph nodes of mice are obtained, and specific B cells against the human LIV-1 antigen are enriched. Single B cell sequencing and analysis are performed to obtain antibody sequences.

After the antibody sequences are obtained, the antibodies can be prepared by recombinant techniques, and the murine antibodies are chimerized and humanized. Specifically, mouse complementarity determining regions are transferred from heavy and light chain variable regions of a mouse immunoglobulin into human variable regions, and then human residues in framework regions are replaced with murine analogs to prepare humanized antibodies. During the preparation of humanized antibodies, CDR regions can be selectively subjected to affinity maturation or removal of hotspot sites to improve the performance of the antibody in a certain aspect.

The antibodies prepared in this example include monoclonal antibodies A, B, and C, wherein the antibody A is a humanized antibody, and the antibody B and the antibody C are chimeric antibodies, wherein the murine antibody of the antibody A comprises the mVH sequence set forth in SEQ ID NO: 28 and the mVL sequence set forth in SEQ ID NO: 29; the antibody A comprises the VH sequence set forth in SEQ ID NO: 1 and the VL sequence set forth in SEQ ID NO: 2; another antibody A-1 obtained by humanizing the murine antibody of the antibody A comprises CDR sequences identical to those of the antibody A, and the antibody A-1 comprises the VH sequence set forth in SEQ ID NO: 32 and the VL sequence set forth in SEQ ID NO: 2; the antibody B comprises the VH sequence set forth in SEQ ID NO: 3 and the VL sequence set forth in SEQ ID NO: 4; the antibody C comprises the VH sequence set forth in SEQ ID NO: 5 and the VL sequence set forth in SEQ ID NO: 6. The heavy chain constant region sequence of each antibody is set forth in SEQ ID NO: 26, and the light chain constant region sequence is set forth in SEQ ID NO: 27.

Further, the binding affinities of the prepared antibodies for the human LIV-1 antigen (with a His tag, see NP_036451.1 for the amino acid sequence, comprising amino acids Phe229-Ile323) were detected. The results show that after humanization, the antibody A has significantly improved binding activity for human LIV-1, as shown in Table 1. The binding affinity was measured by ForteBio.

Table 1. Binding activity of anti-LIV-1 antibodies to LIV-1 protein

| Antibody name | KD (M) | Ka (1/Ms) | Kd (1/s) |
|---|---|---|---|
| Murine antibody of antibody A | 5.05E-11 | 1.04E+06 | 5.27E-05 |
| Antibody A | 2.82E-11 | 9.18E+05 | 2.58E-05 |
| Antibody A-1 | 5.24E-11 | 7.96E+05 | 4.17E-05 |

**Example 2. Assay for Binding Ability of Anti-LIV-1 Antibodies to LIV-1 Protein and Cell Lines Expressing LIV-1**

**2.1 Binding of anti-LIV-1 antibodies to LIV-1 protein**

The binding ability of the anti-LIV-1 antibodies obtained by screening to LIV-1 was determined. This assay could be performed in a solution, in a suspension, or on a solid support. For example, the target antigen could be immobilized on a solid support (e.g., a carbon or plastic surface or chip) and contacted with the antibody. Unbound antibodies or unbound target proteins could be washed away and the bound complexes were then detected. The binding assay could be performed under conditions that reduce non-specific binding, for example, using a high ionic strength buffer (e.g., 0.3-0.4 M NaCl) and a non-ionic detergent (e.g., 0.1% Triton X-100 or Tween 20) and/or a blocking protein (e.g., bovine serum albumin or gelatin). A negative control was included in the analysis. The binding affinity can be determined by ELISA, Gator, BIACore, or other methods.

In this experiment, an enzyme-linked immunosorbent assay (ELISA) was adopted to attach the LIV-1 antigen to a

solid carrier. The test antibody in a sample bound to the antigen to form a solid phase antigen-test antibody complex. Then, an enzyme-labeled secondary antibody bound to the antibody in the solid phase immune complex to form a solid phase antigen-test antibody-enzyme-labeled secondary antibody complex. The color development degree after the substrate was added was determined. The absorbance value was positively correlated with the binding activity of the antibody.

Specifically, hLIV-1 (with a His tag, see NP_036451.1 for the amino acid sequence, comprising amino acids Phe229-Ile323) was diluted with a coating buffer to 2 μg/mL, and the resulting dilution was added to a microplate for coating at 4 °C for 15-20 h. Then, 300 μL of a blocking solution was added to each well for blocking at room temperature for 1 h. The test antibody was diluted with a diluent and incubated at room temperature for 1 h. The goat anti-human Fc domain secondary antibody (JacksonImmune, Cat# 109-035-170) was diluted with a diluent and incubated at room temperature for 1 h. After the incubation of the secondary antibody was completed, the TMB chromogenic solution was added for incubation at room temperature for 1-10 min. After the color development was completed, 50 μL of a stop solution (4 M sulfuric acid) was added to each well to stop the substrate reaction. The absorbance of each well was measured at a detection wavelength of 450 nm on a microplate reader, and the results were calculated. The data were analyzed using Prism software, and dose-response curves were plotted using a Sigmoidal, 4PL four-parameter equation with the concentration of the test antibody as the abscissa and the corresponding average absorbance as the ordinate. Equation:

$$Y = Bottom + (X\verb|^|Hillslope)\times(Top - Bottom)/(X\verb|^|HillSlope + EC50\verb|^|HillSlope).$$

The results are shown in FIGs. 1A and 1B. The anti-LIV-1 antibodies of the present disclosure specifically bind to the human LIV-1 antigen.

**2.2 Binding of anti-LIV-1 antibody to cell lines expressing LIV-1**

The specific binding ability of the anti-hLIV-1 antibody A to the LIV-1 antigen expressed on the cell surface was detected using a flow cytometer.

The cell lines selected for this experiment were as follows: Calu-6 (ATCC HTB-56, anaplastic lung cancer), HCC1806 (ATCC CRL-2335, human breast cancer TNM stage IIB grade 2), PC-3 (ATCC CRL-1435, prostate cancer), and MCF7 overexpressing LIV-1 (ATCC HTB-22, metastatic sites of breast cancer), i.e., MCF7-ATCC-LIV-1#7, #12, and #17. In an incubator at 37 °C with 5% $CO_2$, Calu6 and HCC1806 cells were incubated in an RPMI-1640 medium containing 10% fetal bovine serum, PC-3 cells were incubated in an F12K medium containing 10% fetal bovine serum, and MCF7-ATCC-LIV-1 overexpressing cell lines were incubated in an EMEM medium containing 10% fetal bovine serum and 1 μg/mL. After the cell lines were dissociated and washed in a staining buffer (Biolengend), the number of cells was counted and adjusted to $2 \times 10^5$ cells/100 μL staining buffer. Then, the antibody A was added to a final concentration of 10 μg/mL, and the mixture was reacted at 4 °C for 15 min. After the reaction, the cells were washed in the staining buffer, followed by suspending PE-labeled constant region (Fc)-specific antibody (rabbit anti-human IgG PE conjugate, BioLegend, 410707) at 2 μL/$2 \times 10^5$ cells/100 μL PBS. The mixture was reacted at 4 °C for 15 min. After cell reaction, the cells were washed in the staining buffer, and single cells were analyzed for readings in the PE channel using a Novocyte 3000 (Agilent) apparatus. The negative control was treated with a non-specific isotype control commercial antibody IgG (BioXCell, BE0297) and then treated with the PE-labeled constant region (Fc)-specific antibody. The quotient (MFI ratio: MFI of anti-LIV-1/MFI of control antibody) of the shifted reading of each experimental group treated with the antibody A in the present disclosure divided by the shifted reading of the control group was used to compare the expression levels of LIV-1 among cancer cell lines. The experimental results are shown in Table 2.

The experiments have demonstrated and confirmed that the anti-LIV-1 antibody of the present disclosure specifically binds to LIV-1 expressed in various cancer cell lines including breast cancer, lung cancer, and prostate cancer.

Table 2. Staining readings for binding of antibody A and control antibody without binding function to each cell line

| MFI (RFU) | Calu6 | MCF7-Liv1 #7 | MCF7-Liv1 #12 | MCF7-Liv1 #17 | HCC 1806 | PC-3 |
|---|---|---|---|---|---|---|
| Expression of Liv1 | Endogenous expression | High exogenous expression | Moderate exogenous expression | High exogenous expression | Endogenous expression | Endogenous expression |
| Antibody A | 11143 | 132122 | 20945 | 107363 | 4416 | 5661 |
| IgG1 | 4441 | 3413 | 3123 | 1509 | 2182 | 2840 |
| Ratio | 2.5 | 38.7 | 6.7 | 71.1 | 2.0 | 2.0 |

**Example 3. Synthesis of Compounds for Preparing Antibody-Drug Conjugates**

Experimental methods without specific conditions indicated in this example were generally conducted under conventional conditions or conditions recommended by the manufacturer of the starting materials or commercial products. Reagents without specific origins indicated were commercially available conventional reagents.

The structures of the compounds were determined by nuclear magnetic resonance (NMR) spectroscopy or/and mass spectrometry (MS). NMR shifts (δ) are expressed in $10^{-6}$ (ppm). $^1$HNMR analysis was performed using a Bruker AVANCE-400 nuclear magnetic resonance instrument, with deuterated dimethyl sulfoxide as a solvent and tetramethylsilane (TMS) as an internal standard.

MS analysis was performed using a Shimadzu LCMS-2020 Single Quaddrupole liquid chromatography-mass spectrometry system (manufacturer: Shimadzu; MS model: 2020 Single Quadrupole MS).

RP-HPLC analysis was performed using a Shimadzu LC-2030c Plus liquid chromatograph. The preparation was performed using a Shimadzu Nexera preparative liquid chromatography system, with a preparative chromatographic column: Phenomenex Gemini NX 5 μ, C18, 110 A, 150 × 50 mm, and mobile phase: 0.1% trifluoroacetic acid in water/0.1% trifluoroacetic acid in acetonitrile (ACN).

The silica gel column chromatography used SiliCycle (Canada) 200-300 mesh silica gel as a carrier.

**3.1 Synthesis of compound 3**

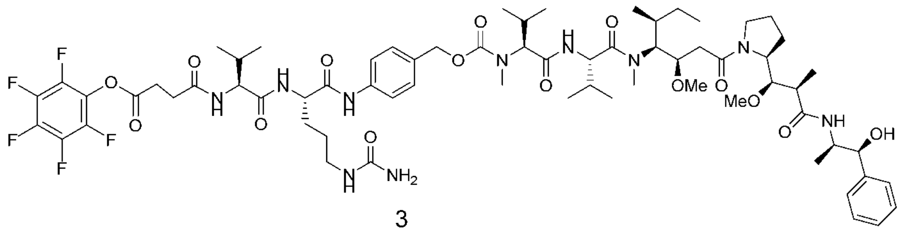

3

The synthesis process is as follows:

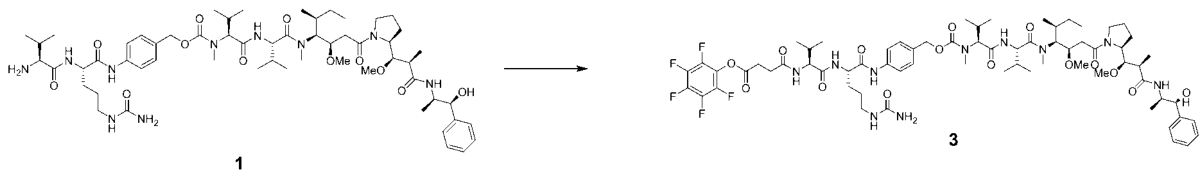

Glutaric anhydride (12 mg) was added to a solution of compound 1 (110 mg, purchased from MedChemExpress, Cat. No. HY-100374) in anhydrous DMF (2 mL), followed by the addition of DIEA (0.04 mL). The reaction mixture was stirred at room temperature for 1 h and then diluted with DCM (10 mL). Pentafluorophenol (90 mg) and (1-(3-dimethylaminopropyl)-3-ethylcarbodiimide hydrochloride (EDC.HCl) (100 mg) were added, and the mixture was stirred at room temperature. After 30 min, the reaction mixture was concentrated under reduced pressure. The residue was purified by RP-HPLC to give **compound 2** (102 mg) as a white powder.

MS assay: m/z 1403.6 $[M+H]^+$.

**3.2 Synthesis of compound 4**

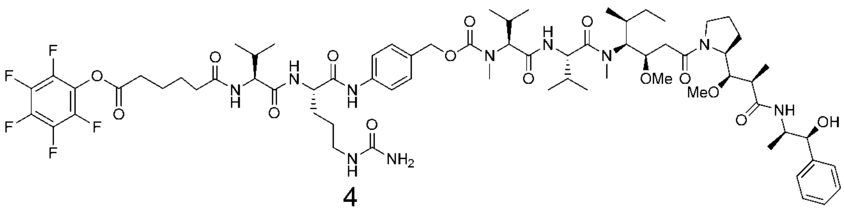

4

The synthesis process is as follows:

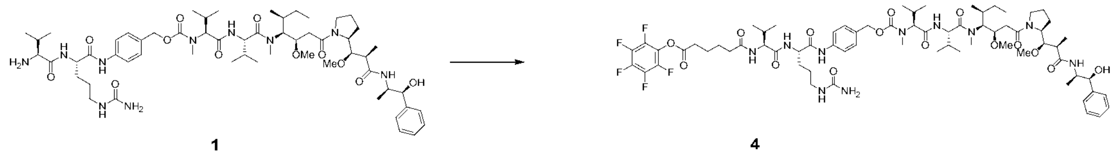

DIEA (40 μL) and bis(pentafluorophenol)adipate (200 mg) were added to a solution of compound 1 (110 mg) in anhydrous DMF (2 mL). The reaction mixture was stirred at room temperature for 30 min and directly purified by RP-HPLC to give compound 4 (89 mg) as a white powder.

MS assay: m/z 1417.6 $[M+H]^+$.

**3.3 Synthesis of compound 5**

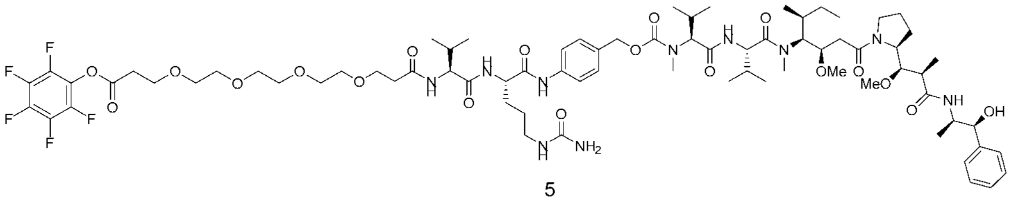

5

The synthesis process is as follows:

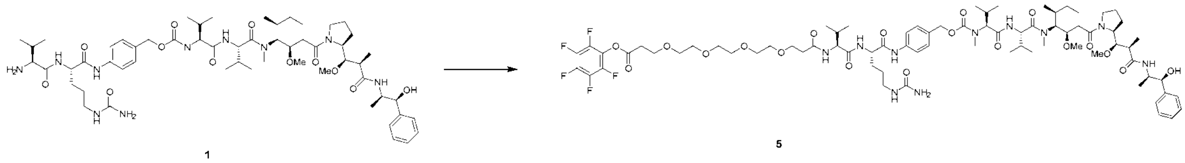

DIEA (40 μL) and bis-PEG3-pentafluorophenol ester (300 mg) were added to a solution of compound 1 (110 mg) in anhydrous DMF (2 mL). The reaction mixture was stirred at room temperature for 30 min and directly purified by RP-HPLC to give compound 5 (105 mg) as a white powder.

MS assay: m/z 1565.8 $[M+H]^+$.

**3.4 Synthesis of compound 10**

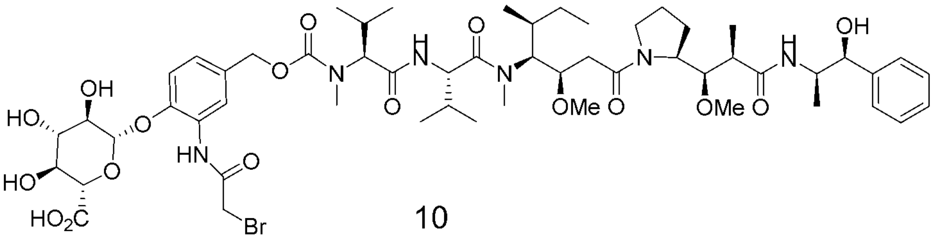

10

The synthesis process is as follows:

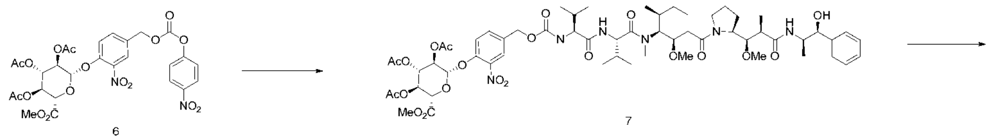

6 7

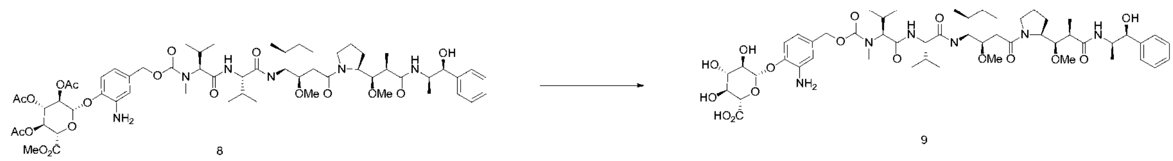

8 9

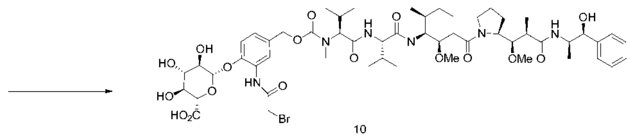

10

DIEA (0.02 mL) was added to a solution of compound 6 (65 mg) and MMAE (72 mg, purchased from MedChemExpress, Cat. No. HY-15162) in anhydrous DMF (2 mL), followed by the addition of 1-hydroxybenzotriazole (HOBt) (3 mg). The mixture was stirred at room temperature for 18 h and then diluted with water (20 mL). The mixture was extracted with ethyl acetate (40 mL). The organic layer was dried over $Na_2SO_4$ and concentrated to dryness under reduced pressure to give crude compound 7, which was dissolved in MeOH (2 mL). Zinc powder (200 mg) was added, followed by the addition of formic acid (0.2 mL). The mixture was stirred at room temperature for 30 min. The solid was removed by filtration, and the filtrate was directly purified by RP-HPLC to give compound 8 (72 mg) as a white powder.

Compound 8 (70 mg) was dissolved in acetonitrile/water (6/4, v/v). NaOH (aq., 1 M, 0.35 mL) was added to the solution, and the mixture was stirred at room temperature. After 1 h, bromoacetic anhydride (52 mg) was added, followed by the addition of sodium hydroxide (aq., 1 M, 0.2 mL). After 30 min, the mixture was directly purified by RP-HPLC to give **compound 10** (38 mg) as a white powder.

MS assay: m/z 1179.6 $[M+H]^+$.

### 3.5 Synthesis of compound 11

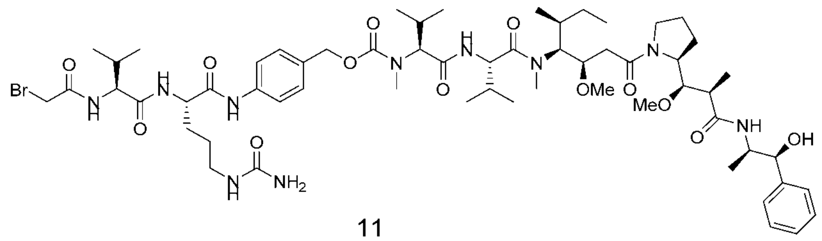

11

The synthesis process is as follows:

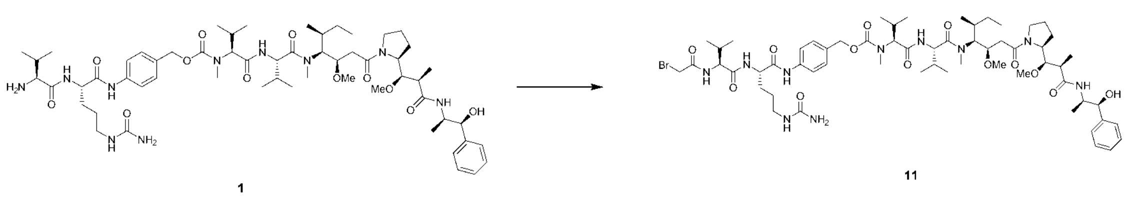

1 11

A saturated $NaHCO_3$ solution (0.02 mL) and bromoacetic anhydride (10 mg) were added to a solution of compound 1 (25 mg) in acetonitrile/water (6/4, v/v, 2 mL). The reaction mixture was stirred at room temperature for 10 min and directly purified by RP-HPLC to give compound 11 (21 mg) as a white powder.

MS assay: m/z 1243.6 $[M+H]^+$.

$^1H$ NMR (400 MHz, DMSO-*d6*) δ 10.00-10.01 (m, 1H), 8.02-8.32 (m, 3H), 7.56-7.89 (m, 3H), 7.15-7.35 (m, 7H),

5.97 (t, J = 6.0 Hz, 1H), 5.33-5.41 (m, 3H), 4.96-5.09 (m, 2H), 4.63-4.73 (m, 1H), 4.48-4.50 (m, 1H), 4.36-4.45 (m, 2H), 4.23-4.28 (m, 2H), 3.91-4.04 (m, 4H), 3.47-3.79 (m, 2H), 3.11-3.36 (m, 9H), 2.85-3.08 (m, 7H), 2.39-2.43 (m, 1H), 2.26-2.30 (m, 1H), 1.17-2.14 (m, 15H), 0.73-1.05 (m, 30H).

### 3.6 Synthesis of compound 22 (MC-VC-PAB-MMAE)

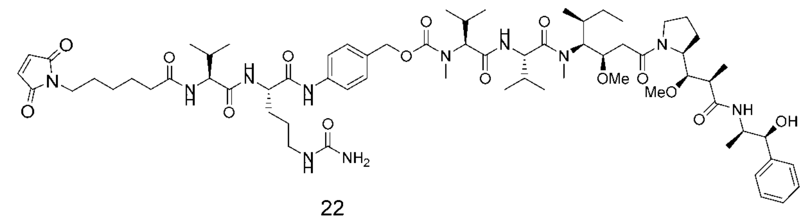

22

The synthesis process is as follows:

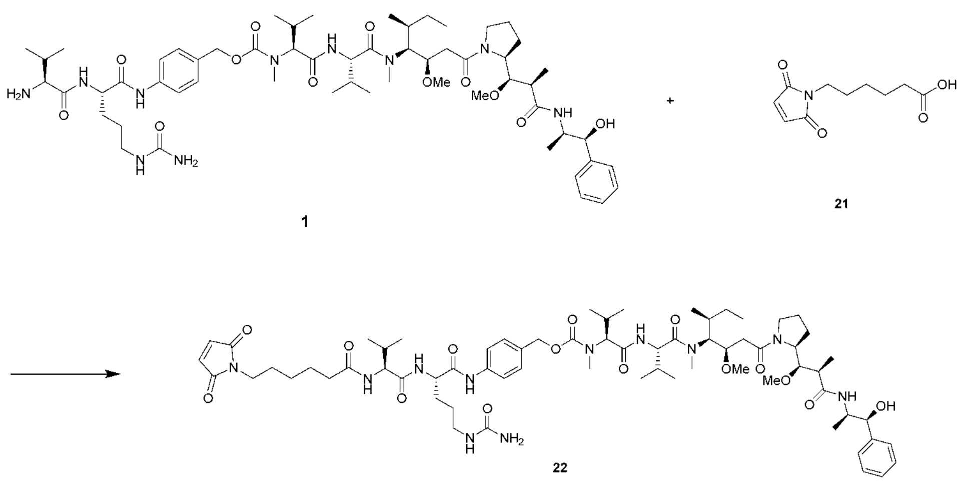

1

21

22

Maleimidohexanoic acid (12 mg) was added to a solution of compound 1 (62 mg) in anhydrous DMF (2 mL), followed by the addition of DIEA (0.02 mL) and HATU (20 mg). The reaction mixture was stirred at room temperature (22 °C). After 15 min, the crude reaction mixture was directly purified by RP-HPLC and lyophilized to give compound 22 (62 mg, TFA salt) as a white solid.

MS assay: m/z 1316.8 $[M+H]^+$.

### 3.7 Synthesis of compound 29 (MC-GGY-PAB-MMAE)

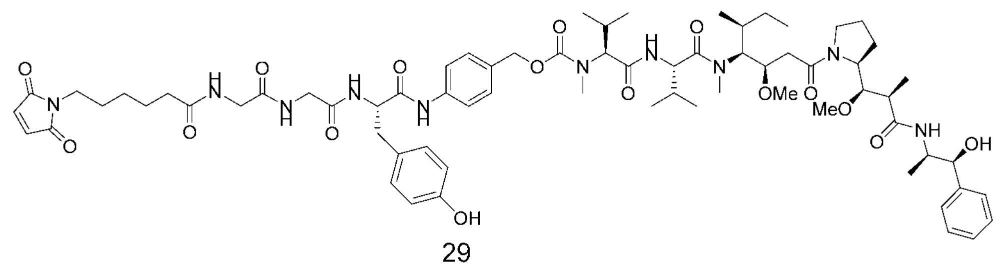

29

The synthesis process is as follows:

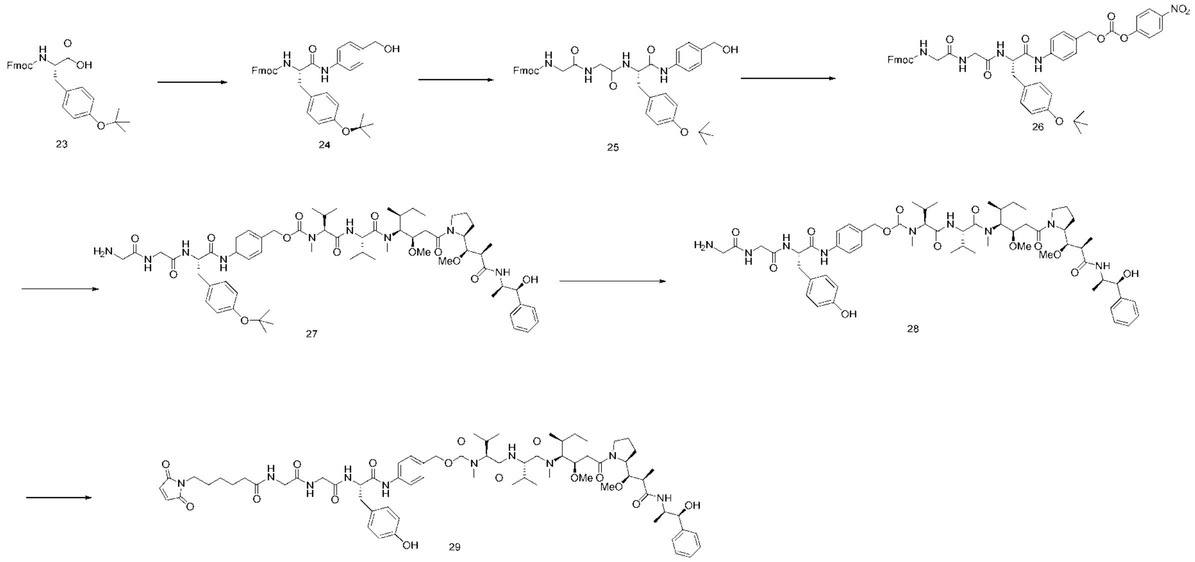

23 24 25 26 27 28 29

DIEA (0.35 mL) was added to a solution of compound 23 (Combi Block, 460 mg) and 4-aminobenzyl alcohol (130 mg) in DMF (5 mL), followed by the addition of HATU (390 mg). The reaction mixture was stirred at room temperature for 20 min and then diluted with EtOAc (100 mL). The mixture was washed with 0.5 M hydrochloric acid (50 mL) and water (50 mL). The organic layer was dried (over $Na_2SO_4$) and concentrated to dryness by evaporation under reduced pressure. The residue was triturated with diethyl ether/hexane (1/1, v/v, 100 mL) to give crude compound 24 as a light brown solid, which was dissolved in DMF (5 mL). Diisopropylamine (5 mL) was added, and the mixture was stirred at room temperature for 2 h. The reaction mixture was then concentrated under reduced pressure to about 4 mL. Fmoc-Gly-Gly-OH (fluorenylmethoxycarbonyl-glycine-glycine, 360 mg) and DIEA (0.35 mL) were added, followed by the addition of HATU (0.4 g). The mixture was stirred at room temperature. After 20 min, the reaction mixture was diluted with EtOAc (120 mL). The mixture was washed with 0.5 M hydrochloric acid (50 mL) and water (50 mL). The organic layer was dried (over $Na_2SO_4$) and concentrated to dryness by evaporation under reduced pressure. The crude product was purified by RP-HPLC to give compound 25 (550 mg) as a white solid.

Compound 25 (340 mg) was dissolved in DMF (4 mL). DIEA (0.08 mL) and bis(p-nitrophenyl) carbonate (bis-PNP carbonate, 300 mg) were added to the solution, and the mixture was stirred at room temperature for 16 h. The reaction mixture was directly purified by RP-HPLC to give compound 26 (335 mg) as a white solid.

DIEA (0.02 mL) was added to a solution of compound 26 (84 mg) and MMAE (0.072 g) in DMF (3 mL), and the reaction mixture was stirred at room temperature for 24 h. Piperidine (0.15 mL) was added. After 30 min, the mixture was directly purified by RP-HPLC to give compound 27 (TFA salt, 92 mg) as a white solid, which was treated with TFA/DCM (1/2, v/v, 3 mL) at room temperature for 40 min. The mixture was concentrated under reduced pressure to give a crude product, which was purified by RP-HPLC to give compound 28 (TFA salt, 64 mg) as a white solid.

6-Maleimidohexanoic acid N-hydroxysuccinimide ester (Sigma Aldrich, 9 mg) was added to a solution of compound 28 (25 mg, TFA salt) in DMF (2 mL), followed by the addition of DIEA (0.007 mL). The mixture was stirred at room temperature. After 30 min, the mixture was purified by RP-HPLC to give **compound 29** (22 mg) as a white solid.

MS: m/z 1337.8 $[M+H]^+$.

### 3.8 Synthesis of compound 39 (MC-GGY(Gal)-PAB-MMAE)

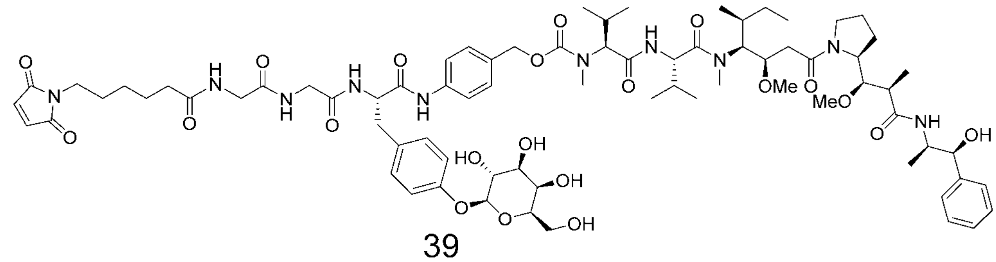

39

The synthesis process is as follows:

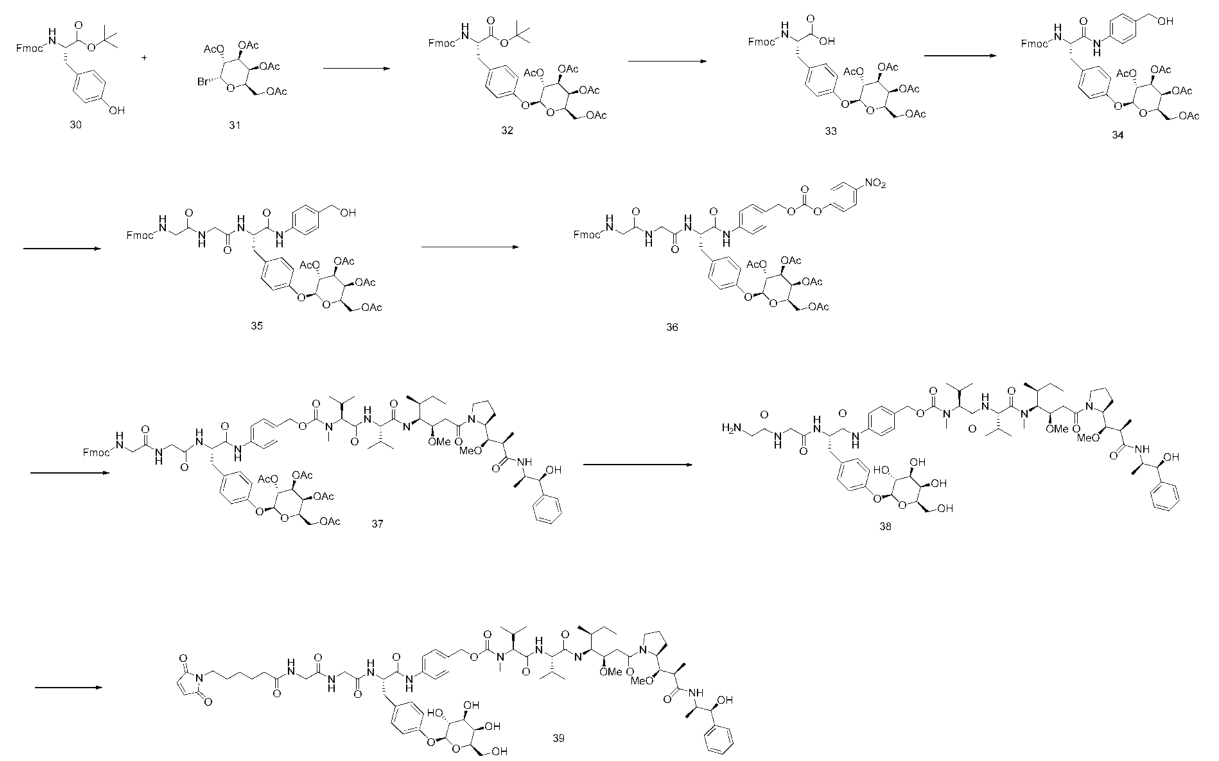

30 31 32 33 34 35 36 37 38 39

Under argon atmosphere, $Ag_2O$ (8 mmol) was added to a stirred solution of acetobromo-α-D-galactose (compound 31, CombiBlocks, 2.3 g) and Fmoc-Tyr-OtBu (tert-butyl fluorenylmethoxycarbonyl tyrosinate, compound 30, 2.1 g) in anhydrous acetonitrile (50 mL) at 0 °C. The solution was stirred at room temperature for 4 h. The mixture was filtered, and the filtrate was concentrated to dryness by evaporation under reduced pressure. The crude product was purified by silica gel column chromatography to give compound 32 (2.7 g) as a white solid.

Compound 32 (2 g) was redissolved in trifluoroacetic acid/dichloromethane (TFA/DCM, 1/1, v/v, 40 mL). After 30 min, the mixture was diluted with DCM (100 mL) and washed with water (40 mL × 4). The organic layer was concentrated to dryness by evaporation to give a crude acid, compound 33, which was dissolved in DMF (30 mL). 4-Aminobenzyl alcohol (0.34 g) was added to the solution, followed by the addition of DIEA (1 mL) and HATU (1 g). The reaction mixture was stirred at room temperature for 20 min and then diluted with EtOAc (120 mL). The mixture was washed with 0.5 M hydrochloric acid (100 mL) and water (50 mL). The organic layer was dried (over $Na_2SO_4$) and concentrated to dryness by evaporation under reduced pressure. The residue was triturated with hexane/diethyl ether (1/1, 70 mL) to give crude compound 34 as a pale yellow solid, which was dissolved in DMF (20 mL). Diisopropylamine (20 mL) was added, and the mixture was stirred at room temperature for 2 h. Then, the reaction solution was concentrated under reduced pressure to about 20 mL and diluted with DMF (20 mL). Fmoc-Gly-Gly-OH (0.9 g) and DIEA (0.9 mL) were added, followed by the addition of HATU (1.0 g). The mixture was stirred at room temperature. After 20 min, the reaction mixture was diluted with EtOAc (80 mL). The mixture was washed with 0.5 M hydrochloric acid (80 mL) and water (100 mL). The organic layer was dried (over $Na_2SO_4$) and concentrated to dryness by evaporation under reduced pressure. The crude product was purified by column chromatography to give compound 35 (1.7 g) as a pale yellow solid.

Compound 35 (1.2 g) was dissolved in DMF (10 mL). DIEA (0.2 mL) and bis-PNP carbonate (0.8 g) were added to the solution, and the mixture was stirred at room temperature for 16 h. The reaction mixture was then diluted with EtOAc (100 mL) and washed with water (3 × 50 mL). The organic layer was dried (over $Na_2SO_4$) and concentrated to dryness by evaporation under reduced pressure. The crude product was purified by column chromatography to give compound 36 (1.2 g) as a white solid.

DIEA (0.018 mL) was added to a solution of compound 36 (0.12 g) and MMAE (0.072 g) in DMF (2 mL), and the reaction mixture was stirred at room temperature for 24 h. The mixture was then diluted with EtOAc (30 mL) and washed with hydrochloric acid (0.5 M, 30 mL). The organic layer was washed with water (20 mL), dried (over $Na_2SO_4$), and concentrated to dryness by evaporation under reduced pressure to give crude compound 37, which was redissolved in MeOH (3 mL), and MeONa (4.4 M in MeOH, 0.1 mL) was added. The reaction mixture was stirred at room temperature for 2 h and then neutralized with 1 N hydrochloric acid (0.5 mL). The mixture was directly purified by RP-HPLC to give compound 38 (TFA salt, 75 mg) as a white solid.

6-Maleimidohexanoic acid N-hydroxysuccinimide ester (Sigma Aldrich, 8 mg) was added to a solution of compound 47 (28 mg, TFA salt) in DMF (2 mL), followed by the addition of DIEA (0.007 mL). The mixture was stirred at room temperature. After 30 min, the mixture was purified by RP-HPLC to give **compound 39** (23 mg) as a white solid.
MS: m/z 1499.9 $[M+H]^+$.

### 3.9 Synthesis of compound 48 (MC-GGY-(Glc)-PAB-MMAE)

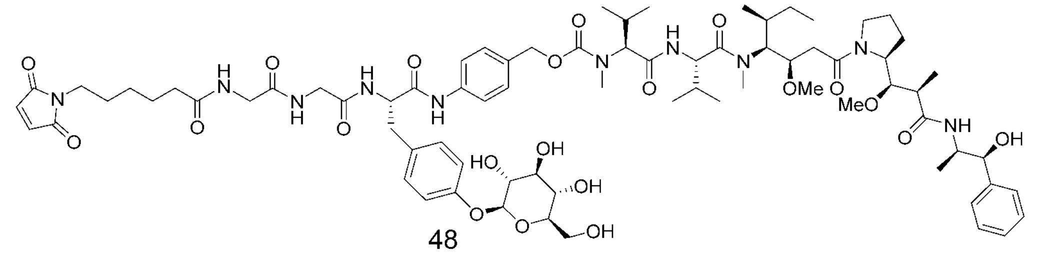

48

The synthesis process is as follows:

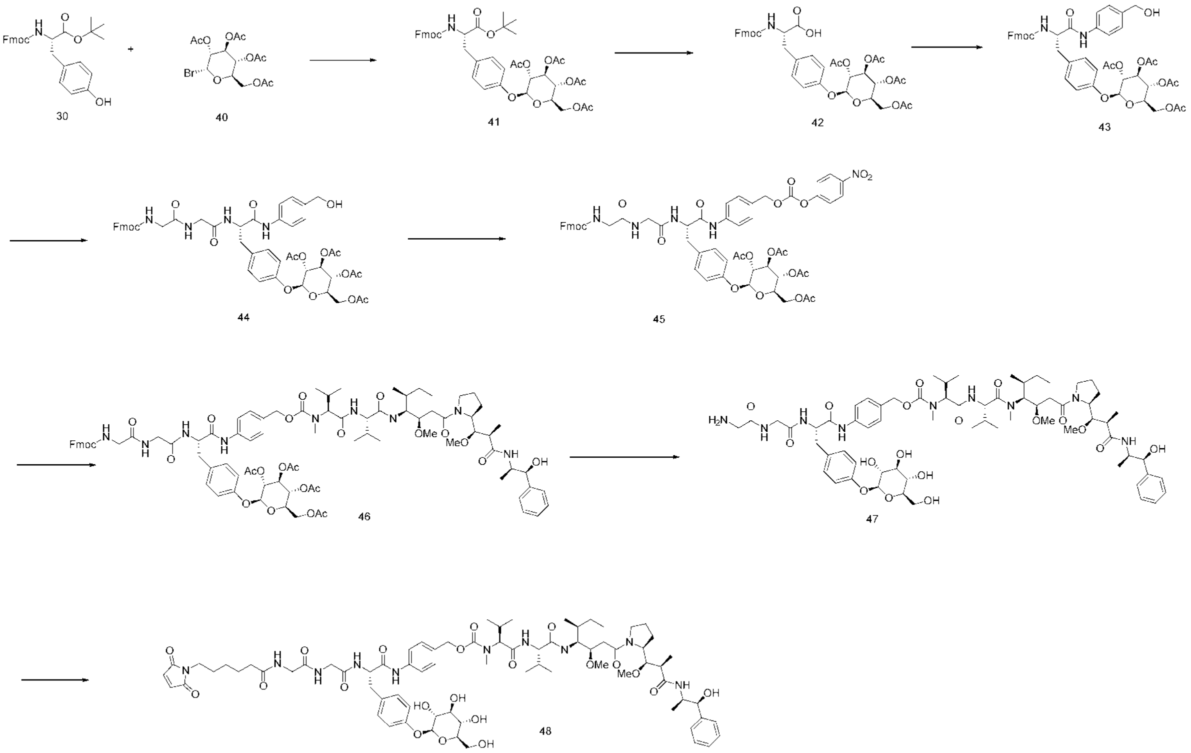

30, 40, 41, 42, 43, 44, 45, 46, 47, 48

Under argon atmosphere, $Ag_2O$ (15 mmol) was added to a stirred solution of acetobromo-α-D-glucose (compound 40, 4.6 g) and Fmoc-Tyr-OtBu (tert-butyl fluorenylmethoxycarbonyl tyrosinate, compound 30, 4.1 g) in anhydrous acetonitrile (100 mL) at 0 °C. The solution was stirred at room temperature for 4 h. The mixture was filtered, and the filtrate was concentrated to dryness by evaporation under reduced pressure. The residue was purified by silica gel column chromatography to give compound 41 (5.3 g) as a white solid.
Compound 41 (4 g) was redissolved in TFA/DCM (1/1, v/v, 80 mL). After 30 min, the mixture was diluted with DCM (200 mL) and washed with water (50 mL × 4). The organic layer was concentrated to dryness by evaporation to give a crude acid, compound 42, which was dissolved in DMF (50 mL). 4-Aminobenzyl alcohol (0.65 g) was added to the solution, followed by the addition of DIEA (1.8 mL) and HATU (1.9 g). The reaction mixture was stirred at room temperature for 20 min and then diluted with EtOAc (200 mL). The mixture was washed with 0.5 M hydrochloric acid (100 mL) and water (150 mL). The organic layer was dried (over $Na_2SO_4$) and concentrated to dryness by evaporation under reduced pressure. The residue was triturated with hexane/diethyl ether (1/1, 100 mL) to give crude compound 43 as a tan solid, which was dissolved in DMF (30 mL). Diisopropylamine (30 mL) was added, and the mixture was stirred at room temperature for 2 h.

The reaction mixture was then concentrated under reduced pressure to about 20 mL and diluted with DMF (30 mL). Fmoc-Gly-Gly-OH (1.8 g) and DIEA (1.8 mL) were added, followed by the addition of HATU (1.9 g). The mixture was stirred at room temperature. After 20 min, the reaction mixture was diluted with EtOAc (150 mL). The mixture was washed with 0.5 M hydrochloric acid (150 mL) and water (200 mL). The organic layer was dried (over $Na_2SO_4$) and concentrated to dryness by evaporation under reduced pressure. The crude product was purified by column chromatography to give compound 44 (3.2 g) as a pale yellow solid.

Compound 44 (2.4 g) was dissolved in DMF (20 mL). DIEA (0.5 mL) and bis(nitrophenyl)carbonate (1.5 g) were added to the solution, and the mixture was stirred at room temperature for 16 h. The reaction mixture was then diluted with EtOAc (200 mL) and washed with water (3 × 100 mL). The organic layer was dried (over $Na_2SO_4$) and concentrated to dryness by evaporation under reduced pressure. The residue was purified by column chromatography to give compound 45 (2.3 g) as a white solid.

DIEA (0.018 mL) was added to a solution of compound 45 (0.12 g) and MMAE (0.072 g) in DMF (2 mL), and the reaction mixture was stirred at room temperature for 24 h. The mixture was then diluted with EtOAc (30 mL) and washed with hydrochloric acid (0.5 M, 30 mL). The organic layer was washed with water (20 mL), dried (over $Na_2SO_4$), and concentrated to dryness by evaporation under reduced pressure to give crude compound 46, which was redissolved in MeOH (3 mL) and MeONa (4.4 M in MeOH, 0.1 mL). The reaction mixture was stirred at room temperature for 2 h and then neutralized with 1 N hydrochloric acid (0.5 mL). The mixture was directly purified by RP-HPLC to give compound 47 (TFA salt, 72 mg) as a white solid.

6-Maleimidohexanoic acid N-hydroxysuccinimide ester (Sigma Aldrich, 8 mg) was added to a solution of compound 47 (28 mg, TFA salt) in DMF (2 mL), followed by the addition of DIEA (0.007 mL). The mixture was stirred at room temperature. After 30 min, the mixture was purified by RP-HPLC to give **compound** 48 (24 mg) as a white solid.

MS: m/z 1499.9 $[M+H]^+$.

### 3.10 Synthesis of compound 55 (MC-AAN-(GlcNAc)-PAB-MMAE)

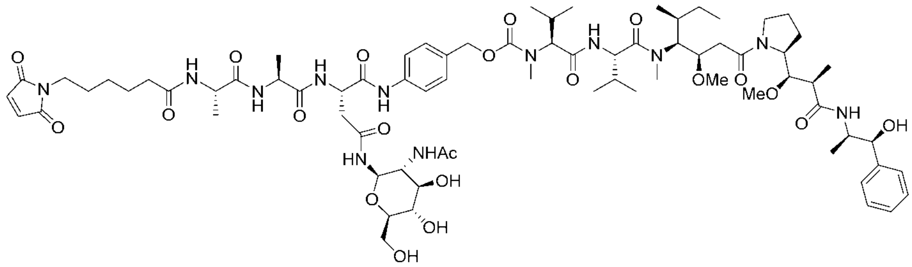

55

The synthesis process is as follows:

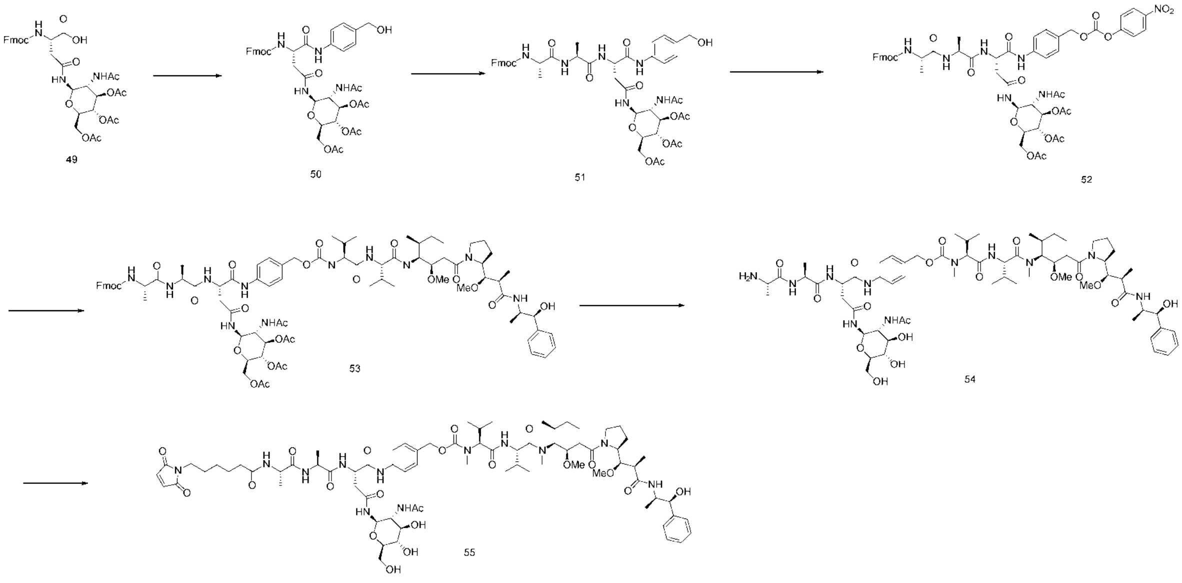

49 50 51 52 53 54 55

DIEA (0.035 mL) was added to a solution of compound 49 (AA Block, 68 mg) and 4-aminobenzyl alcohol (13 mg) in DMF (2 mL), followed by the addition of HATU (40 mg). The reaction mixture was stirred at room temperature for 20 min and then diluted with EtOAc (50 mL). The mixture was washed with 0.5 M hydrochloric acid (30 mL) and water (30 mL). The organic layer was dried (over $Na_2SO_4$) and concentrated to dryness by evaporation under reduced pressure. The residue was triturated with diethyl ether (100 mL) to give crude compound 50 as a tan solid, which was dissolved in DMF (4 mL). Diisopropylamine (4 mL) was added, and the mixture was stirred at room temperature for 2 h. The reaction mixture was then concentrated under reduced pressure to about 3 mL. Fmoc-Ala-Ala-OH (fluorenylmethoxycarbonyl-alanine-alanine, 40 mg) and DIEA (0.035 mL) were added, followed by the addition of HATU (0.04 g). The mixture was stirred at room temperature. After 20 min, the reaction mixture was diluted with EtOAc (40 mL). The mixture was washed with 0.5 M hydrochloric acid (30 mL) and water (50 mL). The organic layer was dried (over $Na_2SO_4$) and concentrated to dryness by evaporation under reduced pressure. The crude product was purified by RP-HPLC to give compound 51 (82 mg) as a pale yellow solid.

Compound 51 (80 mg) was dissolved in DMF (2 mL). DIEA (0.01 mL) and bis-PNP carbonate (60 mg) were added to the solution, and the mixture was stirred at room temperature for 16 h. The reaction mixture was then diluted with EtOAc (50 mL) and washed with water (3 × 15 mL). The organic layer was dried (over $Na_2SO_4$) and concentrated to dryness by evaporation under reduced pressure. The crude product was purified by RP-HPLC to give compound 52 (78 mg) as a white solid.

DIEA (0.01 mL) was added to a solution of compound 52 (55 mg) and MMAE (0.036 g) in DMF (2 mL), and the reaction mixture was stirred at room temperature for 24 h. The mixture was then diluted with EtOAc (30 mL) and washed with hydrochloric acid (0.5 M, 30 mL). The organic layer was washed with water (20 mL), dried (over $Na_2SO_4$), and concentrated to dryness by evaporation under reduced pressure to give crude compound 53, which was redissolved in MeOH (2 mL) and MeONa (4.4 M in MeOH, 0.05 mL). The reaction mixture was stirred at room temperature for 2 h and then neutralized with 1 N hydrochloric acid (0.5 mL). The mixture was directly purified by RP-HPLC to give compound 54 (TFA salt, 56 mg) as a white solid.

6-Maleimidohexanoic acid N-hydroxysuccinimide ester (Sigma Aldrich, 8 mg) was added to a solution of compound 54 (29 mg, TFA salt) in DMF (2 mL), followed by the addition of DIEA (0.007 mL). The mixture was stirred at room temperature. After 30 min, the mixture was purified by RP-HPLC to give **compound 55** (21 mg) as a white solid.

MS: m/z 1519.9 $[M+H]^+$.

### 3.11 Synthesis of compound 56 (BrAc-GGY(Glc)-PAB-MMAE)

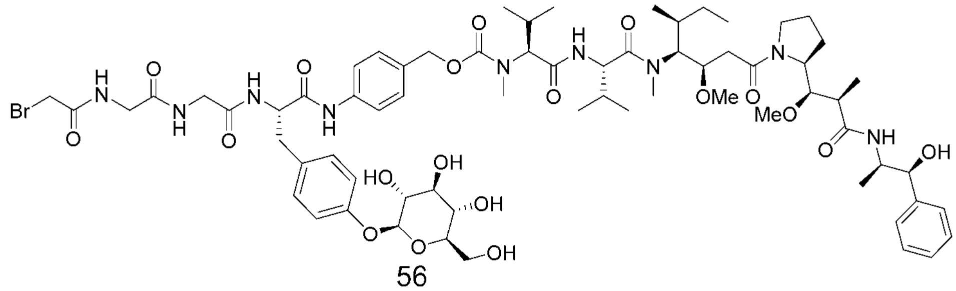

56

The synthesis process is as follows:

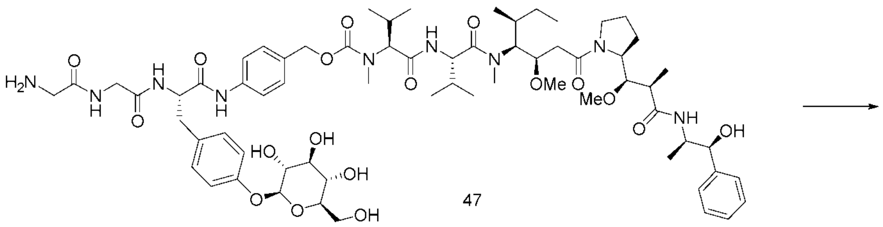

47

56

A saturated $NaHCO_3$ solution (0.04 mL) and bromoacetic anhydride (10 mg) were added to a solution of compound 47 (28 mg) in acetonitrile/water (6/4, v/v, 2 mL). The reaction mixture was stirred at room temperature for 10 min. The crude mixture was purified by RP-HPLC and lyophilized to give **compound 56** (27 mg) as a white solid.

MS: 1426.80 $[M+H]^+$.

**3.12 Synthesis of compound 17 (BrAc-PEG4-GlcA-MMAE)**

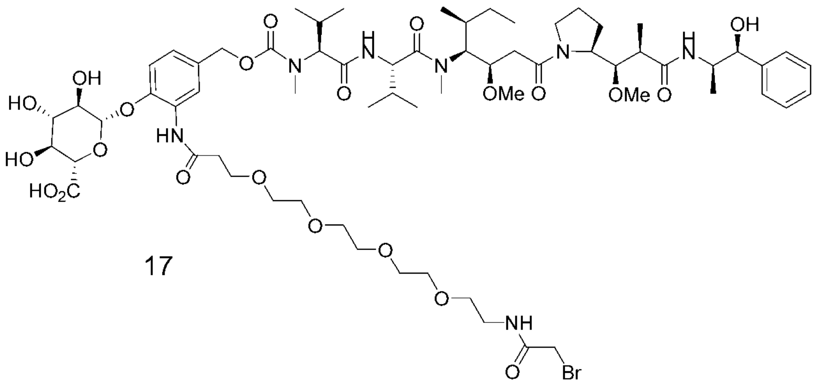

17

The synthesis process is as follows:

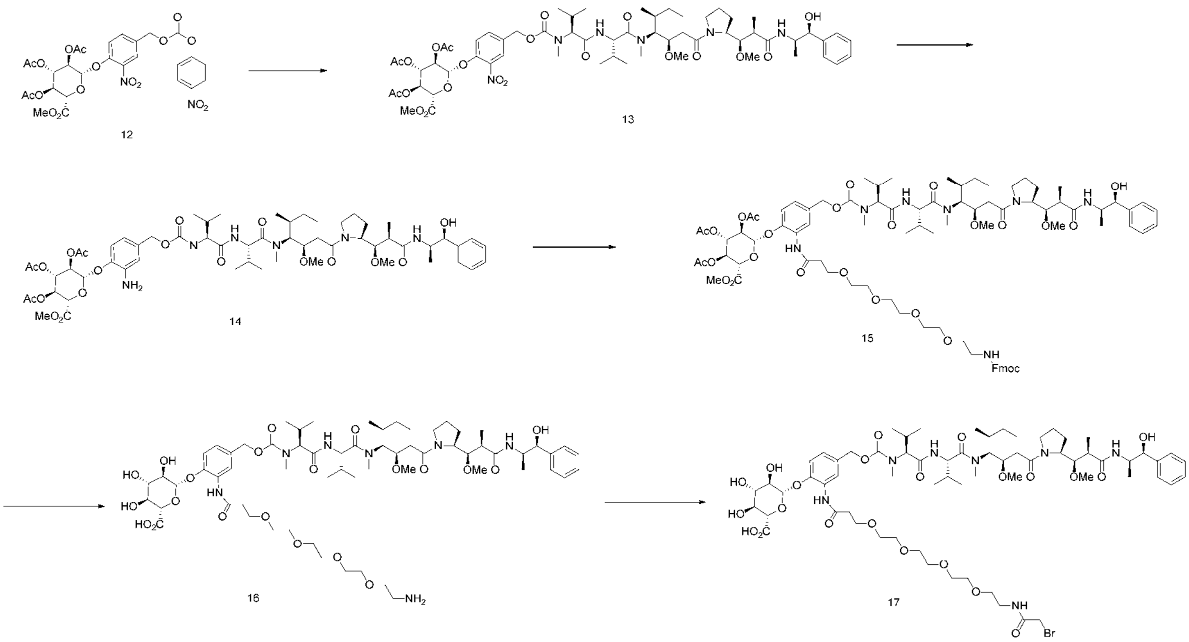

12 13 14 15 16 17

DIEA (0.02 mL) was added to a solution of compound 12 (65 mg, prepared with reference to WO2022026915) and MMAE (72 mg) in anhydrous DMF (2 mL), followed by the addition of HOBt (3 mg). The reaction mixture was stirred at room temperature (22 °C) for 18 h and diluted with water (20 mL). The reaction mixture was extracted with diethyl ether (40 mL). The organic phase was dried over $Na_2SO_4$ and concentrated to dryness under reduced pressure to give crude compound 13, which was finally dissolved in methanol (2 mL). Zinc powder (200 mg) was added to the methanol solution containing compound 13, followed by the addition of formic acid (0.2 mL). The reaction mixture was stirred at room temperature for 30 min. The solid was removed by filtration, and the filtrate was directly purified by RP-HPLC and lyophilized to give compound 14 (72 mg) as a white solid. DIEA (0.025 mL) was added to a solution of compound 14 (TFA salt, 66 mg) and Fmoc-NH-PEG4-COOH (purchased from PurePEG, Cat. No. 433704, 25 mg) in anhydrous DMF (2 mL), followed by the addition of HATU (20 mg). The mixture was stirred at room temperature. After 16 h, the crude mixture was purified by RP-HPLC and lyophilized to give compound 15 (72 mg) as a white powder.

MS: 1471.6 $[M+H]^+$.

Compound 15 (70 mg) was dissolved in an acetonitrile/water (6/4, v/v, 3 mL) solution, and NaOH (aq., 1 M, 0.3 mL) was added. The reaction mixture was stirred at room temperature (22 °C) to give compound 16. After 8 h, hydrochloric acid (1 M, 0.12 mL) was added to the crude product of compound 16, followed by the addition of bromoacetic anhydride (14 mg). The crude reaction mixture was directly purified by RP-HPLC and lyophilized to give **compound 17** (46 mg) as a white solid.

MS: 1426.7 $[M+H]^+$.

### 3.13 Synthesis of compound 20 (BrAc-PEG4-PAB-MMAE)

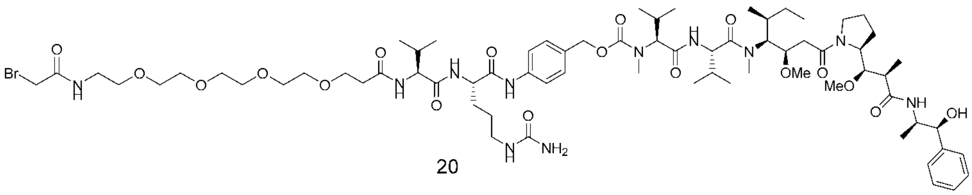

20

The synthesis process is as follows:

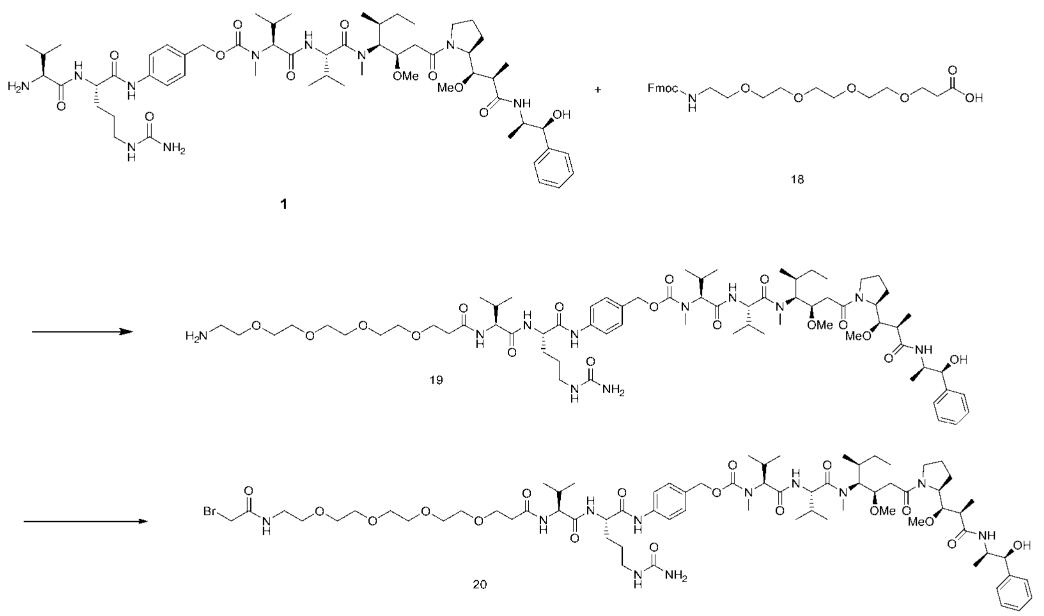

1 + 18

19

20

DIEA (0.025 mL) was added to a solution of compound 1 (62 mg) and Fmoc-NH-PEG4-COOH (compound 18, 25 mg, purchased from PurePEG, Cat. No. 433704-1H) in anhydrous DMF (1 mL), followed by the addition of HATU (20 mg). The reaction mixture was stirred at room temperature (22 °C). After 15 min, piperidine (0.1 mL) was added, and the mixture was reacted for another 30 min. The crude reaction mixture was directly purified by RP-HPLC and lyophilized to give compound 19 (61 mg, TFA salt) as a white solid.

A saturated $NaHCO_3$ solution (0.03 mL) and bromoacetic anhydride (7 mg) were added to a solution of compound 19 (37 mg) in acetonitrile/water (6/4, v/v, 2 mL). The reaction mixture was stirred at room temperature for 10 min. The crude mixture was purified by RP-HPLC and lyophilized to give **compound 20** (32 mg) as a white solid.

MS: 1491.0 $[M+H]^+$.

**Example 4. Preparation and Physicochemical Characterization of Antibody-Drug Conjugates**

The antibody prepared in Example 1 was treated with a reducing agent, such as tris(2-carboxyethyl)phosphine hydrochloride (TCEP) or dithiothreitol (DTT), to reduce part or all of the cysteine disulfide residues to form a highly nucleophilic cysteine sulfhydryl ($-CH_2SH$). Therefore, the partially or fully reduced antibody was reacted with a drug-linker or linker reagent containing an electrophilic functional group such as maleimide or $\alpha$-carbonyl.

For example, the antibody A was dissolved in PBS at pH 7.2, 2 mM EDTA was added, and the antibody was reduced using TCEP (the molar ratio of TCEP:Ab was 2.8:1). After incubation at 37 °C for about 120 min, the drug-linker compound 22, 29, 39, 48, or 55 was added to the reduced antibody (the molar ratio of drug-linker:antibody was 5:1), and 5% (v/v) DMSO was added. After incubation at room temperature for about 1 h, the mixture was purified by elution with the G25 resin, desalted into a phosphate buffer (pH 7.4), filtered through a 0.2 $\mu$m filter under sterile conditions, and cryopreserved. The average DAR value of the ADC was determined to be between 3.5 and 4.0 by hydrophobic interaction chromatography-high performance liquid chromatography.

As another example, the antibody A was dissolved in a phosphate buffer at pH 8.0, 2 mM EDTA was added, and the antibody was reduced using TCEP (the molar ratio of TCEP:Ab was 2.8:1). After incubation at 37 °C for about 120 min, the drug-linker compound 10, 11, 17, 56, or 20 was added to the reduced antibody (the molar ratio of drug-linker: antibody was 5:1), and 5% (v/v) DMSO was added. After incubation at room temperature for about 1 h, the mixture was purified by elution with the G25 resin, desalted, filtered through a 0.2 $\mu$m filter under sterile conditions, and cryopreserved. The average DAR value of the ADC was determined to be between 3.5 and 4.0 by hydrophobic interaction chromatography-high performance liquid chromatography.

As another example, the antibody A was dissolved in PBS at pH 7.2, the drug-linker compound 3, 4, or 5 was added to the antibody (the molar ratio of drug-linker:antibody was 3:1), and 10% (v/v) DMSO was added. After incubation at room temperature for about 2 h, the addition of the drug linker was repeated, and the mixture was reacted at room temperature for another 2 h. The final molar ratio of drug-linker:antibody was 6:1. The mixture was purified by elution with the G25 resin, desalted, filtered through a 0.2 $\mu$m filter under sterile conditions, and cryopreserved. The average DAR value of the ADC was determined to be between 1.5 and 1.9 by hydrophobic interaction chromatography-high performance liquid chromatography.

Determination method for DAR value:

The DAR values of the ADCs of the present disclosure were analyzed using hydrophobic interaction chromatography-high performance liquid chromatography (HIC-HPLC). ADCs were separated in a chromatographic column using an MabPac HIC-Butyl analytical column (4.6 × 100 mm, 5 μm, Cat. No. 088558, ThermoFisher, USA). 25 mM sodium phosphate buffer (pH 6.8) containing 1.5 M ammonium sulfate was used as a buffer A, 25 mM sodium phosphate buffer (pH 6.8) containing 25% acetonitrile was used as a buffer B, and 85% buffer A and 15% buffer B were stabilized as initial conditions. Linear gradient elution was performed for 30 min using 85% buffer A and 15% buffer B compared to 5% buffer A and 95% buffer B, and an additional elution was performed for 5 min using 5% buffer A and 95% buffer B. The flow rate and temperature settings were 0.5 mL/min and 25 °C. The ADC drug distribution was detected at 214 nm and 280 nm and used to calculate DAR values.

Illustratively, some of the prepared antibody-drug conjugates (ADCs) are shown in Table 3.

Table 3. ADCs prepared in the present disclosure

| Name of ADC | DAR | Compound | Structural schematic |
|---|---|---|---|
| A-McMMAE | 3.5-4 | Compound 22 | 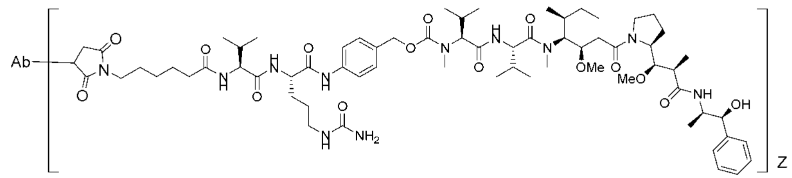 |
| A-BrAcMMAE | 3.5-4 | Compound 11 | 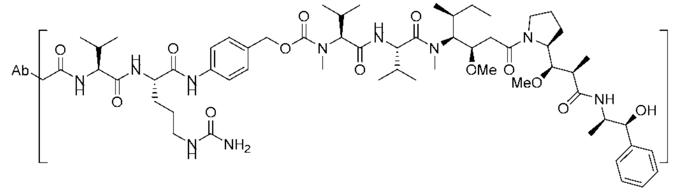 |
| IgG-BrAcMMAE | 3.5-4 | Compound 11 | 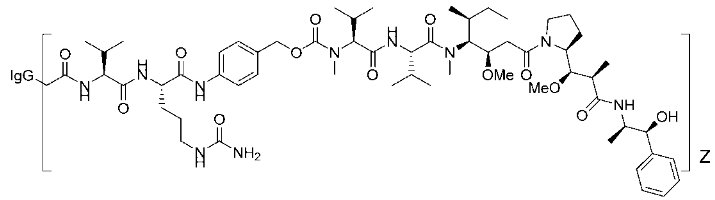 |
| A-BrAc-GGY(Glc)-PAB-MMAE | 3.5-4 | Compound 56 | 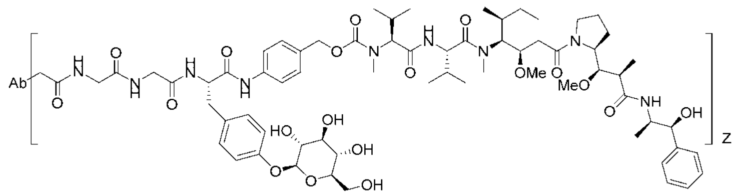 |
| A-BrAc-GlcA-PAB-MMAE | 3.5-4 | Compound 10 | 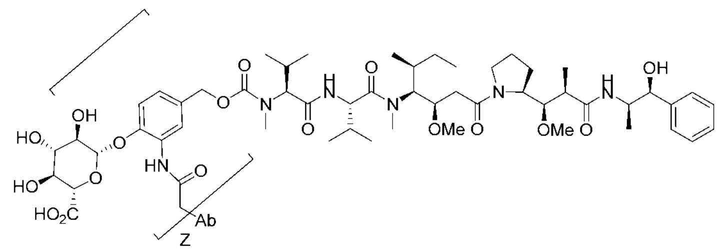 |

(continued)

| Name of ADC | DAR | Compound | Structural schematic |
|---|---|---|---|
| A-BrAc-PEG4-PAB-MMAE | 3.5-4 | Compound 20 | 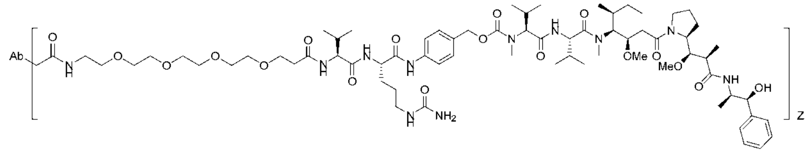 |
| A-BrAc-PEG4-GlcA-MMAE | 3.5-4 | Compound 17 | 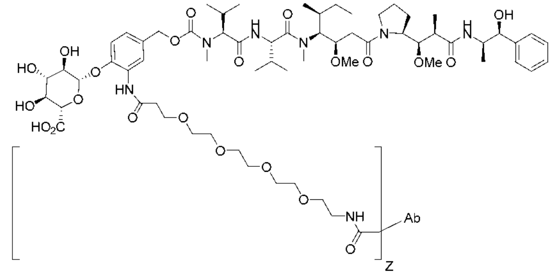 |
| A-MC-GGY(Gal)-PAB-MMAE | 3.5-4 | Compound 39 | 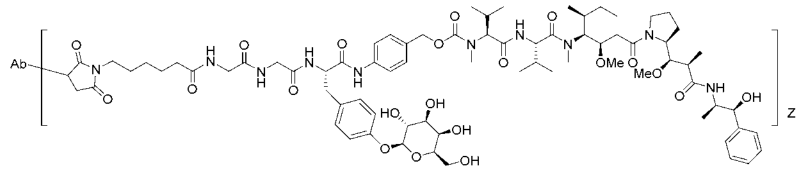 |
| A-MC-GGY-PAB-MMAE | 3.5-4 | Compound 29 | 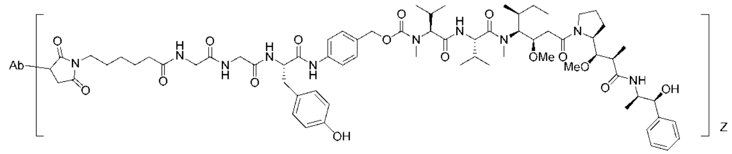 |
| A-MC-GGY-(Glc)-PAB-MMAE | 3.5-4 | Compound 48 | 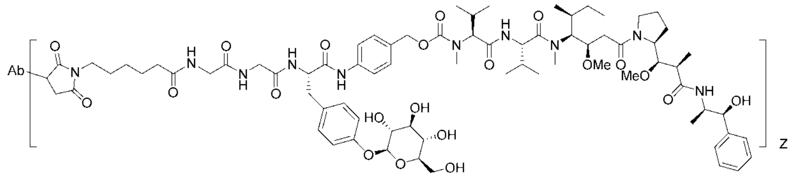 |

(continued)

| Name of ADC | DAR | Compound | Structural schematic |
|---|---|---|---|
| A-MC-AAN-(GlcNAc)-PAB-MMAE | 3.5-4 | Compound 55 | 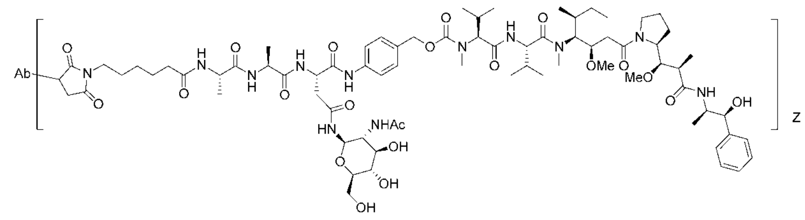 |
| L-McMMAE | 3.5-4 | Compound 22 | 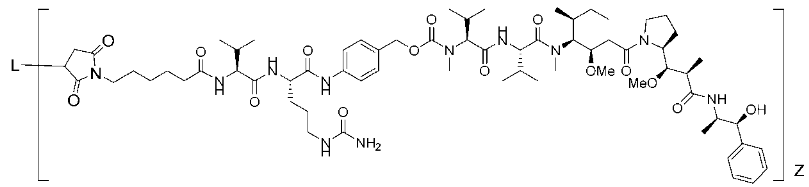 |
| A-C4-VC-PAB-MMAE | 1.5-1.9 | Compound 3 | 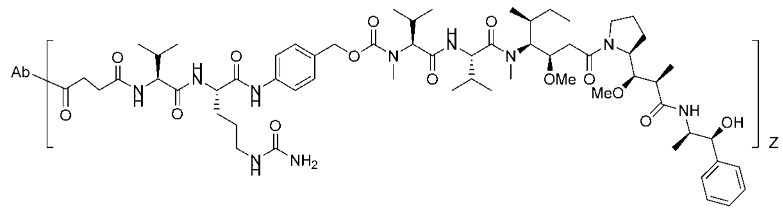 |
| A-C6-VC-PAB-MMAE | 1.5-1.9 | Compound 4 | 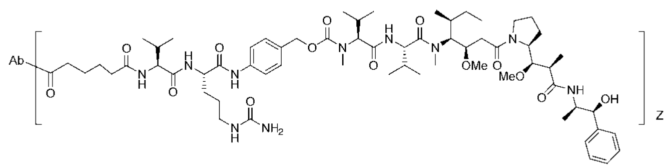 |
| A-PEG3-VC-PAB-MMAE | 1.5-1.9 | Compound 5 | 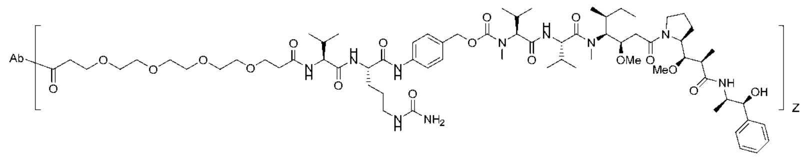 |

### Preparation of control L-MMMAE

Ladiratuzumab antibody, abbreviated as L, was prepared with reference to the patent US2013259860A1. The heavy chain sequence of this antibody is set forth in SEQ ID NO: 33, and the light chain sequence is set forth in SEQ ID NO: 34. The antibody L was conjugated to MC-MMAE to obtain L-Mc-MMAE with a DAR of 3.5-4 for the subsequent *in vivo* efficacy test.

### Example 5. Binding Activity of Antibody-Drug Conjugate to LIV-1 Antigen

hLIV-1 was diluted to 2 μg/mL with a coating buffer, and the resulting dilution was added to a microplate for coating at 4 °C for 15-20 h. 300 μL of a blocking solution was added to each well for blocking at room temperature for 1 h. The test antibody-drug conjugate was diluted with a diluent and incubated at room temperature for 1 h. The mouse anti-toxin monoclonal antibody was diluted with a diluent and incubated at room temperature for 1 h. The goat anti-mouse Fc domain secondary antibody was diluted with a diluent and incubated at room temperature for 1 h. After the incubation of the secondary antibody was completed, the TMB chromogenic solution was added for incubation at room temperature for 1-10 min. After the color development was completed, a stop solution (4 M sulfuric acid) was added at 50 μL/well to stop the substrate reaction. The absorbance of each well was measured at a detection wavelength of 450 nm on a microplate reader, and the results were calculated. The data were analyzed using Prism software, and a dose-response curve was plotted using a Sigmoidal, 4PL four-parameter equation with the protein concentration of the naked antibody A as the abscissa and the corresponding average absorbance as the ordinate. The equation is Y = Bottom + (X^Hillslope)×(Top - Bottom)/(X^HillSlope + EC50^HillSlope).

The results are shown in FIG. 2. The antibody-drug conjugate of the present disclosure specifically binds to the LIV-1 antigen.

### Example *6. In Vitro* Killing Effects of Antibody-Drug Conjugates

The ADC according to the present disclosure was tested *in vitro* for its potency and selectivity by determining its cytotoxicity in relevant cancer cell lines, such as those expressing the antigen corresponding to the antibody moiety of the ADC and similar cancer cell lines lacking the antigen.

In this experiment, the inhibitory effect of an anti-Liv1 antibody conjugate, such as A-BrAcMMAE, on the proliferation of various tumor cell lines was studied.

In this experiment, a CellTiterGlo2 (Promega) reagent was used to evaluate the antiproliferative effect of the drug. The thermotolerant luciferase contained in this reagent can catalyze the mono-oxidation of fluorescein in the presence of Mg2+, ATP produced by living cells, and molecular oxygen, producing a stable "glow-type" luminescent signal, thereby determining the number of living cells in the culture by quantifying ATP, a marker for metabolically active cells.

The cell lines selected in this experiment were as follows: a human lung cancer cell (undifferentiated) line Calu6, and human breast cancer cell lines overexpressing LIV-1, i.e., MCF7-ATCC-LIV-1#7, #12, and #17.

In an incubator at 37 °C with 5% $CO_2$, Calu6 cells were incubated in an RPMI-1640 medium containing 10% fetal bovine serum, and MCF7-ATCC-LIV-1 overexpressing cell lines were incubated in an EMEM medium containing 10% fetal bovine serum and 1 μg/mL. The four types of cells were seeded at 50 μL/well into 96-well plates at a density of $2 \times 10^3$ to $5 \times 10^3$ cells/well. After 24 h of incubation, different concentrations of antibody A-MMAE conjugates or control antibody-MMAE conjugates (IgG1-BrAc-MMAE) diluted with a culture medium were added at 100 μL/well. Duplicate wells were set for each concentration, and vehicle control and cell-free culture medium wells at corresponding concentrations were set. After 96 h of incubation in an incubator at 37 °C with 5% $CO_2$, 100 μL of CellTiterGlo2 was added to each well. The plates were placed on an orbital shaker for mixing at room temperature for 15 min. The glow values were determined, and the $IC_{50}$ values (nM) of the anti-LIV-1 antibody A-BrAcMMAE conjugate for various cells were calculated (Table 3). The calculation results and the inhibitory effect on the proliferation of the four types of tumor cells are shown in FIGs. 3, 4, 5, and 6 and Table 4-1.

Table 4-1. $IC_{50}$ values (nM) for inhibition of proliferation of each cell line by MMAE conjugates of antibody A and control antibody without binding function

| $IC_{50}$ (nM) | Calu6 | MCF7-Liv1 #7 | MCF7-Liv1 #12 | MCF7-Liv1 #17 |
|---|---|---|---|---|
| A-BrAc-MMAE | 11.0 | 0.03 | 0.031 | 0.05 |
| IgG1-BrAc-MMAE | 40.7 | 46.0 | 19.5 | 51.2 |

As can be seen from Table 4-1 and FIGs. 3, 4, 5, and 6, A-BrAcMMAE exhibited significant killing effects on the three tumor cells with different expression levels of LIV-1, and the killing effect was in direct proportion to the expression level of LIV-1. The conjugate also exhibited significant inhibitory effects on two different cancer cell lines derived from breast cancer and lung cancer.

In addition, the present disclosure also detected the *in vitro* killing activity of ADCs conjugated with toxins and the antibody A using different linkers on different cells. The results are shown in Tables 4-2- and 4-3.

Table 4-2. $IC_{50}$ values (nM) of ADCs in inhibiting proliferation of MCF7-Liv1 #17 cells

| **Experiment 2** | |
|---|---|
| **ADC** | **$IC_{50}$ (nM)** |
| A-BrAc-MMAE) | 0.025 |
| A-BrAc-GGY(Glc)-PAB-MMAE | 0.057 |
| **Experiment 3** | |
| **ADC** | **$IC_{50}$ (nM)** |
| A-BrAc-MMAE | 0.0041 |
| A-McMMAE | 0.0013 |
| A-BrAc-GlcA-PAB-MMAE | 0.0037 |

Table 4-3. $IC_{50}$ values (nM) of ADCs in inhibiting proliferation of Calu6 cells

| **ADC** | **$IC_{50}$ (nM)** |
|---|---|
| A-BrAc-MMAE | 2.21 |
| A-BrAc-PEG4-PAB-MMAE | 1.36 |
| A -BrAc-PEG4-GlcA-MMAE | 3.52 |

In conclusion, the anti-LIV-1 antibody-MMAE conjugates described in the present disclosure have significant anti-tumor activity, have good targeting property, and can transport a small molecule toxic drug to a tumor site, thereby providing a novel antibody-drug conjugate for treating LIV-1-positive breast cancer.

**Example 7. *In Vivo* Efficacy Study of Antibody-Drug Conjugates**

Tumor cells grown in the implantation culture of CD1 athymic nude mice: Calu-6 ($2.5 \times 10^6$ cells in 50% matrigel) from ATCC, PC-3 ($2.5 \times 10^6$ cells) from ATCC, and PA-1 ($5 \times 10^6$ cells in 50% matrigel) from ATCC. Tumor cells grown in the implantation culture of NSG mice: HCC1806 ($2.5 \times 10^6$ cells in 50% matrigel) from ATCC and MCF-7 ($10 \times 10^6$ cells; for the *in vivo* growth of MCF-7, 8.5 μg/mL estradiol was added to the drinking water of female mice) from NCI.

When the tumors grew to 150 $mm^3$, the humanized LIV-1 ADC or the non-binding control ADC (1 mg/kg, 3 mg/kg, 6 mg/kg, or 10 mg/kg) was administered by intravenous injection once every four days for a total of four administrations (q4d $\times$ 4), by intravenous injection once every week for a total of 2 administrations (qw $\times$ 2), or by single intravenous injection. The tumor volume was monitored using a caliper, and the animals were euthanized when the tumor volume reached about 2000 $mm^3$. Tumor volume plots were continued for each group until one or more animals were euthanized. All animal procedures were conducted in a facility approved by the Association for Assessment and Accreditation of Laboratory Animal Care in accordance with protocols approved by the Institutional Animal Care and Use Committee. TGI was calculated as follows:

$$\text{TGI}(\%) = 1 - [(\text{Td} - \text{T0})/(\text{Cd} - \text{C0})] \times 100\%$$

where Td and Cd are the average tumor volumes of the treatment and control groups on the day of tumor volume measurement, and T0 and C0 are the average tumor volumes of the treatment and control groups on day 0.

The results are shown in FIGs. 7-11. In the MCF7 efficacy study, the tumors in 4 mice completely regressed on day 22 after administration. On day 36 after administration, the TGI was 126.3%. In the HCC1806 efficacy study, the TGI was 102.99% on day 21 after administration. In the PC-3 efficacy study, the TGI was 101.5% on day 11 after administration. In the Calu-6 efficacy study, in the multiple-dose administration groups, on day 25 after administration, the TGI of the 3

mg/kg group was 75.14%, and the TGI of the 6 mg/kg group was 111.56%; and on day 28 after administration, the tumors in 7 mice in the 6 mg/kg group completely regressed. In the single-dose administration groups, on day 25 after administration, the TGI of the 6 mg/kg group was 52.72%, and the TGI of the 10 mg/kg group was 76.96%. The above results show that A-BrAcMMAE exhibits significant anti-tumor efficacy in different tumor models.

In addition, the present disclosure also detected the anti-tumor effects of A-BrAcMMAE and ADC ladiratuzumab vedotin targeting Liv-1 (L-Mc-MMAE for short) in the prior art in PC3 and PA-1 mouse tumor models. The results are shown in FIG. 12 and Tables 5-1 and 5-2.

Table 5-1. Tumor growth inhibition rates of different ADCs against PC3 mouse subcutaneous tumors

| ADC | Route and dose of administration | Tumor growth inhibition rate TGI% (day 24 after administration) |
|---|---|---|
| A-BrAcMMAE | Single dose of 10 mg/kg, intravenous injection | 116.19 |
| L-Mc-MMAE | Single dose of 10 mg/kg, intravenous injection | 103.92 |

Table 5-2. Tumor growth inhibition rates of ADCs against PA-1 mouse subcutaneous tumors

| ADC | Route and dose of administration | Tumor growth inhibition rate TGI% (day 13 after administration) |
|---|---|---|
| A-BrAcMMAE | Single dose of 3 mg/kg, intravenous injection | 93.77 |
| L-McMMAE | Single dose of 3 mg/kg, intravenous injection | 26.68 |

The above results indicate that A-BrAcMMAE can delay or inhibit the growth of LIV-1-expressing tumors in prostate cancer (PC-3) and ovarian cancer (PA-1) models, showing significant anti-tumor activity. Although it has been reported in the previous literature that L-McMMAE also had anti-tumor activity, A-BrAcMMAE was significantly more effective than L-McMMAE in the mouse tumor models. Thus, the A-BrAcMMAE antibody-drug conjugate can be used to treat various cancers expressing LIV-1.

Sequence information of the present disclosure:

SEQ ID NO: 1 (VH of antibody A)

EVQLVQSGAEVKKPGATVKISCKASGLNIEDYYMHWVQQAPGKGLEWMG
WIDPENGDTEYAEKFQGRVTITADTSTNTAYMELSSLRSEDTAVYYCTVHNAHYGTW
FAYWGQGTTVTVSS

SEQ ID NO: 2 (VL of antibody A)

DIVMTQTPLSLSVTPGQPASISCRSSQTLVRSDGNTYLEWYLQKPGQSPQLLI
YRVSNRFSGVPDRFSGSGSGTDFTLKISRVEAEDVGVYYCFQGSHVPYTFGGGTKLEIK

SEQ ID NO: 3 (VH of antibody B)

EVQLQQSGAELVRSGASVKLSCTASGFNIKDYYMHWVKQRPEQGLEWIGWI
DPENGDTEYAPKFQGKATLTADTSSNTAYLLLSSLTSEDTAVYYCKRRFYSMDYWGQ
GTSVTVSS

SEQ ID NO: 4 (VL of antibody B)

DVLMTQTPLSLPVSLGDQASISCRSSQSIVHSNGNTYLEWYLQKPGQSPKLLI YKVSNRFSGVPDRFSGSGSGTDFTLKISRVEAEDLGVYYCFQGSHVPLTFGAGTKLEL K

SEQ ID NO: 5 (VH of antibody C)

DVQLQESGPGLVKPSQSLSLTCTVTGYSITSDYAWNWIRQFPGNKLEWMGYI TYSGSTSYNPSLKSRISITRDTSKNQFFLQLNSVTTEDTATYYCAREGRLRPFAYWGQ GTLVTVSA

SEQ ID NO: 6 (VL of antibody C)

NIMMTQSPSSLAVSAGEKVTMSCKSSQSVLYSSNQKNYLAWYQQKPGQSPK LLIYWASTRESGVPDRFTGSGSGTDFTLTISSVQAEDLAVYYCHQYLSSWTFGGGTKL EIK

SEQ ID NO: 7 (antibody A VH CDR-H1)
DYYMH
SEQ ID NO: 8 (antibody A VH CDR-H2)
WIDPENGDTEYAEKFQG
SEQ ID NO: 9 (antibody A VH CDR-H3)
HNAHYGTWFAY
SEQ ID NO: 10 (antibody A VL CDR-L1)
RSSQTLVRSDGNTYLE
SEQ ID NO: 11 (antibody A VL CDR-L2)
RVSNRFS
SEQ ID NO: 12 (antibody A VL CDR-L3)
FQGSHVPYT
SEQ ID NO: 13 (antibody B VH CDR-H1)
DYYMH
SEQ ID NO: 14 (antibody B VH CDR-H2)
WIDPENGDTEYAPKFQG
SEQ ID NO: 15 (antibody B VH CDR-H3)
RFYSMDY
SEQ ID NO: 16 (antibody B VL CDR-L1)
RSSQSIVHSNGNTYLE
SEQ ID NO: 17 (antibody B VL CDR-L2)
KVSNRFS
SEQ ID NO: 18 (antibody B VL CDR-L3)
FQGSHVPLT
SEQ ID NO: 19 (antibody C VH CDR-H1)
SDYAWN
SEQ ID NO: 20 (antibody C VH CDR-H2)
YITYSGSTSYNPSLKS
SEQ ID NO: 21 (antibody C VH CDR-H3)
EGRLRPFAY
SEQ ID NO: 22 (antibody C VL CDR-L1)
KSSQSVLYSSNQKNYLA
SEQ ID NO: 23 (antibody C VL CDR-L2)
WASTRES
SEQ ID NO: 24 (antibody C VL CDR-L3)

HQYLSSWT

SEQ ID NO: 25 (amino acid sequence of LIV-1 antigen)

MARKLSVILILTFALSVTNPLHELKAAAFPQTTEKISPNWESGINVDLAISTRQ YHLQQLFYRYGENNSLSVEGFRKLLQNIGIDKIKRIHIHHDHDHHSDHEHHSDHERHS DHEHHSEHEHHSDHDHHSHHNHAASGKNKRKALCPDHDSDSSGKDPRNSQGKGAH RPEHASGRRNVKDSVSASEVTSTVYNTVSEGTHFLETIETPRPGKLFPKDVSSSTPPSV TSKSRVSRLAGRKTNESVSEPRKGFMYSRNTNENPQECFNASKLLTSHGMGIQVPLN ATEFNYLCPAIINQIDARSCLIHTSEKKAEIPPKTYSLQIAWVGGFIAISIISFLSLLGVILV PLMNRVFFKFLLSFLVALAVGTLSGDAFLHLLPHSHASHHHSHSHEEPAMEMKRGPLF SHLSSQNIEESAYFDSTWKGLTALGGLYFMFLVEHVLTLIKQFKDKKKKNQKKPEND DDVEIKKQLSKYESQLSTNEEKVDTDDRTEGYLRADSQEPSHFDSQQPAVLEEEEVMI AHAHPQEVYNEYVPRGCKNKCHSHFHDTLGQSDDLIHHHHDYHHILHHHHHQNHH PHSHSQRYSREELKDAGVATLAWMVIMGDGLHNFSDGLAIGAAFTEGLSSGLSTSVA VFCHELPHELGDFAVLLKAGMTVKQAVLYNALSAMLAYLGMATGIFIGHYAENVSM WIFALTAGLFMYVALVDMVPEMLHNDASDHGCSRWGYFFLQNAGMLLGFGIMLLISI FEHKIVFRINF

SEQ ID NO: 26 (heavy chain constant region)

ASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK

SEQ ID NO: 27 (light chain constant region)

RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGEC

SEQ ID NO: 28 (murine antibody mVH of antibody A, with CDRs underlined)

EVQLQQSGAELVRSGASVKVSCKASGLNIEDYYMHWVKQRPEQGLEWIGWIDPENGDTEYGPKFQGKATMTADTSSNTAYLQLSSLTSGDTAVYYCTVHNAHYGTWFAYWGQGTLVTVSS

SEQ ID NO: 29 (murine antibody mVL of antibody A, with CDRs underlined)

DVLMTQTPLSLPVSLGDQASISCRSSQTLVRSDGNTYLEWYLQKPGQSPKLLIYRVSNRFSGVPDRFSGSGSGTDFTLRISRVEAEDLGLYYCFQGSHVPYTFGGGTKLEIK

SEQ ID NO: 30 (light chain of antibody A)

DIVMTQTPLSLSVTPGQPASISCRSSQTLVRSDGNTYLEWYLQKPGQSPQLLIYRVSNRFSGVPDRFSGSGSGTDFTLKISRVEAEDVGVYYCFQGSHVPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

SEQ ID NO: 31 (heavy chain of antibody A)

EVQLVQSGAEVKKPGATVKISCKASGLNIEDYYMHWVQQAPGKGLEWMGWIDPENGDTEYAEKFQGRVTITADTSTNTAYMELSSLRSEDTAVYYCTVHNAHYGTWFAYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 32 (heavy chain variable region of antibody A-1):

EVQLVQSGAEVKKPGATVKISCKASGLNIEDYYMHWVQQAPGKGLEWMGWIDPENGDTEYAEKFQGRVTITADTSTDTAYMELSSLRSEDTAVYYCTVHNAHYGTWFAYWGQGTTVTVSS

SEQ ID NO: 33 (heavy chain of Ladiratuzumab):

QVQLVQSGAEVKKPGASVKVSCKASGLTIEDYYMHWVRQAPGQGLEWMG WIDPENGDTEYGPKFQGRVTMTRDTSINTAYMELSRLRSDDTAVYYCAVHNAHYGT WFAYWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALP APIEKTISKAKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYTQKSLSLSP GK

SEQ ID NO: 34 (light chain of Ladiratuzumab):

DVVMTQSPLSLPVTLGQPASISCRSSQSLLHSSGNTYLEWYQQRPGQSPRPLI YKISTRFSGVPDRFSGSGSGTDFTLKISRVEAEDVGVYYCFQGSHVPYTFGGGTKVEI KRRTVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC

All documents mentioned in the present disclosure are incorporated herein by reference, just as each document is individually cited as a reference. In addition, it should be understood that various modifications or changes may be made by those skilled in the art after reading the above teachings of the present disclosure, and these equivalent forms also fall within the scope defined by the appended claims of the present application.

## Claims

1. An anti-human LIV-1 antibody or antigen-binding fragment thereof, comprising a heavy chain variable region (VH) and a light chain variable region (VL), wherein the antibody is selected from antibody A, antibody B, and antibody C, wherein:
the VH of the antibody A comprises three CDRs, CDR-H1, CDR-H2, and CDR-H3, wherein:

    the CDR-H1 comprises the amino acid sequence set forth in SEQ ID NO: 7;
    the CDR-H2 comprises the amino acid sequence set forth in SEQ ID NO: 8;
    the CDR-H3 comprises the amino acid sequence set forth in SEQ ID NO: 9;
    and the VL of the antibody A comprises three CDRs, CDR-L1, CDR-L2, and CDR-L3, wherein:

        the CDR-L1 comprises the amino acid sequence set forth in SEQ ID NO: 10;
        the CDR-L2 comprises the amino acid sequence set forth in SEQ ID NO: 11;
        the CDR-L3 comprises the amino acid sequence set forth in SEQ ID NO: 12;
        the VH of the antibody B comprises three CDRs, CDR-H1, CDR-H2, and CDR-H3, wherein:

            the CDR-H1 comprises the amino acid sequence set forth in SEQ ID NO: 13;
            the CDR-H2 comprises the amino acid sequence set forth in SEQ ID NO: 14;
            the CDR-H3 comprises the amino acid sequence set forth in SEQ ID NO: 15;
            and the VL of the antibody B comprises three CDRs, CDR-L1, CDR-L2, and CDR-L3; wherein:

                the CDR-L1 comprises the amino acid sequence of SEQ ID NO: 16;
                the CDR-L2 comprises the amino acid sequence of SEQ ID NO: 17;

the CDR-L3 comprises the amino acid sequence of SEQ ID NO: 18; and
the VH of the antibody C comprises three CDRs, CDR-H1, CDR-H2, and CDR-H3, wherein:

the CDR-H1 comprises the amino acid sequence set forth in SEQ ID NO: 19;
the CDR-H2 comprises the amino acid sequence set forth in SEQ ID NO: 20;
the CDR-H3 comprises the amino acid sequence set forth in SEQ ID NO: 21;
and the VL of the antibody C comprises three CDRs, CDR-L1, CDR-L2, and CDR-L3, wherein:

the CDR-L1 comprises the amino acid sequence set forth in SEQ ID NO: 22;
the CDR-L2 comprises the amino acid sequence set forth in SEQ ID NO: 23;
the CDR-L3 comprises the amino acid sequence set forth in SEQ ID NO: 24.

**2.** The anti-human LIV-1 antibody or the antigen-binding fragment thereof according to claim 1, wherein

the VH of the antibody A comprises a sequence having at least 95% identity to the amino acid sequence of SEQ ID NO: 1, and the VL of the antibody A comprises a sequence having at least 95% identity to the amino acid sequence of SEQ ID NO: 2;
the VH of the antibody B comprises a sequence having at least 95% identity to the amino acid sequence of SEQ ID NO: 3, and the VL of the antibody B comprises a sequence having at least 95% identity to the amino acid sequence of SEQ ID NO: 4;
the VH of the antibody C comprises a sequence having at least 95% identity to the amino acid sequence of SEQ ID NO: 5, and the VL of the antibody C comprises a sequence having at least 95% identity to the amino acid sequence of SEQ ID NO: 6; or
the VH of the antibody A comprises a sequence having at least 95% identity to the amino acid sequence of SEQ ID NO: 32, and the VL of the antibody A comprises a sequence having at least 95% identity to the amino acid sequence of SEQ ID NO: 2.

**3.** The anti-human LIV-1 antibody or the antigen-binding fragment thereof according to claim 1, wherein the antibody is a humanized antibody, a chimeric antibody, or a murine antibody.

**4.** The anti-human LIV-1 antibody or the antigen-binding fragment thereof according to claim 1, wherein the antibody or antigen-binding fragment thereof is a tetramer comprising two light chains and two heavy chains, an antibody comprising one or more heavy chains or light chains, a Fab, a Fab', a $F(ab')_2$, an Fv, or a single-chain antibody; preferably, the antibody comprises a heavy chain constant region set forth in SEQ ID NO: 26 and/or a light chain constant region set forth in SEQ ID NO: 27; more preferably, the antibody comprises a light chain set forth in SEQ ID NO: 30 and a heavy chain set forth in SEQ ID NO: 31.

**5.** An antibody-drug conjugate (ADC) or pharmaceutically acceptable salt thereof, comprising the anti-human LIV-1 antibody or antigen-binding fragment thereof according to any one of claims 1-4.

**6.** The antibody-drug conjugate or the pharmaceutically acceptable salt thereof according to claim 5, wherein the antibody-drug conjugate or the pharmaceutically acceptable salt thereof comprises a structure represented by [Formula I] below:

[Formula I] Ab-[(AG)k-(L1)x-(OP)p-(L2)y-D]z

wherein:

Ab is the anti-human LIV-1 antibody or the antigen-binding fragment thereof according to any one of claims 1-4;
AG is a conjugating group, wherein:

k is 0 or 1; and
AG is selected from the following groups:

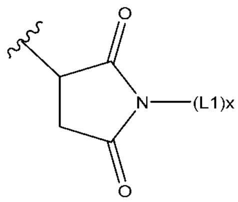
,
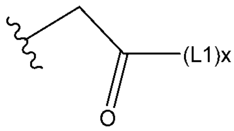
, or
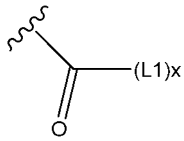
,

wherein the wavy line indicates the point of linkage to Ab;

(L1)x is a first linker group, wherein:

x is 0 or 1; and

L1 represents a chain containing 1 to 18 groups selected from $-CH_2-$, -C(=O)-, -NH-, -O-, and -S-, wherein at least two carbon atoms, preferably at least two $-CH_2-$, are contained between each NH, O, and S and another NH, O, or S;

(OP)p is an enzyme-cleavable oligopeptide, wherein p is 0 or 2-10;

(L2)y is a second linker group, wherein:

y is 0 or 1; and

L2 represents a chain containing 1 to 18 groups selected from $-CH_2-$, -C(=O)-, -NH-, -O-, and -S-, wherein at least two $CH_2$ groups are contained between each NH, O, and S and another NH, O, or S; and/or

L2 represents an o-, m-, or p-hydroxybenzyl or o-, m-, or p-aminobenzyl group;

preferably, y is 1, and L2 is $-NH-Ph-CH_2-O-C(=O)-$, wherein the benzene ring optionally contains a hydroxyl substituent; more preferably, L2 is p-aminobenzyloxycarbonyl or p-hydroxy-m-aminobenzyloxycarbonyl; optionally, a hydroxyl group on the benzene ring is glycosylated, preferably with glucuronic acid, N-acetylglucosamine, glucose, or galactose;

D is a drug of the antibody-drug conjugate, wherein the drug is a cytotoxic compound, an immunomodulator, or an enzyme or hormone inhibitor; and

z is a drug-to-antibody ratio and is an integer or decimal of 1-24.

**7.** The antibody-drug conjugate or the pharmaceutically acceptable salt thereof according to claim 6, wherein,

L1 is $-(CH_2-)o-C(=O)-$ or $-(NH)j-(CH_2CH_2O)n-(CH_2)q-(C-O)-$, wherein o is 1-10, preferably 1-7, j is 0-4, n is 2-8, and q is 1-6;

(OP)p is selected from oligopeptides formed from a combination of valine, citrulline, alanine, glycine, asparagine, tyrosine, phenylalanine, proline, isoleucine, lysine, serine, glutamic acid, threonine, or asparagine, and p is 2-10; more preferably, (OP)p is a dipeptide, a tripeptide, or a tetrapeptide; optionally, a phenolic hydroxyl group of tyrosine or an amide group of asparagine is glycosylated, preferably with glucuronic acid, N-acetylglucosamine, glucose, or galactose;

preferably,

the antibody-drug conjugate of [Formula I] or the pharmaceutically acceptable salt thereof has a structure selected from the following:

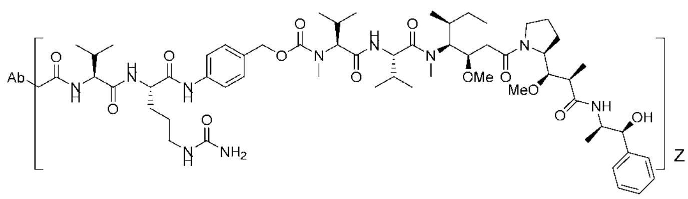
,

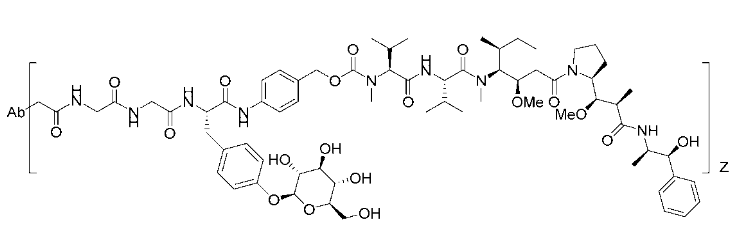
,

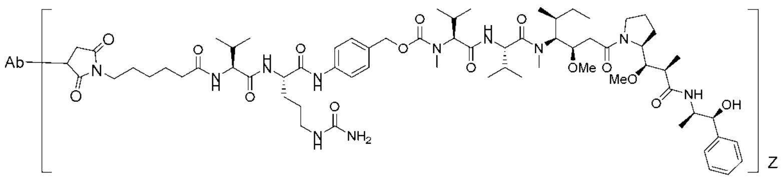

,

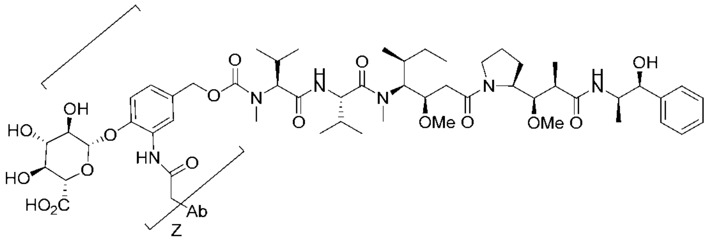

,

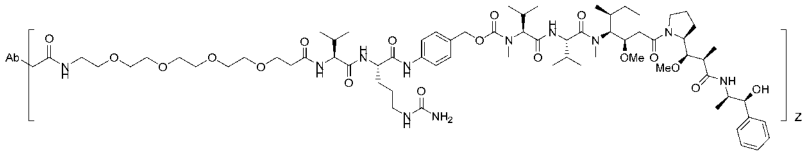

,

and

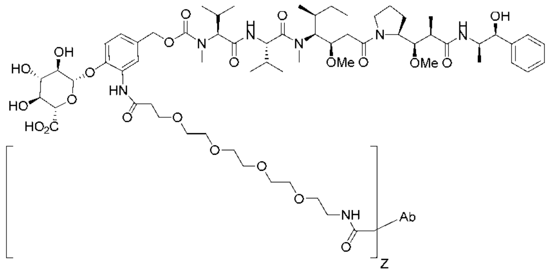

;

more preferably, Ab comprises a light chain set forth in SEQ ID NO: 30 and a heavy chain set forth in SEQ ID NO: 31, and z is a decimal or integer of 2-8.

**8.** The antibody-drug conjugate or the pharmaceutically acceptable salt thereof according to claim 6, wherein the drug is selected from monomethyl auristatin E (MMAE), monomethyl auristatin F (MMAF), maytansine, SN-38, and exatecan.

**9.** The antibody-drug conjugate or the pharmaceutically acceptable salt thereof according to any one of claims 5-8, wherein the antibody is a bivalent tetramer comprising two light chains and two heavy chains, wherein a first pair of heavy and light chains comprises the heavy chain variable region and the light chain variable region of the anti-human LIV-1 antibody or the antigen-binding fragment thereof according to any one of claims 1-4, and a second pair of heavy or light chains of the tetramer is capable of binding to a non-LIV-1 antigen, wherein the non-LIV-1 antigen is selected from CD3, EGFR, HER2, HER3, PD-L1, c-MET, TROP-2, CEA5, B7-H3, SIRP$\alpha$, PSMA, ROR1, and CD47.

**10.** Use of the anti-human LIV-1 antibody or the antigen-binding fragment thereof according to any one of claims 1-4 or the antibody-drug conjugate or the pharmaceutically acceptable salt thereof according to any one of claims 5-9 in the preparation of a medicament for treating a tumor or cancer,
preferably, the tumor or cancer is selected from the group consisting of breast cancer, bladder cancer, ovarian cancer, pancreatic cancer, hepatocellular carcinoma, gastric cancer, non-Hodgkin's lymphoma, Hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, colorectal cancer, glioma, mesothelioma, cervical cancer, triple-negative breast cancer, lung cancer, head and neck cancer, esophageal cancer, skin cancer, and uterine cancer.

**11.** An immunoconjugate, comprising:

(a) the anti-human LIV-1 antibody or the antigen-binding fragment thereof according to any one of claims 1-4; and
(b) a conjugated moiety selected from the group consisting of a detectable label, a radionuclide, a cytokine, an enzyme, a gold nanoparticle/nanorod, a magnetic nanoparticle, a viral capsid protein, a VLP, and a combination thereof.

**12.** A method for detecting an LIV-1 molecule in a sample, comprising the steps of: (1) contacting the sample with the immunoconjugate according to claim 11; and (2) detecting whether an antigen-antibody complex is formed, wherein the formation of the complex indicates the presence of LIV-1 in the sample.

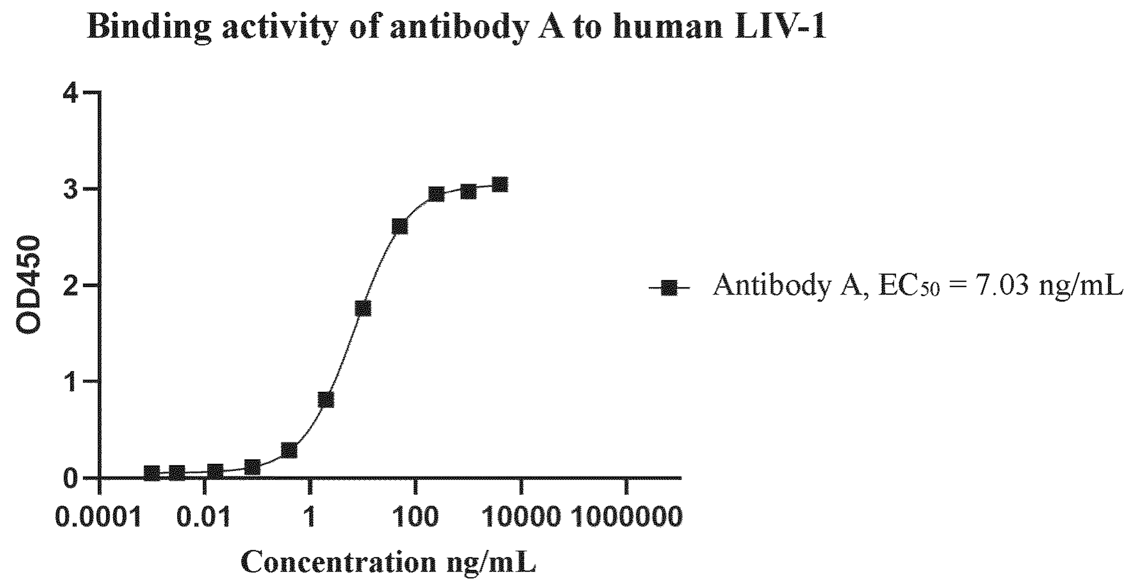
Binding activity of antibody A to human LIV-1
OD450
4
3
2
1
0
0.0001
0.01
1
100
10000
1000000
Concentration ng/mL
Antibody A, EC50 = 7.03 ng/mL

FIG. 1A

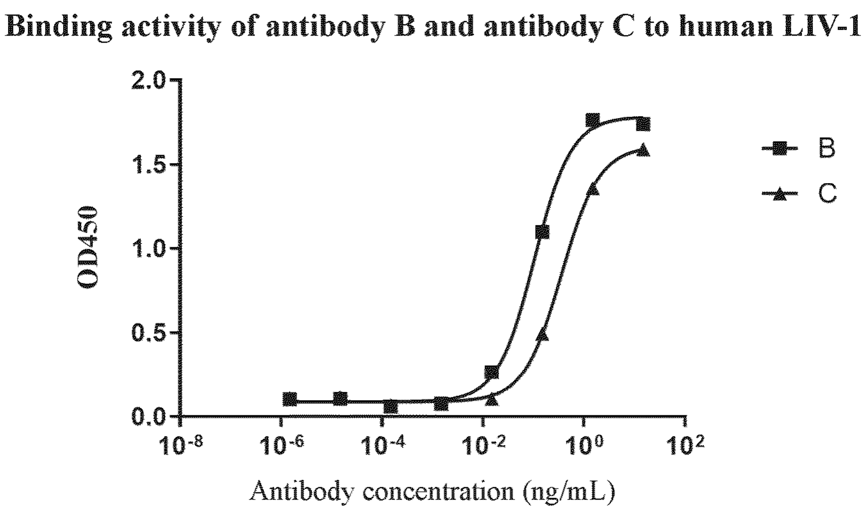
Binding activity of antibody B and antibody C to human LIV-1
OD450
2.0
1.5
1.0
0.5
0.0
10-8
10-6
10-4
10-2
100
102
Antibody concentration (ng/mL)
B
C

FIG. 1B

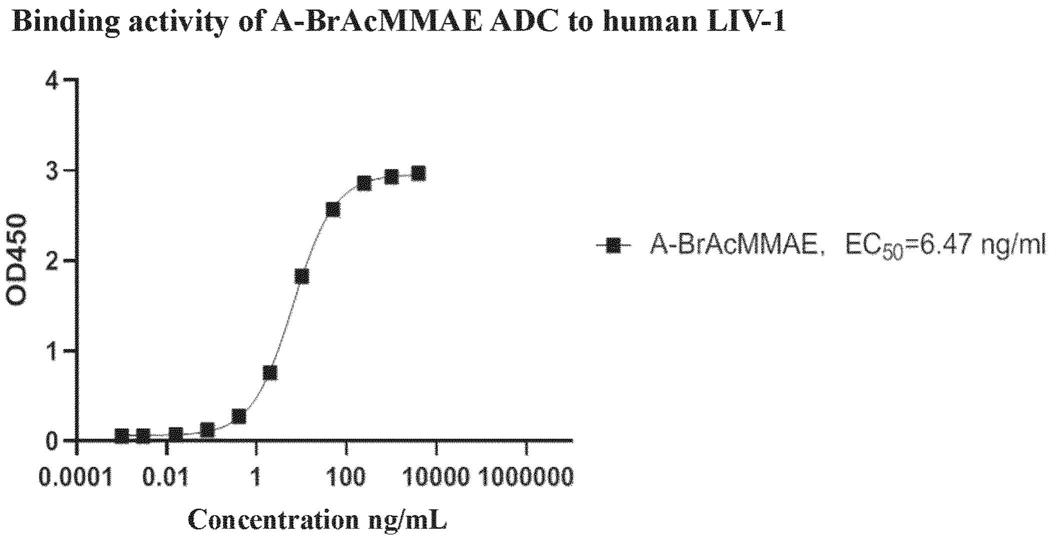
Binding activity of A-BrAcMMAE ADC to human LIV-1
OD450
4
3
2
1
0
0.0001
0.01
1
100
10000
1000000
Concentration ng/mL
A-BrAcMMAE, EC50=6.47 ng/ml

FIG. 2

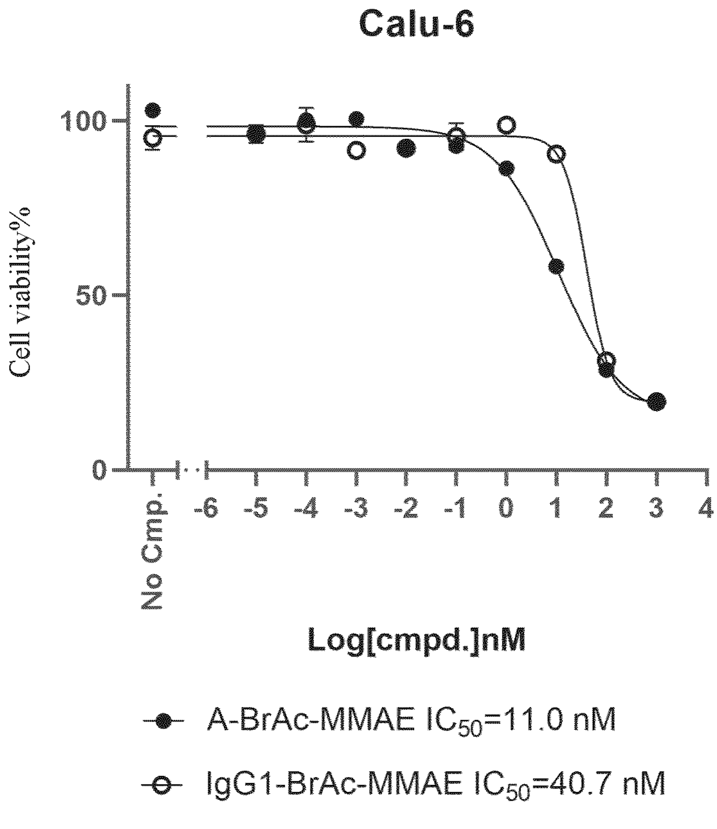
Calu-6
Cell viability%
100
50
0
No Cmp.
-6
-5
-4
-3
-2
-1
0
1
2
3
4
Log[cmpd.]nM
A-BrAc-MMAE IC50=11.0 nM
IgG1-BrAc-MMAE IC50=40.7 nM

FIG. 3

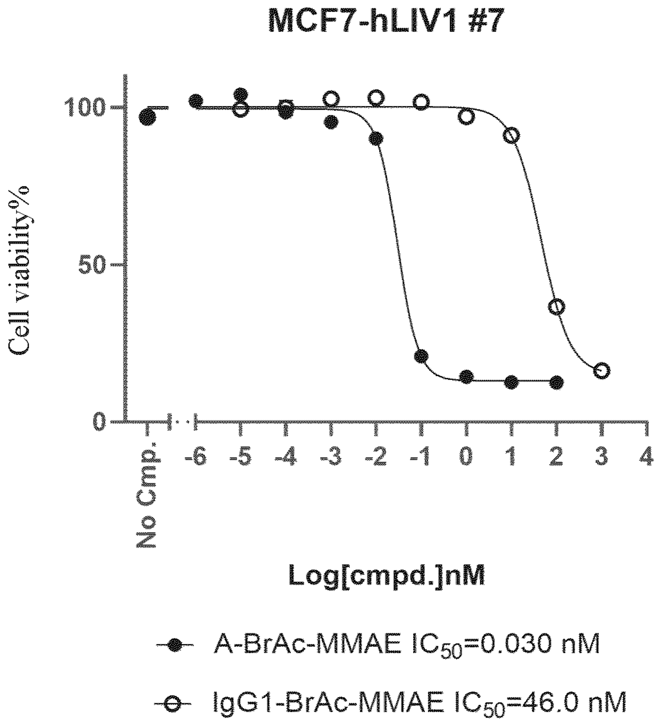
MCF7-hLIV1 #7
Cell viability%
100
50
0
No Cmp.
-6 -5 -4 -3 -2 -1 0 1 2 3 4
Log[cmpd.]nM
A-BrAc-MMAE IC50=0.030 nM
IgG1-BrAc-MMAE IC50=46.0 nM

FIG. 4

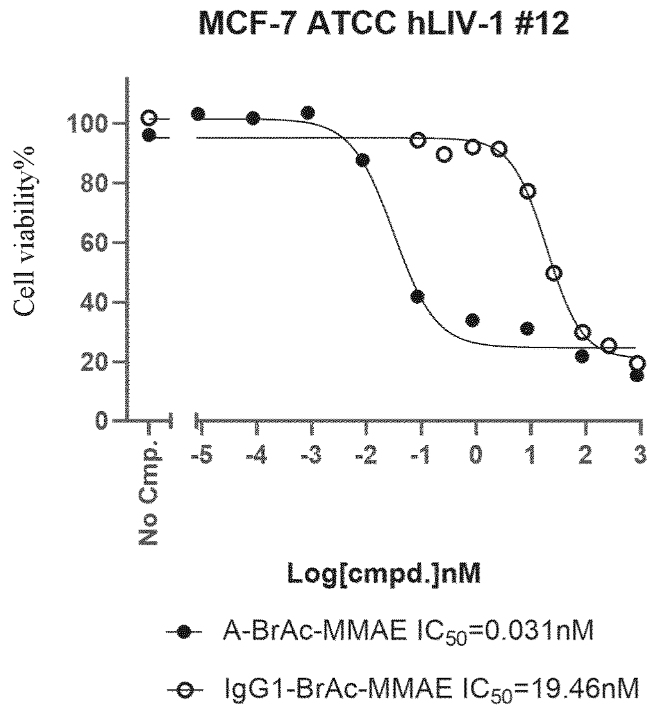
MCF-7 ATCC hLIV-1 #12
Cell viability%
100
80
60
40
20
0
No Cmp.
-5 -4 -3 -2 -1 0 1 2 3
Log[cmpd.]nM
A-BrAc-MMAE IC50=0.031nM
IgG1-BrAc-MMAE IC50=19.46nM

FIG. 5

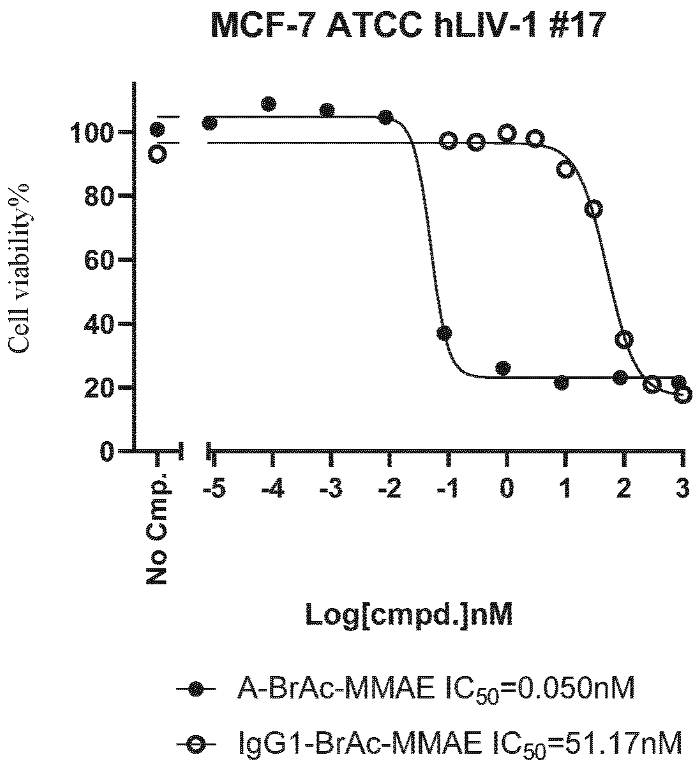
MCF-7 ATCC hLIV-1 #17
Cell viability%
100
80
60
40
20
0
No Cmp.
-5
-4
-3
-2
-1
0
1
2
3
Log[cmpd.]nM
A-BrAc-MMAE IC50=0.050nM
IgG1-BrAc-MMAE IC50=51.17nM

FIG. 6

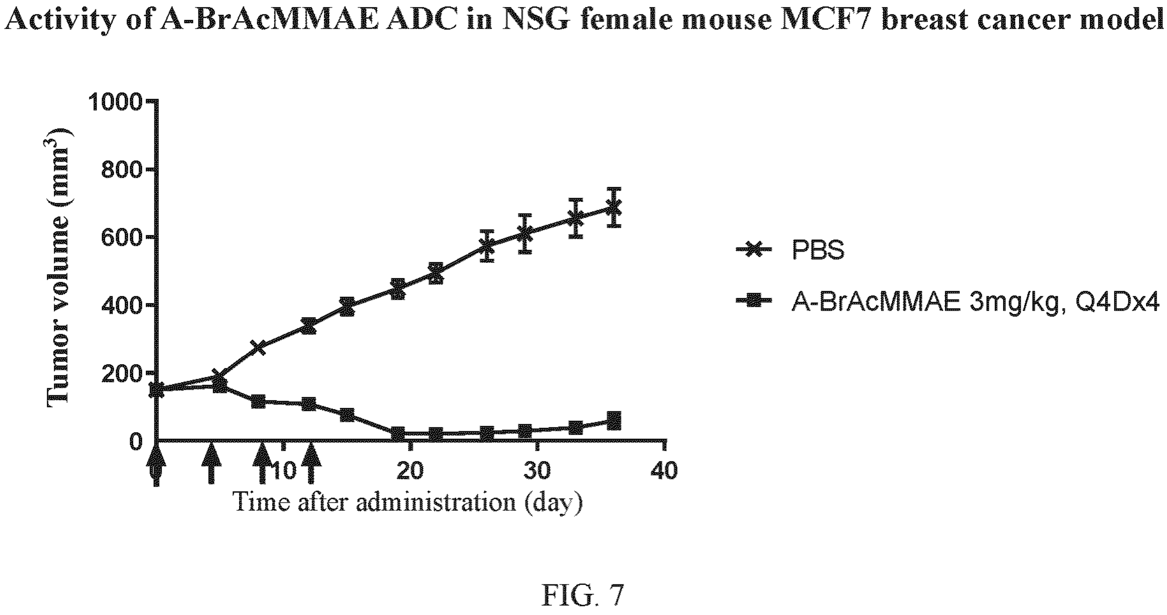
Activity of A-BrAcMMAE ADC in NSG female mouse MCF7 breast cancer model
Tumor volume (mm3)
1000
800
600
400
200
0
10
20
30
40
Time after administration (day)
PBS
A-BrAcMMAE 3mg/kg, Q4Dx4

FIG. 7

Activity of A-BrAcMMAE ADC in NSG female mouse HCC1806 breast cancer model

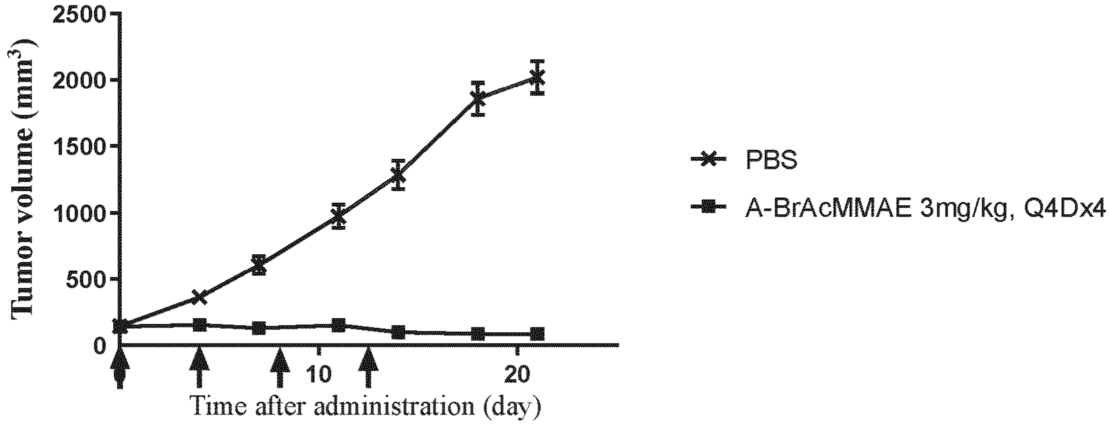


FIG. 8

Activity of ADCs in nude female mouse PC3 prostate cancer model

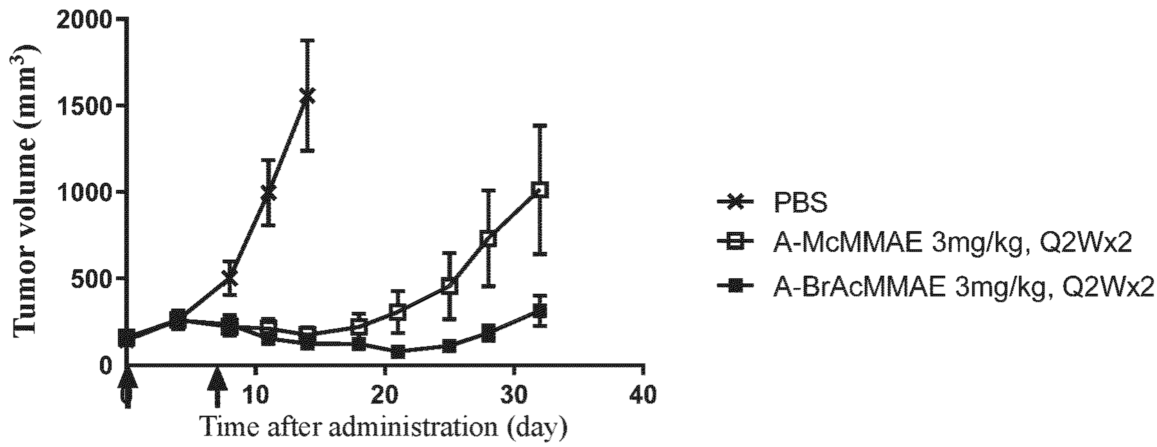


FIG. 9

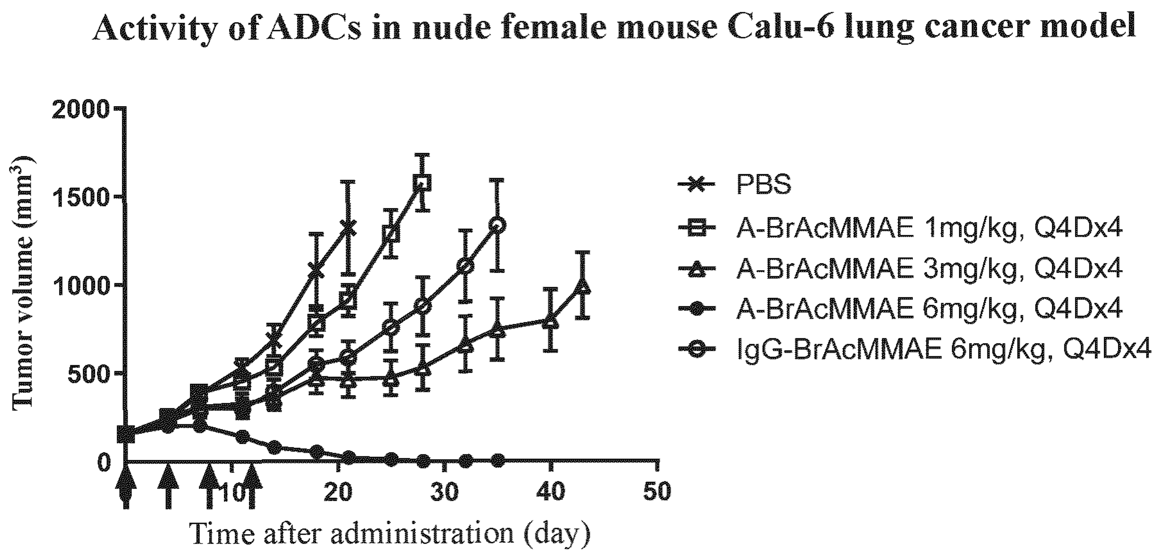
Activity of ADCs in nude female mouse Calu-6 lung cancer model
Tumor volume (mm³)
2000
1500
1000
500
0
10
20
30
40
50
Time after administration (day)
PBS
A-BrAcMMAE 1mg/kg, Q4Dx4
A-BrAcMMAE 3mg/kg, Q4Dx4
A-BrAcMMAE 6mg/kg, Q4Dx4
IgG-BrAcMMAE 6mg/kg, Q4Dx4

FIG. 10

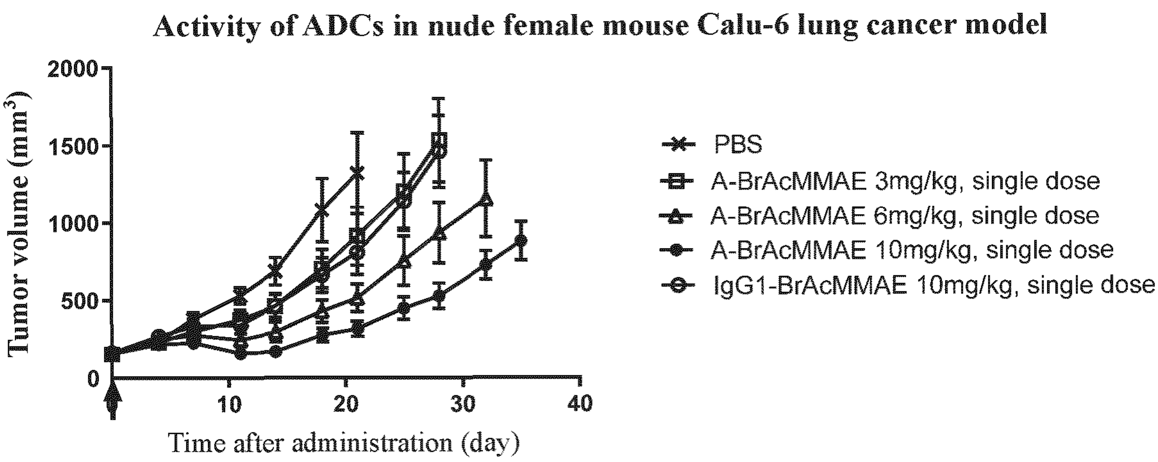
Activity of ADCs in nude female mouse Calu-6 lung cancer model
Tumor volume (mm³)
2000
1500
1000
500
0
10
20
30
40
Time after administration (day)
PBS
A-BrAcMMAE 3mg/kg, single dose
A-BrAcMMAE 6mg/kg, single dose
A-BrAcMMAE 10mg/kg, single dose
IgG1-BrAcMMAE 10mg/kg, single dose

FIG. 11

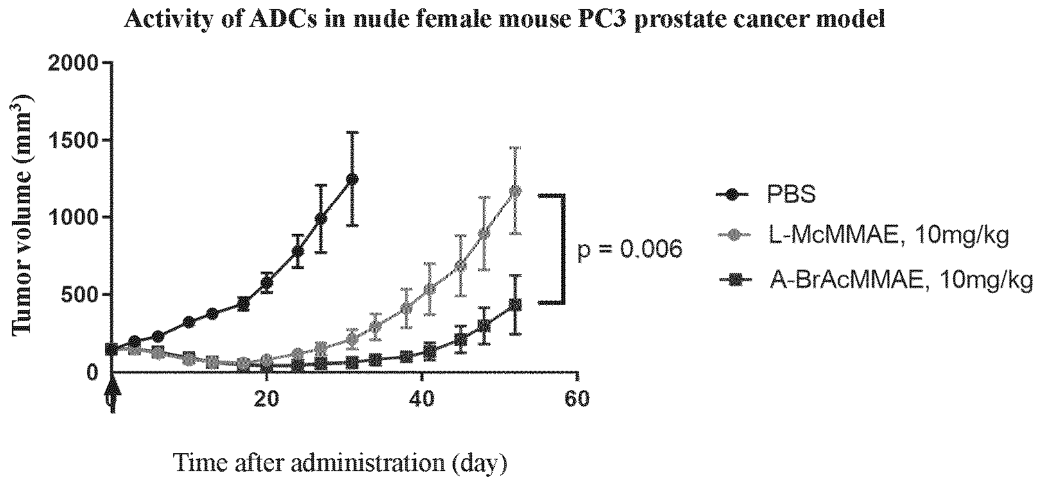
Activity of ADCs in nude female mouse PC3 prostate cancer model
Tumor volume (mm$^3$)
2000
1500
1000
500
0
0
20
40
60
Time after administration (day)
p = 0.006
PBS
L-McMMAE, 10mg/kg
A-BrAcMMAE, 10mg/kg

FIG. 12

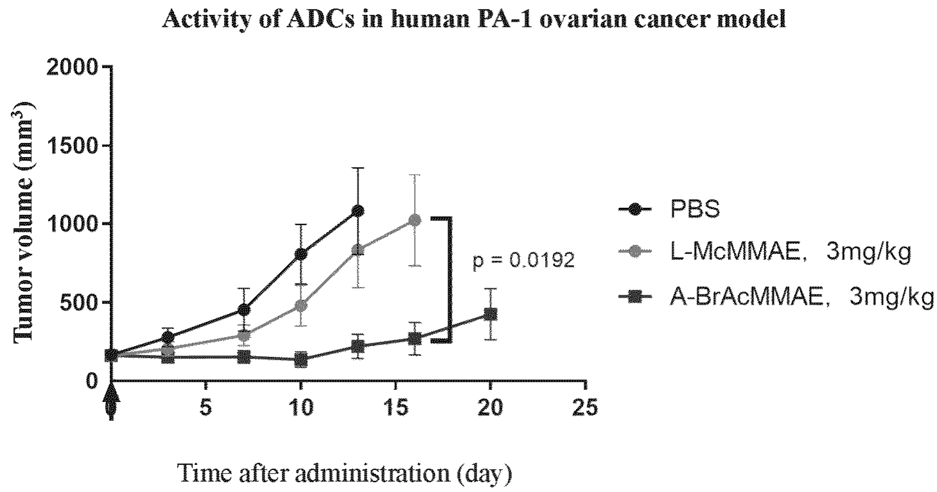
Activity of ADCs in human PA-1 ovarian cancer model
Tumor volume (mm$^3$)
2000
1500
1000
500
0
0
5
10
15
20
25
Time after administration (day)
p = 0.0192
PBS
L-McMMAE, 3mg/kg
A-BrAcMMAE, 3mg/kg

FIG. 13

**INTERNATIONAL SEARCH REPORT**

International application No.
**PCT/CN2024/081911**

**A. CLASSIFICATION OF SUBJECT MATTER**

C07K 16/28(2006.01)i; A61K 39/395(2006.01)i; C07K 16/30(2006.01)i; C07K 16/46(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07K,A61K,A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, CNKI, Web of Science, incoPat, NCBI, EBI, STNext, 百度, BAIDU, 读秀, DUXIU: LIV-1, SLC39A6, ZIP6, monoclonal antibody, conjugate, CAR, cancer, tumor, 抗体, 偶联物, 肿瘤, SEQ ID NOs: 1-24, 26-27, and 30-32

**C. DOCUMENTS CONSIDERED TO BE RELEVANT**

| Category* | Citation of document, with indication, where appropriate, of the relevant passages | Relevant to claim No. |
|---|---|---|
| A | CN 111757892 A (SEATTLE GENETICS, INC.) 09 October 2020 (2020-10-09) claims 1-55 | 1-12 |
| A | CN 103533957 A (SEATTLE GENETICS, INC.) 22 January 2014 (2014-01-22) claims 1-42 | 1-12 |
| A | CN 108697801 A (SEATTLE GENETICS, INC.) 23 October 2018 (2018-10-23) claims 1-30 | 1-12 |
| A | CN 110893236 A (SUN YAT-SEN UNIVERSITY) 20 March 2020 (2020-03-20) entire document | 1-12 |
| A | US 2008171039 A1 (PROTEIN DESIGN LABS, INC.) 17 July 2008 (2008-07-17) entire document | 1-12 |
| A | SUSSMAN, D. et al. "SGN-LIV1A A Novel Antibody–Drug Conjugate Targeting LIV-1 for the Treatment of Metastatic Breast Cancer" *Molecular Cancer Therapeutics*, Vol. 13, No. 12, 31 December 2014 (2014-12-31), pages 2991-3000 | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 May 2024** | **06 June 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/081911**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/081911**

| Patent document cited in search report | Publication date (day/month/year) | Patent family member(s) | Publication date (day/month/year) |
|---|---|---|---|
| CN 111757892 A | 09 October 2020 | JP 2024001187 A | 09 January 2024 |
| | | US 2020283540 A1 | 10 September 2020 |
| | | SG 11202004751 TA | 29 June 2020 |
| | | US 2024076402 A1 | 07 March 2024 |
| | | AU 2018375182 A1 | 11 June 2020 |
| | | KR 20200090838 A | 29 July 2020 |
| | | CA 3084495 A1 | 06 June 2019 |
| | | EA 202091360 A1 | 24 August 2020 |
| | | BR 112020010937 A2 | 17 November 2020 |
| | | MA 50943 A | 07 October 2020 |
| | | MX 2020005640 A | 20 August 2020 |
| | | WO 2019109007 A1 | 06 June 2019 |
| | | IL 274766 A | 30 July 2020 |
| | | JP 2021505540 A | 18 February 2021 |
| | | EP 3717518 A1 | 07 October 2020 |
| CN 103533957 A | 22 January 2014 | PT 3461847 T | 24 December 2020 |
| | | US 2013259860 A1 | 03 October 2013 |
| | | US 9228026 B2 | 05 January 2016 |
| | | AU 2017203851 A1 | 22 June 2017 |
| | | AU 2017203851 B2 | 31 January 2019 |
| | | PL 3156420 T3 | 30 August 2019 |
| | | DK 3156420 T3 | 29 April 2019 |
| | | USRE 48959 E | 08 March 2022 |
| | | ES 2620264 T3 | 28 June 2017 |
| | | MX 2020010639 A | 28 October 2020 |
| | | SG 10201912646 UA | 27 February 2020 |
| | | KR 20200145867 A | 30 December 2020 |
| | | SI 3156420 T1 | 31 May 2019 |
| | | HRP 20170567 T1 | 16 June 2017 |
| | | SI 3461847 T1 | 31 March 2021 |
| | | KR 20190076068 A | 01 July 2019 |
| | | KR 102198189 B1 | 05 January 2021 |
| | | EP 2648752 A2 | 16 October 2013 |
| | | EP 2648752 A4 | 20 May 2015 |
| | | EP 2648752 B1 | 15 February 2017 |
| | | RU 2013130609 A | 20 January 2015 |
| | | RU 2608646 C2 | 23 January 2017 |
| | | IL 273607 A | 31 May 2020 |
| | | IL 273607 B2 | 01 June 2023 |
| | | BR 122021020513 B1 | 25 October 2022 |
| | | EP 3786185 A1 | 03 March 2021 |
| | | EP 3156420 A1 | 19 April 2017 |
| | | EP 3156420 B1 | 20 February 2019 |
| | | SG 10201510041 QA | 28 January 2016 |
| | | ES 2719548 T3 | 11 July 2019 |
| | | IL 261900 A | 31 October 2018 |
| | | CY 1123883 T1 | 24 March 2022 |
| | | US 2018079810 A1 | 22 March 2018 |
| | | HUE 043355 T2 | 28 August 2019 |
| | | BR 112013013781 A2 | 13 September 2016 |

Form PCT/ISA/210 (patent family annex) (July 2022)

**INTERNATIONAL SEARCH REPORT**
**Information on patent family members**

International application No.
**PCT/CN2024/081911**

| Patent document cited in search report | Publication date (day/month/year) | Patent family member(s) | | | Publication date (day/month/year) |
|---|---|---|---|---|---|
| | | AU | 2011338480 | A1 | 13 June 2013 |
| | | AU | 2011338480 | B2 | 16 March 2017 |
| | | AU | 2011338480 | A8 | 23 March 2017 |
| | | AU | 2019202530 | A1 | 02 May 2019 |
| | | AU | 2019202530 | B2 | 01 April 2021 |
| | | NZ | 611468 | A | 26 June 2015 |
| | | IL | 226754 | B | 31 October 2018 |
| | | CY | 1121546 | T1 | 29 May 2020 |
| | | KR | 20230057485 | A | 28 April 2023 |
| | | WO | 2012078688 | A2 | 14 June 2012 |
| | | WO | 2012078688 | A3 | 23 August 2012 |
| | | AU | 2021204473 | A1 | 29 July 2021 |
| | | SG | 190938 | A1 | 31 July 2013 |
| | | MX | 2013006365 | A | 15 August 2013 |
| | | MX | 351027 | B | 28 September 2017 |
| | | DK | 3461847 | T3 | 14 December 2020 |
| | | PL | 3461847 | T3 | 05 July 2021 |
| | | PT | 3156420 | T | 27 May 2019 |
| | | IL | 299837 | A | 01 March 2023 |
| | | CA | 2819038 | A1 | 14 June 2012 |
| | | CA | 2819038 | C | 17 October 2023 |
| | | KR | 20130135884 | A | 11 December 2013 |
| | | KR | 101993921 | B1 | 28 June 2019 |
| | | EP | 3461847 | A1 | 03 April 2019 |
| | | EP | 3461847 | B1 | 23 September 2020 |
| | | EP | 3461847 | A8 | 14 April 2021 |
| | | JP | 2023011709 | A | 24 January 2023 |
| | | ES | 2842895 | T3 | 15 July 2021 |
| | | KR | 20220011811 | A | 28 January 2022 |
| | | ZA | 201303803 | B | 30 July 2014 |
| | | CY | 1118874 | T1 | 10 January 2018 |
| | | PT | 2648752 | T | 28 March 2017 |
| | | US | 2020165335 | A1 | 28 May 2020 |
| | | JP | 2019073515 | A | 16 May 2019 |
| | | JP | 6869218 | B2 | 12 May 2021 |
| | | HUE | 052806 | T2 | 28 May 2021 |
| | | LT | 2648752 | T | 10 April 2017 |
| | | JP | 2017149717 | A | 31 August 2017 |
| | | JP | 6453924 | B2 | 16 January 2019 |
| | | US | 2016185858 | A1 | 30 June 2016 |
| | | US | 9783608 | B2 | 10 October 2017 |
| | | CA | 3211246 | A1 | 14 June 2012 |
| | | PL | 2648752 | T3 | 31 July 2017 |
| | | DK | 2648752 | T3 | 27 March 2017 |
| | | JP | 2014506120 | A | 13 March 2014 |
| | | JP | 6105481 | B2 | 29 March 2017 |
| | | SI | 2648752 | T1 | 30 June 2017 |
| | | HUE | 031726 | T2 | 28 July 2017 |
| | | RS | 55843 | B1 | 31 August 2017 |
| | | JP | 2021106599 | A | 29 July 2021 |

Form PCT/ISA/210 (patent family annex) (July 2022)

**INTERNATIONAL SEARCH REPORT**
**Information on patent family members**

International application No.
**PCT/CN2024/081911**

| Patent document cited in search report | Publication date (day/month/year) | Patent family member(s) | Publication date (day/month/year) |
|---|---|---|---|
| | | JP 7166384 B2 | 07 November 2022 |
| CN 108697801 A | 23 October 2018 | SG 11201807526 SA | 27 September 2018 |
| | | JP 2022058676 A | 12 April 2022 |
| | | MX 2018010847 A | 04 July 2019 |
| | | US 2019085091 A1 | 21 March 2019 |
| | | US 11325980 B2 | 10 May 2022 |
| | | KR 20180121571 A | 07 November 2018 |
| | | JP 2019508433 A | 28 March 2019 |
| | | BR 112018068129 A2 | 15 January 2019 |
| | | CA 3016485 A1 | 21 September 2017 |
| | | US 2023235083 A1 | 27 July 2023 |
| | | MX 2023008849 A | 15 August 2023 |
| | | WO 2017161007 A1 | 21 September 2017 |
| | | SG 10201912908 QA | 27 February 2020 |
| | | AU 2017235545 A1 | 25 October 2018 |
| | | KR 20230169462 A | 15 December 2023 |
| | | EP 3429626 A1 | 23 January 2019 |
| | | EP 3429626 A4 | 06 May 2020 |
| | | KR 20220157515 A | 29 November 2022 |
| | | EA 201891968 A1 | 28 February 2019 |
| | | IL 261505 A | 31 October 2018 |
| | | MA 45324 A | 23 January 2019 |
| | | AU 2024201856 A1 | 11 April 2024 |
| CN 110893236 A | 20 March 2020 | US 2023066474 A1 | 02 March 2023 |
| | | JP 2022551061 A | 07 December 2022 |
| | | WO 2021068890 A1 | 15 April 2021 |
| | | EP 4043034 A1 | 17 August 2022 |
| | | EP 4043034 A9 | 25 January 2023 |
| | | EP 4043034 A4 | 01 November 2023 |
| US 2008171039 A1 | 17 July 2008 | US 2010074841 A1 | 25 March 2010 |
| | | WO 2004067564 A2 | 12 August 2004 |
| | | WO 2004067564 A3 | 17 February 2005 |
| | | WO 2004067564 B1 | 14 April 2005 |
| | | US 2012328513 A1 | 27 December 2012 |
| | | US 8591863 B2 | 26 November 2013 |
| | | US 2014037540 A1 | 06 February 2014 |
| | | US 8906342 B2 | 09 December 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US RE48959 E
- WO 2022026915 A
- US 2013259860 A1


### Non-patent literature cited in the description


- **TAYLOR et al.** *Mol Med*, 2007, vol. 13 (7-8), 396-406
- **SONGSIVILAI** ; **LACHMANN**. *Clin. Exp. Immunol.*, 1990, vol. 79, 315-321
- **KOSTELNY et al.** *J. Immunol.*, 1992, vol. 148, 1547-53
- Fundamental Immunology. Raven Press, 1989
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- Epitope Mapping Protocols. *Methods in Molecular Biology*, 1996, vol. 66
- **JUNGHANS et al.** *Cancer Res.*, 1990, vol. 50, 1495
- Fundamental Immunology. Lippincott-Raven, 1997
- **UCHIDA et al.** *J. Exp. Med.*, 2004, vol. 199, 1659-69
- **AKEWANLOP et al.** *Cancer Res.*, 2001, vol. 61, 4061-65
- **WATANABE et al.** *Breast Cancer Res. Treat.*, 1999, vol. 53, 199-207
- **JANEWAY et al.** Immunobiology. Garland Science, 2005
- Bioconjugation Technology. Elsevier